# EUROPEAN PATENT SPECIFICATION

(11) **EP 3 688 016 B1**
(45) Date of publication and mention of the grant of the patent: **31.01.2024**
(21) Application number: 18778500.1
(22) Date of filing: 01.10.2018
(51) Int. Cl.: C07K 14/415, A01H 1/04, A01H 5/12, A01H 6/02, C12N 15/82

(54) **CMV RESISTANCE ALLELE**
CMV-RESISTENZALLEL
ALLÈLE DE RÉSISTANCE AU CMV

(30) Priority: 29.09.2017 WO PCT/EP2017/074862; 29.09.2017 US 201715720757
(43) Date of publication of application: 05.08.2020
(73) Proprietor: Rijk Zwaan Zaadteelt en Zaadhandel B.V., 2678 KX De Lier (NL)
(72) Inventor: KOCK, Vincent Laurens Adrianus, 2678 KX De Lier (NL); FEITSMA, Johannes Geert Jan, 2678 KX De Lier (NL); FRIJTERS, Raoul Jacobus Johannes Maria, 2678 KX De Lier (NL)
(74) Representative: Arnold & Siedsma
(86) International application number: PCT/EP2018/076640
(87) International publication number: WO 2019/063839

(56) References cited:
- WO-A1-2018/060474
- US-B1- 9 402 363
- SCHMIDT H E ET AL: "MULTIPLE RESISTANCE OF SPINACH SPINACIA-OLERACEA L. TO CUCUMBER MOSAIC AND BEET MILD YELLOWING VIRUSES", ZENTRALBLATT FUER MIKROBIOLOGIE, vol. 144, no. 1, 1989, pages 13-18, XP009502335, ISSN: 0232-4393
- FENG CHUNDA ET AL: "Construction of a Spinach Bacterial Artificial Chromosome (BAC) Library as a Resource for Gene Identification and Marker Development", PLANT MOLECULAR BIOLOGY REPORTER, SPRINGER SCIENCE+BUSINESS MEDIA B.V, NL, vol. 33, no. 6, 16 May 2015 (2015-05-16), pages 1996-2005, XP035928473, ISSN: 0735-9640, DOI: 10.1007/S11105-015-0891-9 [retrieved on 2015-05-16]
- HIDEKI TAKAHASHI ET AL: "RCY1, an Arabidopsis thaliana RPP8/HRT family resistance gene, conferring resistance to cucumber mosaic virus requires salicylic acid, ethylene and a novel signal transduction mechanism", THE PLANT JOURNAL, vol. 32, no. 5, 1 December 2002 (2002-12-01), pages 655-667, XP055434281, GB ISSN: 0960-7412, DOI: 10.1046/j.1365-313X.2002.01453.x
- IRISH B M ET AL: "Characterization of a Resistance Locus (Pfs-1) to the Spinach Downy Mildew Pathogen (Peronospora farinosa f. sp. spinaciae) and Development of a Molecular Marker Linked to Pfs-1", PHYTOPATHOLOGY, AMERICAN PHYTOPATHOLOGICAL SOCIETY, US, vol. 98, no. 8, 1 August 2008 (2008-08-01) , pages 894-900, XP002672007, ISSN: 0031-949X, DOI: 10.1094/PHYTO-98-8-0894

## Description

### FIELD OF THE INVENTION

The invention relates to an allele capable of conferring resistance in a spinach plant against Cucumber Mosaic Virus (CMV). The disclosure also relates to a spinach plant, to propagation material of said spinach plant, to a cell of said spinach plant, and to seed of said spinach plant carrying the allele. The disclosure further relates to a method of producing a spinach plant carrying the allele and to the use of the allele in breeding to confer CMV resistance.

### BACKGROUND OF THE INVENTION

Spinach (*Spinacia oleracea* L.) is a flowering plant from the Amaranthaceae family that is grown as a vegetable. The consumable parts of spinach are the leaves and petioles from the vegetative stage. Spinach is sold loose, bunched, in pre-packed bags, canned, or frozen. There are three basic types of spinach: industry-, fresh and Asiatic spinach. Within these types three different leaf types can be recognised: savoy, semi-savoy and smooth types. Savoy has crinkly and curly leaves. Flat or smooth leaf spinach has broad, smooth leaves. Semi-savoy is a variety with slightly crinkled leaves. The main market for spinach is baby-leaf. Baby spinach leaves are often of the flat-leaf variety and usually the harvested leaves are not longer than about eight centimeter. These tender, sweet leaves are sold loose rather than in bunches. They are often used in salads, but can also be lightly cooked.

One of the diseases threatening the production of spinach is Cucumber Mosaic Virus (CMV). Cucumber Mosaic Virus is a positive-sense single stranded RNA virus belonging to the Bromoviridae family and the Cucumovirus genus. The virus has a worldwide distribution and it is believed to have the broadest host range of any known plant virus. It has been reported to be able to infect over 1200 plant species in over 100 plant families.

The virus can be transmitted in many different ways e.g. mechanically, by insect vectors such as aphids, on seeds and even by parasitic weeds. The symptoms and severity of CMV infection depend on the species and the age of the plant. Typical symptoms for CMV are mottling, yellowing, the formation of ringspots, stunting and distortion of leaf, fruit and flowers.

CMV is in spinach also known as spinach blight. Symptoms observed on infected spinach plants commonly are leaf chlorotic mottle, narrowing, wrinkling and inward rolling of the leaves, and distortion of the veins.

Although CMV resistance is observed in some spinach cultivars, the genetic basis for the resistance in those cultivars has never been characterized at the molecular level, i.e. the responsible gene and its sequence until now were unknown. Furthermore, there are no closely linked molecular markers known in the art that may identify CMV resistance in spinach, nor are the molecular characteristics of the genes themselves known in the art. Therefore, the identification of CMV resistance in spinach plants is currently based on phenotypic assays in which many accessions are screened for resistance based on the absence of disease symptoms in a disease test.

Therefore, it is the object of the invention to provide a new resistance allele conferring resistance to CMV in spinach and to provide molecular tools for identifying this resistance allele.

### SUMMARY OF THE INVENTION

In the research leading to the present invention, it was surprisingly found that at the same locus on which several *Peronosporafarinosa* f. sp. *spinaciae* resistance conferring alleles have been observed also a CMV resistance conferring allele is located.

At this locus one or two so-called WOLF-genes are located. These one or two genes, which are either "*alpha-WOLF*" type or "*beta-WOLF*" type genes (together referred to as "the *WOLF* genes or *alphalbeta-WOLF* genes") each encode a protein that belongs to the CC-NBS-LRR (Coiled Coil - Nucleotide Binding Site - Leucine-Rich Repeat) family. Depending on the allelic variant (or the allelic variants) that is (are) present in a spinach plant, said plant will produce a variant of the WOLF protein that confers a certain resistance profile to pathogenic races of downy mildew. The research leading to the present invention has now elucidated that one allelic variant of the *alpha-WOLF* type does not provide resistance to downy mildew, but instead confers resistance to CMV.

In the context of this invention the term "allele" or "allelic variant" is used to designate a version of a gene that is linked to a specific phenotype, i.e. resistance, more in particular CMV resistance. It was found that a spinach plant may carry one or two *WOLF* genes. For each of these two *WOLF* genes multiple alleles exist. The *WOLF* gene allele of the invention confers resistance to CMV. In the context of this invention an allele or allelic variant is a nucleic acid.

The beta-*WOLF* gene of spinach variety Viroflay is located on scaffold12735 (sequence: GenBank: KQ143339.1), at position 213573-221884. In case the spinach plant also carries or only carries an alpha-WOLF gene, the alpha-WOLF gene is located at approximately the same location as where the beta-WOLF gene is located on scaffold!2735 in the Viroflay genome assembly.

### DETAILED DESCRIPTION OF THE INVENTION

A genome assembly for spinach variety Viroflay - which is susceptible to CMV - is publicly available (*Spinacia oleracea* cultivar SynViroflay, whole genome shotgun sequencing project; Bioproject: PRJNA41497; GenBank: AYZV00000000.2; BioSample: SAMN02182572, see also Dohm et al, 2014, Nature 505: 546-549). In this genome assembly for Viroflay, the beta-*WOLF* gene is located on scaffold12735 (sequence: GenBank: KQ143339.1), at position 213573-221884. The sequence covered by this interval comprises the entire genomic sequence of the beta-*WOLF* gene of Viroflay, plus 2000 basepairs sequence upstream of the gene, plus the sequence downstream of the gene, up to the locus of the neighbouring gene that is situated downstream of the *WOLF* gene. Spinach variety Viroflay only possesses a single *WOLF* gene, namely a beta-*WOLF* gene, but many other spinach lines harbor a single alpha-type *WOLF* gene at the same location in the genome. Other spinach lines harbor two *WOLF* genes at approximately the same location in the genome. In such cases, the two *WOLF* genes are positioned adjacent to each other. In most spinach lines that harbor two *WOLF* genes, one of said *WOLF* genes belongs to the alpha-type, and the other *WOLF* gene belongs to the beta-type. In the research leading to the present invention, it was observed that this allelic variation in the *WOLF* locus is responsible for differences in resistance to pathogenic races of *Peronospora farinosa* f. sp. *spinaciae*, but surprisingly enough one of the allelic variants confers resistance to CMV instead of downy mildew.

The difference between an allele of an alpha-*WOLF* gene and an allele of a beta-*WOLF* gene lies in the presence of specific conserved amino acid motifs in the encoded protein sequence. As mentioned above, all WOLF proteins possess - from N- to C-terminus - the following domains that are generally known in the art: a coiled coil domain (RX-CC-like, cd14798), an NBS domain (also referred to as "NB-ARC domain", pfam00931; van der Biezen & Jones, 1998, Curr. Biol. 8: R226-R228), and leucine-rich repeats (IPR032675) which make up the LRR domain. In addition, all WOLF proteins comprise in their amino acid sequence the motif "MAEIGYSVC" at the N-terminus. In addition to this, all alpha-WOLF proteins comprise the motif "KWMCLR" in their amino acid sequence, whereas all beta-WOLF proteins comprise the motif "HVGCVVDR" in their amino acid sequence.

The present invention relates to a new CMV resistance conferring allele of the alpha-WOLF gene designated alpha-CMV.

In particular, the invention relates to a CMV resistance conferring allele designated alpha-CMV which confers resistance to CMV when present in a spinach plant, wherein the protein encoded by said allele is a CC-NBS-LRR protein that comprises in its amino acid sequence: a) the motif "MAEIGYSVC" at its N-terminus; b) the motif "KWMCLR"; and c) an LRR domain that has in order of increased preference at least 95%, 96%, 97%, 98%, 99%, 100% sequence similarity to SEQ ID No: 10. Optionally, the alpha-CMV allele further comprises an additional motif in its amino acid sequence, namely "DQEDEGEDN".

For the purpose of this invention, the LRR domain of the protein encoded by the alpha-CMV allele is defined as the amino acid sequence that in order of increased preference has at least 95%, 96%, 97%, 98%, 99%, 100% sequence similarity to SEQ ID No: 10. The LRR domain of the invention starts with the motif "KWMCLR".

The skilled person is familiar with methods for the calculation of sequence similarity. Suitably, sequence similarity is calculated using EMBOSS stretcher 6.6.0 (www.ebi.ac.uk/Tools/psa/emboss stretcher), using the EBLOSUM62 matrix and the resulting "similarity score".

In one embodiment, the LRR domain of the protein encoded by the alpha-CMV allele is defined as the amino acid sequence that in order of increased preference has at least 95%, 96%, 97%, 98%, 99%, 100% sequence similarity to SEQ ID No: 10.

In one embodiment, the invention relates to a nucleic acid encoding a CC-NBS-LRR protein that comprises in its amino acid sequence: a) the motif "MAEIGYSVC" at its N-terminus; b) the motif "KWMCLR"; and c) an LRR domain that has in order of increased preference at least 95%, 96%, 97%, 98%, 99%, 100% sequence similarity to SEQ ID No: 10.

In a further embodiment, the invention relates to a nucleic acid having a genomic nucleotide sequence which in order of increased preference has at least 80%, 81%, 82%, 83%, 84%,85%, 86%, 87%, 88%, 89%, 90%, 91%, 92%, 93%, 94%, 95%, 96%, 97%, 98%, 99%, 100% sequence similarity to SEQ ID No: 1.

In one embodiment, the invention relates to a nucleic acid having a coding sequence which in order of increased preference has at least 95%, 96%, 97%, 98%, 99%, 100% sequence similarity to SEQ ID No:2 or SEQ ID No:3.

The LRR domain of the alpha-CMV allele as defined herein may be determined by amplifying and sequencing the genomic DNA encoding for the amino acid sequence of LRR domain using specific primers, and subsequently translating the DNA sequence into an amino acid sequence, thereby applying common sense in choosing the correct reading frame. The skilled person is capable of doing this, using freely available online bioinformatics tools such as can be found here: http://web.expasy.org/translate/

The genomic sequence of an LRR domain of an alpha-WOLF gene such as alpha-CMV may be amplified using a primer pair having a forward primer which is a nucleic acid molecule having the sequence of SEQ ID No:6 and a reverse primer which is a nucleic acid molecule having the sequence of SEQ ID No:7.

PCR conditions for amplifying the LRR domain-encoding region of an alpha-*WOLF* gene such as the alpha-CMV allele using primers having SEQ ID No:6 and SEQ ID No:7 are, using Platinum Taq enzyme (Thermo Fisher Scientific): 3 minutes at 95°C (initial denaturing step); 40 amplification cycles, each cycle consisting of: 30 seconds denaturation at 95°C, 30 seconds annealing at 60°C, and 30 seconds extension at 72°C; 2 minutes at 72°C (final extension step).

The LRR domain of a beta-WOLF gene, e.g. the null allele as present in variety Viroflay, may be amplified using a forward primer which is a nucleic acid molecule having the sequence of SEQ ID No:8 and a reverse primer which is a nucleic acid molecule having the sequence of SEQ ID No:7.

PCR conditions for amplifying the LRR domain-encoding region of a beta- *WOLF* gene using primers having SEQ ID No:8 and SEQ ID No:7 are as follows, using Platinum Taq enzyme (Thermo Fisher Scientific): 3 minutes at 95°C (initial denaturing step); 40 amplification cycles, each cycle consisting of: 30 seconds denaturation at 95°C, 50 seconds annealing at 58°C and 50 seconds extension at 72°C; 2 minutes at 72°C (final extension step).

Therefore, the invention also relates to a primer pair for amplifying the LRR domain of an alpha-CMV allele wherein the forward primer is a nucleic acid molecule comprising the sequence of SEQ ID No:6 and the reverse primer which is a nucleic acid molecule comprising the sequence of SEQ ID No:7. The primers disclosed herein have been specifically designed for selectively amplifying part of a *WOLF* gene, and not of any other CC-NBS-LRR protein-encoding genes using the conditions as set forth above.

The invention relates to an alpha-CMV which confers resistance to CMV when present in a spinach plant, wherein the protein encoded by said allele is a CC-NBS-LRR protein that comprises in its amino acid sequence: a) the motif "MAEIGYSVC" at its N-terminus; and b) the motif "KWMCLR"; and wherein the allele has a genomic nucleotide sequence which in order of increased preference has at least 80%, 81%, 82%, 83%, 84%, 85%, 86%, 87%, 88%, 89%, 90%, 91%, 92%, 93%, 94%, 95%, 96%, 97%, 98%, 99%, 100% sequence similarity to SEQ ID No:1.

The invention relates to an alpha-CMV allele which has a genomic sequence that in order of increased preference has at least 80%, 81%, 82%, 83%, 84%, 85%, 86%, 87%, 88%, 89%, 90%, 91%, 92%, 93%, 94%, 95%, 96%, 97%, 98%, 99%, 100% sequence similarity to SEQ ID No:1.

SEQ ID No:21 provided in Table 2 represents the genomic sequence of the alpha-CMV allele and the genomic sequence of the beta-CMV allele. SEQ ID No:21 further comprises a 2 kilobase region upstream of the start codon (ATG) of the alpha-CMV allele. By using the coding sequence of the alpha-CMV allele represented by SEQ ID No: 2 the skilled person is able to identify the genomic sequence of the alpha-CMV allele with a 2 kilobase region upstream of the start codon (ATG) represented by SEQ ID No: 1.

The invention relates to two different splice variants. In one embodiment, the invention relates to an alpha-CMV allele which has a coding sequence which in order of increased preference has at least 95%, 96%, 97%, 98%, 99%, 100% sequence similarity to SEQ ID No:2. This is the first splice variant of the alpha-CMV allele. In another embodiment, the invention relates to an alpha-CMV allele which has a coding sequence which in order of increased preference has at least 94%, 95%, 96%, 97%, 98%, 99%, 100% sequence similarity to SEQ ID No:3. This is the second splice variant of the alpha-CMV allele.

In a further aspect of the invention the alpha-CMV allele encodes for a protein having an amino acid sequence which in order of increased preference has at least 95%, 96%, 97%, 98%, 99%, 100% sequence similarity to SEQ ID No:4.

In another embodiment the alpha-CMV allele encodes for a protein having an amino acid sequence which in order of increased preference has at least 95%, 96%, 97%, 98%, 99%, 100% sequence similarity to SEQ ID No:5.

The alpha-CMV allele when present in a spinach plant confers resistance to CMV, preferably complete resistance to CMV. Resistance against CMV in spinach plants may be tested by mechanically inoculating plants with the CMV virus which is a mixture of two isolates (NL 16 and SP 43) when the plant has two or three true leaves. Plants to be tested are grown under a regime with a day temperature of 20°C and a night temperature of 18°C and receive at least 16 hours of light. Inoculation is done by dusting all true leaves of the plants with carborundrum powder and subsequently rubbing them with a sponge soaked with inoculum. The inoculum is a mixture of equal amounts of both isolates diluted in water, preferably a 1:10 dilution. After inoculation, plants may be slightly rinsed with water. Symptoms may be observed 7 to 9 days after inoculation. A resistant plant, i.e. a plant comprising the allele of the invention, shows no symptoms, while a susceptible plant typically shows dwarf growth and mosaic symptoms in the heart of the plant.

A detailed example of the test described herein can be found in the CPVO protocol for tests on distinctness, uniformity, and stability for spinach as available on: http://www.cpvo.europa.eu/documents/TP/vegetales/TP 055-5Rev Spinacia oleracea.pdf.

Also disclosed herein is a spinach plant, comprising the alpha-CMV allele of the invention, of which a representative sample of seed was deposited with the NCIMB under NCIMB accession number 42651.

Further disclosed herein is a plant which comprises the alpha-CMV allele being an agronomically elite spinach plant.

An agronomically elite spinach plant is a non-naturally occurring plant having a genotype that results into an accumulation of distinguishable and desirable agronomic traits which is the result of human intervention, and is e.g. achieved by crossing and selection, mutagenizing, transforming or otherwise introducing such traits. An agronomically elite spinach plant includes any cultivated *Spinacia oleracea* plant regardless of type, such as breeding lines (e.g. backcross lines, inbred lines), cultivars and varieties (open pollinated or hybrids). Plants of *Spinacia oleracea* occurring in the wild (i.e. not cultivated spinach) or wild relatives of *Spinacia oleracea*, such as *Spinacia tetrandra* and *Spinacia turkestanica*, are not encompassed by this definition.

Preferably, the agronomically elite spinach plant comprising the alpha-CMV allele is a plant of an inbred line or a hybrid.

As used herein, a plant of an inbred line is a plant of a population of plants that is the result of three or more rounds of selfing, or backcrossing; or which plant is a double haploid. An inbred line may e.g. be a parent line used for the production of a commercial hybrid.

As used herein, a hybrid plant is a plant which is the result of a cross between two different plants having different genotypes. More in particular, a hybrid plant is the result of a cross between plants of two different inbred lines, such a hybrid plant may e.g. be a plant of an F₁ hybrid variety.

A plant carrying the alpha-CMV allele in heterozygous form may further comprise a beta-WOLF 0 allele as e.g. present in variety Viroflay wherein the beta-WOLF 0 allele does not confer any resistance to CMV or downy mildew. Alternatively, a plant heterozygous for the alpha-CMV allele may further comprise an allele of the alpha/beta-*WOLF* gene that does provide resistance to downy mildew. Preferably, such an allele would provide at least an intermediate resistance to one or more races of downy mildew. More preferably, such an allele would provide at least an intermediate resistance to at least 10 races of downy mildew. Most preferably the allele of the alpha/beta-WOLF gene confers resistance against downy mildew such that the plant is completely resistant to *Peronospora farinosa* f. sp. *spinaciae* races Pfs:1 to Pfs:16.

Additionally disclosed herein is a plant comprising the alpha-CMV allele and a downy mildew resistance conferring allele of the alpha/beta-WOLF gene, wherein the downy mildew resistance conferring allele is beta-WOLF 3 having a genomic sequence which in order of increased preference has at least 90%, 91%, 92%, 93%, 94%, 95%, 96%, 97%, 98%, 99%, 100% sequence similarity to SEQ ID No: 13, and wherein the plant is at least resistant to CMV and *Peronosporafarinosa* f. sp. *spinaciae* races Pfs:1, Pfs:3, Pfs:5, Pfs:9, Pfs:11, Pfs:12, Pfs:14, and Pfs:16.

Further disclosed herein is a plant comprising the alpha-CMV allele and a downy mildew resistance conferring allele of the alpha/beta-*WOLF* gene, wherein the downy mildew resistance conferring allele is alpha-WOLF 6 having a genomic sequence which in order of increased preference has at least 90%, 91%, 92%, 93%, 94%, 95%, 96%, 97%, 98%, 99%, 100% sequence similarity to SEQ ID No: 14, and wherein the plant is at least resistant to CMV and *Peronosporafarinosa* f. sp. *spinaciae* races Pfs:1, Pfs:2, Pfs:3, Pfs:4, Pfs:5, Pfs:6, Pfs:9, Pfs:11, Pfs:12, Pfs:14, Pfs:15, and Pfs:16.

Furthermore disclosed herein is a plant comprising the alpha-CMV allele and a downy mildew resistance conferring allele of the alpha/beta-*WOLF* gene, wherein the downy mildew resistance conferring allele is alpha-WOLF 6b having a genomic sequence which in order of increased preference has at least 90%, 91%, 92%, 93%, 94%, 95%, 96%, 97%, 98%, 99%, 100% sequence similarity to SEQ ID No: 15, and wherein the plant is at least resistant to CMV and *Peronospora farinosa* f. sp. *spinaciae* races Pfs: 1, Pfs:2, Pfs:3, Pfs:4, Pfs:5, Pfs:6, Pfs:9, Pfs: 11, Pfs:12, Pfs:14, Pfs:15, Pfs:16 and isolate US1508.

Also disclosed herein is a plant comprising the alpha-CMV allele and a downy mildew resistance conferring allele of the alpha/beta-*WOLF* gene, wherein the downy mildew resistance conferring allele is alpha-WOLF 8 having a genomic sequence which in order of increased preference has at least 90%, 91%, 92%, 93%, 94%, 95%, 96%, 97%, 98%, 99%, 100% sequence similarity to SEQ ID No: 16, and wherein the plant shows at least resistance to CMV and *Peronospora farinosa* f. sp. *spinaciae* races Pfs:1, Pfs:2, Pfs:6, Pfs:8, Pfs:15, and intermediate resistance to Pfs:16.

Further disclosed herein is a plant comprising the alpha-CMV allele and a downy mildew resistance conferring allele of the alpha/beta-*WOLF* gene, wherein the downy mildew resistance conferring allele is alpha-WOLF 9 having a genomic sequence which in order of increased preference has at least 90%, 91%, 92%, 93%, 94%, 95%, 96%, 97%, 98%, 99%, 100% sequence similarity to SEQ ID No: 17, and wherein the plant is at least resistant to CMV and *Peronospora farinosa* f. sp. *spinaciae* races Pfs:1, Pfs:2, Pfs:3, Pfs:4, Pfs:5, Pfs:6, Pfs:7, Pfs:8, Pfs:9, Pfs:10, Pfs: 11, Pfs:12, and Pfs:13.

Additionally disclosed herein is a plant comprising the alpha-CMV allele and a downy mildew resistance conferring allele of the alpha/beta-*WOLF* gene, wherein the downy mildew resistance conferring allele is alpha-WOLF 11 having a genomic sequence which in order of increased preference has at least 90%, 91%, 92%, 93%, 94%, 95%, 96%, 97%, 98%, 99%, 100% sequence similarity to SEQ ID No: 18, and wherein the plant is at least resistant to CMV and *Peronosporafarinosa* f. sp. *spinaciae* races Pfs:1, Pfs:3, Pfs:4, Pfs:5, Pfs:7, Pfs:11, Pfs:13, Pfs:15, Pfs:16 and isolate US1508.

Furthermore disclosed herein is a plant comprising the alpha-CMV allele and a downy mildew resistance conferring allele of the alpha/beta-*WOLF* gene, wherein the downy mildew resistance conferring allele is alpha-WOLF 12 having a genomic sequence which in order of increased preference has at least 90%, 91%, 92%, 93%, 94%, 95%, 96%, 97%, 98%, 99%, 100% sequence similarity to SEQ ID No:19, and wherein the plant is at least resistant to CMV and *Peronosporafarinosa* f. sp. *spinaciae* races Pfs:1, Pfs:2, Pfs:3, Pfs:4, Pfs:6, Pfs:7, Pfs:8, Pfs:9, Pfs: 10, Pfs: 11, Pfs: 12 and isolate Pfs: 13.

Also disclosed herein is a plant comprising the alpha-CMV allele and a downy mildew resistance conferring allele of the alpha/beta-*WOLF* gene, wherein the downy mildew resistance conferring allele is alpha-WOLF 15 having a genomic sequence which in order of increased preference has at least 90%, 91%, 92%, 93%, 94%, 95%, 96%, 97%, 98%, 99%, 100% sequence similarity to SEQ ID No:20, and wherein the plant is at least resistant to CMV and *Peronospora farinosa* f. sp. *spinaciae* races Pfs:1, Pfs:2, Pfs:3, Pfs:4, Pfs:5, Pfs:6, Pfs:9, Pfs:11, Pfs:12, Pfs:13, Pfs:14, and Pfs:15.

Further disclosed herein is a plant comprising the alpha-CMV allele and an allele of an alpha/beta-*WOLF* gene conferring resistance to one or more *Peronospora farinosa* f. sp. *spinaciae* races being an agronomically elite plant, preferably a hybrid plant.

The disclosure further relates to propagation material comprising the alpha-CMV allele. In one embodiment, the propagation material is suitable for sexual reproduction. Such propagation material comprises for example a microspore, pollen, ovary, ovule, embryo sac and egg cell. In another embodiment, the propagation material is suitable for vegetative reproduction. Such propagation material comprises for example a cutting, root, stem, cell, protoplast, and a tissue culture of regenerable cells. A part of the plant that is suitable for preparing tissue cultures is in particular a leaf, pollen, an embryo, a cotyledon, a hypocotyl, a meristematic cell, a root tip, an anther, a flower, a seed and a stem.

Further disclosed herein is the use of a tissue culture comprising the alpha-CMV allele for the production of a spinach plant showing resistance to CMV.

Additionally disclosed herein is the use of a spinach plant comprising the alpha-CMV allele, as a source of propagating material.

Also disclosed herein is the use of a spinach plant comprising the alpha-CMV allele, as a source of seed.

Furthermore disclosed herein is a cell of a spinach plant comprising the alpha-CMV allele. Such a cell may be either in isolated form or may be part of the complete plant or parts thereof and then still constitutes a cell as disclosed herein because such a cell harbors the alpha-CMV allele that confers resistance to CMV. Each cell of the plant as disclosed herein carries the genetic information that confers resistance to CMV. Such a cell as disclosed herein may also be a regenerable cell that may be used to regenerate a new plant comprising the allele of the invention.

Also disclosed herein is the use of a cell comprising the alpha-CMV allele for the production of a spinach plant showing resistance to CMV.

Additionally disclosed herein is a method for making a hybrid spinach seed comprising crossing a first parent spinach plant with a second parent spinach plant and harvesting the resultant hybrid spinach seed, wherein said first and/or second parent spinach plant comprises the CMV allele. In a particular embodiment, the first and/or second parent plant is a plant of an inbred line as defined herein.

Further disclosed herein is a hybrid spinach plant grown from seed produced by crossing a first parent spinach plant with a second parent spinach plant and harvesting the resultant hybrid spinach seed, wherein said first and/or second parent spinach plant comprises the alpha-CMV allele.

Furthermore, disclosed herein is a method for identifying or detecting or genotyping or selecting a spinach plant carrying the alpha-CMV allele, comprising determining or detecting or measuring or quantitatively or qualitatively ascertaining or assaying a plant for the presence (or absence) of a genomic nucleotide sequence, wherein said sequence has in order of increased preference 80%, 81%, 82%, 83%, 84%,85%, 86%, 87%, 88%, 89%, 90%, 91%, 92%, 93%, 94%, 95%, 96%, 97%, 98%, 99%, 100% sequence similarity to SEQ ID No:1. Also disclosed herein is characterizing a spinach plant as carrying or not carrying the alpha-CMV allele comprising determining or detecting or measuring or quantitatively or qualitatively ascertaining or assaying a plant for the presence (or absence) of a genomic nucleotide sequence, wherein said sequence has in order of increased preference 80%, 81%, 82%, 83%, 84%,85%, 86%, 87%, 88%, 89%, 90%, 91%, 92%, 93%, 94%, 95%, 96%, 97%, 98%, 99%, 100% sequence similarity to SEQ ID No:1; said method may include extracting or obtaining DNA or RNA of the plant and analysis thereof as to the presence or absence therein of a nucleotide sequence, wherein said sequence has in order of increased preference 80%, 81%, 82%, 83%, 84%,85%, 86%, 87%, 88%, 89%, 90%, 91%, 92%, 93%, 94%, 95%, 96%, 97%, 98%, 99%, 100% sequence similarity to SEQ ID No:1. By using the sequence information and the methods described herein, the skilled person is able to identify or detect or genotype or select a spinach plant carrying the alpha-CMV allele, by using SEQ ID No:21, wherein said sequence has in order of increased preference 80%, 81%, 82%, 83%, 84%,85%, 86%, 87%, 88%, 89%, 90%, 91%, 92%, 93%, 94%, 95%, 96%, 97%, 98%, 99%, 100% sequence similarity to SEQ ID No:21. It is further understood that within the ambit of the invention one can also analyze expression product(s) such as a coding sequence or protein, of a spinach plant to ascertain that from which the product(s) was/were expressed, from which it can be determined whether the plant has (or does not have) a genomic nucleotide sequence, wherein said sequence has in order of increased preference 80%, 81%, 82%, 83%, 84%, 85%, 86%, 87%, 88%, 89%, 90%, 91%, 92%, 93%, 94%, 95%, 96%, 97%, 98%, 99%, 100% sequence similarity to SEQ ID No:1. Such a coding sequence would have in order of increased preference 95%, 96%, 97%, 98%, 99%, 100% sequence similarity to SEQ ID No:2 and/or SEQ ID No:3, whereas a protein has an amino acid sequence which has in order of increased preference 95%, 96%, 97%, 98%, 99%, 100% sequence similarity to SEQ ID No:4 and/or to SEQ ID No:5.

In one embodiment, a method for identifying or selecting a spinach plant carrying the alpha-CMV allele comprises determining the presence of a genomic nucleotide sequence in the genome of a plant, wherein said sequence has in order of increased preference 80%, 81%, 82%, 83%, 84%, 85%, 86%, 87%, 88%, 89%, 90%, 91%, 92%, 93%, 94%, 95%, 96%, 97%, 98%, 99%, 100% sequence similarity to SEQ ID No:1.

Also disclosed herein is a method for identifying or selecting a spinach plant carrying the alpha-CMV allele comprising determining the presence of a genomic nucleotide sequence or a part thereof in the genome of a plant, wherein said sequence has in order of increased preference 80%, 81%, 82%, 83%, 84%, 85%, 86%, 87%, 88%, 89%, 90%, 91%, 92%, 93%, 94%, 95%, 96%, 97%, 98%, 99%, 100% sequence similarity to SEQ ID No:21.

The invention further relates to a method for identifying or selecting a spinach plant carrying the alpha-CMV allele comprising determining the presence of a coding sequence or a part thereof in the genome of a plant, wherein said sequence has in order of increased preference 95%, 96%, 97%, 98%, 99%, 100% sequence similarity to SEQ ID No:2.

The invention further relates a method for identifying or selecting a spinach plant carrying the alpha-CMV allele comprising determining the presence of a coding sequence or a part thereof in the genome of a plant, wherein said sequence has in order of increased preference 94%, 95%, 96%, 97%, 98%, 99%, 100% sequence similarity to SEQ ID No:3.

Determining the genomic DNA or coding DNA sequence of at least part of a *WOLF* gene, to which the alpha-CMV allele belongs, in the genome of a spinach plant may be performed using any suitable molecular biological method known in the art, including but not limited to (genomic) PCR amplification followed by Sanger sequencing, whole-genome-sequencing, transcriptome sequencing, sequence-specific target capture followed by next-generation sequencing (using, for example, the xGen^{®} target capture system of Integrated DNA Technologies), specific amplification of LRR-domain-comprising gene sequences (using, for example, the Resistance Gene Enrichment Sequencing (RenSeq) methodology, as described in Jupe et al., 2013, Plant J. 76: 530-544) followed by sequencing, etcetera.

In another embodiment, the invention relates to a method for identifying or selecting a plant carrying the alpha-CMV allele comprises determining the DNA sequence coding for the LRR domain as defined herein.

In a further embodiment of the method, the LRR domain of the alpha-CMV allele is determined by using a primer pair to amplify the genomic DNA region of the LRR domain. The forward primer is preferably a nucleic acid molecule comprising the sequence of SEQ ID No:6 and the reverse primer is preferably a nucleic acid molecule comprising the sequence of SEQ ID No:7.

Further disclosed herein is a method for producing a spinach plant comprising resistance to CMV comprising: (a) crossing a plant comprising the alpha-CMV allele, with another plant; (b) optionally performing one or more rounds of selfing and/or crossing; (c) optionally selecting after each round of selfing or crossing for a plant that comprises the alpha-CMV allele.

Selecting a plant comprising the alpha-CMV allele may be done genotypically by determining the presence of the genomic DNA sequence of the allele having in order of increased preference 80%, 81%, 82%, 83%, 84%,85%, 86%, 87%, 88%, 89%, 90%, 91%, 92%, 93%, 94%, 95%, 96%, 97%, 98%, 99%, 100% sequence similarity to SEQ ID No: 1.

Selecting a plant comprising the alpha-CMV allele may be done genotypically by determining the presence of the genomic DNA sequence of the allele having in order of increased preference 80%, 81%, 82%, 83%, 84%,85%, 86%, 87%, 88%, 89%, 90%, 91%, 92%, 93%, 94%, 95%, 96%, 97%, 98%, 99%, 100% sequence similarity to SEQ ID No:21.

In another embodiment, selecting a plant comprising the alpha-CMV allele may be done genotypically by determining the presence the coding sequence of an allele having in order of increased preference 95%, 96%, 97%, 98%, 99%, 100% sequence similarity to SEQ ID No:2.

In another embodiment, selecting a plant comprising the alpha-CMV allele may be done genotypically by determining the presence the coding sequence of the allele having in order of increased preference 94%, 95%, 96%, 97%, 98%, 99%, 100% sequence similarity to SEQ ID No:3.

Alternatively, the presence of the alpha-CMV allele may be determined phenotypically by assaying a plant in a disease test, for example the test as described herein, and identifying or selecting a plant carrying the alpha-CMV allele based on the absence of symptoms as described herein.

Furthermore disclosed herein is the use of a spinach plant carrying the alpha-CMV allele in breeding to confer resistance against CMV.

Additionally disclosed herein is a breeding method for the development of spinach plants carrying the alpha-CMV allele of the invention wherein germplasm which comprises said allele is used. Seed capable of growing into a plant comprising the allele of the invention and being representative for the germplasm was deposited with the NCIMB under deposit number NCIMB 42651.

Further disclosed herein is a method for the production of a spinach plant which comprises the alpha-CMV allele, which method comprises: (a) crossing a plant comprising the allele with another plant; (b) optionally selecting for plants comprising said allele in the F1; (c) optionally backcrossing the resulting F1 with the preferred parent and selecting for plants that have the said allele in the BC1F1; (d) optionally performing one or more additional rounds of selfing, crossing, and/or backcrossing, and subsequently selecting for a plant which comprises the said allele or shows resistance to CMV as conferred by said allele. Also disclosed herein is a spinach plant produced by this method.

Additionally disclosed herein is the use of a spinach plant, of which representative seed was deposited with the NCIMB under accession number NCIMB 42651, in the production of a spinach plant comprising the alpha-CMV allele.

Furthermore, disclosed herein is a harvested leaf of a spinach plant as disclosed herein and to a food product which comprises a harvested leaf of a spinach plant as disclosed herein, either in natural or in processed form.

Also disclosed herein is the use of a spinach plant comprising the alpha-CMV allele, for consumption.

Spinach leaves are usually sold in packaged form, including without limitation as prepackaged spinach leaves or as processed in a salad comprising said leaves. Mention of such a package is e.g. made in US Patent No. 5,523,136, which provides packaging film, and packages from such packaging film, including such packaging containing leafy produce, and methods for making and using such packaging film and packages, which are suitable for use with the spinach leaves as disclosed herein.

Furthermore disclosed herein is a container which comprises one or more plants as disclosed herein, or one or more spinach plants derived from a plant as disclosed herein, in a growth substrate for harvest of leaves from the plant, in a domestic environment. This way the consumer may pick very fresh leaves for use in salads, when the plant is in a ready-to-harvest condition.

### DEPOSIT INFORMATION

Seeds of a plant comprising the alpha-CMV allele of the invention heterozygously in its genome were deposited with NCIMB Ltd, Ferguson Building, Craibstone Estate, Bucksburn, Aberdeen AB21 9YA, UK, on September 9, 2016, under deposit accession number 42651. The deposit was made pursuant to the terms of the Budapest Treaty. Upon issuance of a patent, all restrictions upon the deposit will be removed, and the deposit is intended to meet the requirements of 37 CFR § 1.801-1.809. The deposit will be irrevocably and without restriction or condition released to the public upon the issuance of a patent. The deposit will be maintained in the depository for a period of 30 years, or 5 years after the last request, or for the effective life of the patent, whichever is longer, and will be replaced if necessary during that period.

### SEQUENCE INFORMATION

**Table 2. Sequence information.**

| | |
|---|---|
| SEQ ID No:!: | TTTGAAGTTAGGCTTAACTTGCTCACCAATTCTTAAATAGACTCGA CTTACACTGAATTTATTGTGTACTATGTCTAGAAAGTAAGGTCAAC AACTTTCTTCTAAATTATTTGGCATGTTATTAGGGTTATTATGAA AAACATATCAAATTGGTGTTGTTAGTTAGGCTTGAATAATATGATT TTAAGTCACGAGACTTTATAAATTAGGTAATTTGATTTAAAAAAT TGTTACATATCTAAGAAAATGGATGTTAAATTTATCAGTCAATGTA TAAACAATAAAAGTCAATATGTATGTAAAAGGGTTGCTAGATAAA ATCTTTGGTTTTTGGTCCACAACTCCACACTAAGAGGAACTCCAA TGCTTAGTTACAAGGTGGTGTAGTTAAAAAGTAACTCCAATAGTT AACTACACGGTATTATAGTTAAATTTGCCAACTCAAATTTCTAACT ACAATATATTTAAGCTACAAAGTTTCTCATTGGCTGACTACAATAC GTTGTAGCGCCTTATAATATTTATTCAATATACAATTTTATTTATT TTACCTTTTTAACAATTTTTTTTGATCTACCTGCTGTCCTGTTCAT ATGAGCTACACTAATTTGATAGCTGCTTACGCAATTCTTATATCAA CGGTTGGCTACTTGTTCAAATATTTTTATTTTTTTACGAGTAAGTCA TTTTATGATCATTGAAGTGGCTCTATTATTATTATCATGCACCGATT AACGCAAGAATAATTAACTCGGTACGAATTAGTTTCAAAATAAAA TCCCTCAAAAAAAAAAGTTTCAAAATAAAATTAACAGAAAACCAA CCTTCTCCGGTTTACTGTTGTTAGAGCATGGAATTTTCCAGTAATC GCAGACCCCAAATTATCTTCCAGTTGAATCAATCCTTGATTTTTGG ATTTGCCAGAAAACTCCTTGAATTTTAGGGTTCATATTTGATCCGT AATTGGGAAAATTTTCAGCAATTGATCTTCCAAATCAGCCCTACTT GTTTCCAGACTGCAAATGAAAGGTGCGAACTTTATACTGCATTTTG GTTTTCCATTAGTGTAATTTATTAAGATGAACTGCATTTTGCAATT GTTTTATTCGACTACTCATTTTTAAATCAAATTGCTTAATTGCTAGT TAGTTTTCTTATCATATTGCCAAAAAAAATTATTTTTGTTTAATTGG TGAAAAAGGGTAAATTATACCTAGTGTACAAGATTTCTTGCACA CCACCGTTAATTTGTTGACACATCATCAAACGTACTGAAAAATGA GAATGAAAGACAATAAATATGTCATTTTAACCAATAGAAAACAT GATGTAGTAAGATCCTTAATTGATAGATAAATAATTAAATATCAG TCCATTAGTTGAATATTCAATGAAAATGTATGGTCCAAAAATGGC GTTTAATAGTCAATGTCATGCTTTATGGGGTGGTGGAGTACTATGT GACTGTGTGTGGACTTGGAGAAGACTAGAGAGTATGATTATCAAC CTATGGACCCTCAAAATGAAAATGAAAATGATGTTTACACGTGCT ATTTGCACGGACTTCAATGCAAATAGTATAAATTTACGGTCAAAG TTTTCATTCTAAAGCGTAAATAACTTTCATGAATGGAGGACGGTAG TATAAGTATAACGTTATAGCCTACCATTTTCTTATCATATTCACAT AAATTTGTTGCTGAAATTTGTTTTACTTGGCTAAAATACTTTTGTTC TTATTGGCAGATAAACATCAGTACGTCCATTATTGGCCAACTTGCT GAGTCCATTATTGGCCAACTTGAACATATACCTCCAAACAATAATC AATAATGTCGATTATGAAGTTTGTGAATGCAATTTATTATCACTTT CATTTATAAAATGACTACTTGATTAACACATACAATATTACCTTTC TCCAAACACCCTTTCAATTCTGCTTAATCTTGTTTTCTCATCATCTC TTCATCTTTCTGAAAACACAACCCAATGGCCGAAATCGGATACTC GGTTTGTGCGAAACTCATCGAAGTGATTGGCAGTGAGCTGATCAA AGAGATTTGTGACACATGGGGTTACAAATCTCTTCTTGAGGACCTC AACAAAACTGTATTGACGGTCAGGAACGTTCTCATTCAAGCCGGG GTGATGCGGGAGCTTACTAGTGAACAACAAGGTTTCATTGCAGAC CTTAAAGATGTTGTTTATGATGCTGATGACTTGTTCGACAAGTTAC TCACTCGTGCTGAGCGAAAACAGATTGATGGAAACGAAATCTCTG AAAAGGTACGTCGTTTCTTTTCCTCTAGTAACAAGATCGGTCAAGC |
| Genomic sequence of alpha-CMV (with a 2kb region upstream of the start codon) | |
| | TTACTACATGTCTCGTAAGGTTAAGGAAATTAAGAAGCAGTTGGA TGAAATTGTTGATAGGCATACAAAATTTGGGTTTAGTGCTGAGTTT ATACCTGTTTGTAGGGGAAGGGGAAACGAGAGGGAAACACGTTC ATATATAGATGTCAAGAATATTCTTGGAGGGATAAAGATAAGAA TGATATCATAGATAGGTTGCTTAATCGTAATGGTAATGAAGCTTGT AGTTTCCTGACCATAGTGGGAGCGGGAGGATTGGGAAAAACTGCT CTTGCACAACTTGTGTTCAATGATGAAAGGGTCAAAATTGAGTTCC ATGATTTGAGGTATTGGGTTTGTGTCTCTGATCAAGATGGGGGCCA ATTTGATGTGAAAGAAATCCTTTGTAAGATTTAGAGGTGGTTACT AAGGAGAAAGTTGATAATAGTTCCACATTGGAATTGGTACAAAGC CAATTTCAAGAGAAGTTAAGAGGAAAGAAGTACTTCCTTGTTCTT GATGATGTATGGAACGAAGATCGTGAGAAGTGGCTTCCTTTGGAA GAGTTGTTAATGTTGGGTCAAGGGGGAAGCAAGGTTGTAGTGACC ACACGTTCAGAGAAGACAGCAAATGTCATAGGGAAAAGACATTTT TATACACTGGAATGTTTGTCACCAGATTATTCATGGAGCTTATTTG AAATGTCGGCTTTTCAGAAAGGGCATGAGCAGGAAAACCATCACG AACTAGTTGATATTGGGAAAAAGATTGTTGAAAAATGTTATAACA ATCCACTTGCTATAACGGTGGTAGGAAGTCTTCTTTATGGAGAGG AGATAAGTAAGTGGCGGTCATTTGAAATGAGTGAGTTGGCCAAAA TTGGCAATGGGGATAATAAGATTTTGCCGATATTAAAGCTCAGTT ACCATAATCTTATACCCTCGTTGAAGAGTTGTTTTAGTTATTGTGC AGTGTTTCCCAAGGATCATGAAATAAAGAAGGAGATGTTGATTGA ACTTTGGATGGCACAAGGATATGTTGTGCCGTTGGATGGAGGTCA AAGTATAGAAGATGCTGCCGAGGAACATTTTGTAATTTTGTTACG AAGGTGTTTCTTTCAAGATGTAAAGAAGGATAAATATGGTGATGT TGATTCTGTTAAAATCCACGACTTGATGCACGATGTCGCCCAAGA AGTGGGGAGGGAGGAATTATGTGTAGTGAATGATAATACAAAGA ACTTGGGTGATAAAATCCGTCATGTACATCGTGATGTCATTAGATA TGCACAAAGAGTCTCTCTGTGTAGCCATAGCCATAAGATTCGTTCG TATATTGGTGGTAATTGTGAAAAACGTTGTGTGGATACACTAATA GACAAGTGGATGTGTCTTAGGATGTTGGACTTGTCATGGTCGGAT GTTAAAAATTTGCCTAATTCAATAGGTAAATTGTTGCACTTGAGGT GTCTTAACCTGTCTTATAATAAAGATCTGTTGATACTCCCTGATGC AATTACAAGACTGCATAATTTGCAGACACTGCTTTTAAAAGAGTG CAGAAGTTTAAAGGAGTTGCCAAAAGATTTTTGCAAATTGGTCAA ACTGAGGCACTTGGATTTAAGGTGTTCTGATTTGAAGGAGTTGCCA AAAGATTTTTGCAAATTGGTCAAACTGAGGCACTTGGATTTATGG GGTTGTGATGATTTGATTGGTGTGCCATTGGGAATGGATAGGCTA ATTAGTCTTAGAGTACTGCCATTCTTTGTGGTGGGTAGGAAGGAAC AAAGTGATGATGATGAGCTGAAAGCCCTAAAAGGCCTCACCGAGA TAAAAGGCTCCATTCGTATTAGAATCTATTCAAAGTATAGAATAGT TGAAGGCATGAATGACACAGGAGGAGCTGGGTATTTAAAGAGCAT GAAACATCTCACGGGGGTTGATATTACATTTGATGGTGGATGTGTT AACCCTGAAGCTGTGTTGGCAACCCTAGAGCCACCTTCAAATATC AAGAGGTTAGAGATGTGGCATTACAGTGGTACAACAATTCCAGTA TGGGGAAGAGCAGAGATTAATTGGGCAATCTCCCTCTCACATCTT GTCGACATCCAGCTTTGGCATTGTCGTAATTTGCAGGAGATGCCAG TGCTGAGTAAACTGCCTCATTTGAAATCACTGGAACTTTATAATTT GATTAGTTTAGAGTACATGGAGAGCACAAGCAGAAGCAGTAGCA GTGACACAGAAGCAGCAACACCAGAATTACCAACATTCTTCCCTT CCCTTGAAAAACTTACACTTTGGCGTCTGGACAAGTTGAAGGGTTT TGGGAACAGGAGATCGAGTAGTTTCCCCGCCTCTCTAAATTGGA AATCTGGAAATGCCCAGATCTAACGTCATTTCCTTCTTGTCCAAGC CTTGAAGAGTTGGAATTGAAAGAAAACAATGAAGCATTGCAAATA |
| | ATAGTAAAAATAACAACAACAAGAGGTAAAGAAGAAAAAGAAGA AGACAAGAATGCTGGTGTTGGAAATTCACAAGATGATGACAATGT CAAATTATGGACGGTGGAAATAGACAATCTGGGTTATCTCAAATC ACTGCCCACAAATTGTCTTACTCTGTTGGACTCACTCGAACTTTCA AATATAGAAGACCAGGAAGATGAGGGCGAAGACAACATCATATT CTGGAAATCCTTTCCTCAAAACCTCCGCAGTTTGGAAATTGAAAAC TCTTACAAAATGACAAGTTTGCCCATGGGGATGCAGTACTTAACCT CCCTCCAAACCCTCTATCTACACCATTGTTATGAATTGAATTCCCT TCCAGAATGGATAAGCAGCTTATCATCTCTTCAATCCCTGCACATA GGAAAATGTCCAGCCCTAAAATCACTACCAGAAGCAATGCGGAAC CTCACCTCCCTTCAGACACTTGGGATATCGCGGTGTCCAGACCTAA TTGAAAGATGCGAAGAACCCAACGGCGAGGACTATCCCAAAATTC AACACATCCCCAAAATTGTAAGTCATTGCAGAAAGTAATTTATTC ATTTATATTTATTTTATGCTTAGAATGATATACACCGTCGTCCTTTG GTTTCAAATCTTGAATTTGGTTTTGTTTCTTTCTTTGTTTCTTTAT TCAACACCAGCCCATTTATGATTGATTCATTAAAAAAAGGATGGA GTTTTGTGGATTTGAAGAAGACAACGAATTGAGATTCCTGGGGTTT TCTTTTTGTTGGGGTTGGATTTCATGTATATGTTGCTGATTAAATAC GAGACTGATGATGATGATGATGTGTTTATGGGTTTTAAATCAGATT AAATATATGGGAAATGTAAGTTAATTTGGGATGCACATAAGGTGT TTGCTGAAATGTCTATGAGAAATGTTGTTTCTTGGACTTAGAATGA TATACACTGTCGTCCATTGGTTTCCAATCTTACATTTGGTTTGTGTT TTCTTAGTTTGTTTCTTTAATCAACACCAACCCATTTTTTAAAACT ACCTGCAACTACTAATTTACGTTGACCCTGTATCTCAGGTACTAAA TGAATATTGGTGATTTTCAGTTACTCAACACTAGCTTGATCCTGAA CGCACCCAACCTTCAGGTTAGAATCCGGCTTACTCATCCTTTTGTC CAGTTTTCAAGTAATTGTTTTGGCAGGATCAATTCTCTAATTGTTG TACACCGTATATTGCAATTTATAGTGACTACAGTTAATGAATGTTT ACAAAAAATTAGTCATGTAAAAACTTCTTCTCTGTCCATTACATAA ACTCTTTTTCTCTTTCTAACTTATCATGTTCATGTCTAAAAAATTAA ACATGCTCACATCAATGTTCATTTAAGCTAACTTACTTCTGTAAGA GAGCGAGCTAGTTAAAAACTCCTTTAACTTTCTGTTTTATACTCAG GACATGGATTGATGCAAGCATGAAGAACTTCGGGAATTTGCTAAA ACTCTACCAAAGCGATGAGAGTTTGGACTTTGTTTCACTTGAAGTC AGGGACTGTCAACAAAGCCACAGTGTGCATGTTGGCTGTTTCACTT GGACGATAAAAAGGTTTATTTAATTGTTTTCCTAAGTGTATTTGGC TTACAAGCTTTTACTTTTCGCTTGAAAGGGTTTTTCTTGTTTTAAGC TTTTTGAATTAGAGTTTCGGTTGAAGTAAGAGTAGTCGTATTAGTC TTTTACTTTGCAGGAGTCTATGCCTATATAAGGTAAGACTCGTATT TGTAATTTTCAGATTATGCAATTCAAGTTTTCGAGTGTTTTCTTAAA AAAACATATCATACCTGTGTGTAGCATAAAGATAAATTCTGATGC TGTGCTTCTTGTTATGGCTCACATTGGTTTTCATTGTTTGGATTGTT TCACAGGGAAGCAGAGAATACCTGGAACCTGTAAAGGACGCTATA TCTATAGCAGCTTTCACAGGCCAAGTAATATAATGTTCTTTATCAA TCACTCAATACCTGGAGATTGTTTGAACACATAATTCACCTCTTTT TTTCCATCTCAAATTGCAGACTTTTACTGGGATTTGAATGACAGCA AACATTTGATTGTCACGAGGATTGATCTAACTGGTGAATGTTGTGA TCACCATATTGGTCTTTCTCATTCAAACAAACTTGCATACCGTTCTT TTGAAGTTTGAAGACAAGCAGGCGATAATCAACTCAGGTCGAAGC TCATGTGCAGGAGAGAATAGAATATAGAGGAGATTATTTTTAAAA GAAACGCTCAAAGGTATTGTAGCACCTTTCCTATTTGCTACTGCAG TGATGTTTTTATTTCTTGTTTGCAGCGCTTGTTTCTCATATTTCAAC CTACTTTTAGAAAAAAAAACATCTCCGAACATAACCAAAAATAAA GTTCCCTTATCAGTGCTTTCCCTGCTTTCTTCTAAACAACATATACA |
| | ATTATAAACCCTTTTCTCTCTTACCTTTGTTATTCTTCCTTGCTTCA TTGAGATAACACTCTCCTGTTTTGTTTGTTGTTAGTCATTACATG GATATATCAAGGACAACAGTTCTGTAGTCCGTCAACTGTGGTTAG GAAGGCTAAACTGGAGCACAATAACCCCATGTCAATTGAATAGTA AAGGTGTGCTATATCAGTTTCGTTTGGCTTGGCTTACCTGAAAAAT GGCTGGTTATTTATCCTTGTCTCTTTCTATGACGTGCAGTGGCTTGT TAATGTGTCTCGGACAACAATTCCTCACTTTCCAAGTTCCATACAC GCTGATGTAACTATCTTCTGCAGTCTGTTCTTTCATTTTTGCCACGT GCTCTAATTATAACTTTTTGTACTCAATAATCAACTCCTTGTCCCG GTATTTGCAGAGACTACTTAAACAGGTAAAGTGACAATCCTGGCG AAGTTGTCTGTTTCTTAGCTCTGAACCCATCATTCAGGTAAGATTA AGTATTTAAGAAGAAATTTTGTTTTTACCTAAAATGAATGATCTTG TGTAACTGCTTGCTTCTTGCATTAAATAAGAACTTTCTGCTGCATA TGTGACAGTTACATCCACAAAAAAGTTGGAGGTTTGTTCAGGGAT TGGAAATGAAGGTACTTCAGAATTCCTGGAATGTTTATGAATGCTC CAGACTTCAGAGTCTTTAATGGAAAATTCGAGTCACTAAAAAAAC ATTATTCCTATCATCAGAGCTTTGAAGTTCCTCTATACAAGGTCAA CTGAGTTCCTCTTTGCCTCTTGTTTAATTGTATTTACTTGTACCTTA ATTATAATCTGTATATTGTTTTAGTTAAGTTCTAAAACAGGTTATT ATTCATCATTTGTGCAGCATATTGCTGGAATCAAGAATCTAGTGCT TCTTCTTCCTGACTTCACTGTCAAAATAGCAGTTCCCATGCTGGAA ATAAGCGCGCTAGCAATGTAATTGATGACATGGATGTTGCTGCTTC TGAGTTTTGATCATAAAAAGCTGTTATGTGTTTCTTGAATGTAATG AAGGAGAGGAGAAAACTGAAAACTCTTGGCAAAAACGTGAAATT GCAGTGCCTCGGGGTGGTAGGGATCACCCGGATTCAGTTCAGACG ATACTTTTTTCAACCCGGTTTGTTCCGGTTTTCAGCTTCGGGTTTTC CTTGTACTTTTGGGGCACACATACAAGGCTACTTCATGTTTGAGAA ACCTAATTGAGGCTATCTTGTAGCAAATGCACACCACACTCTTTCT CTCTTCTCTCCTTTTTTCACCTTCCATTTGTAAAAATCCTCTTTTAA AGGTTAATAAAAAAAAGCTTCAAGTCTCGTAGGGTGGATGTAGGT CACATTGACCGAACCACGGTAAACTCTTTGTGTTCTTTCTTCTCTCT TTGTTTCTCATTTTTACGGCAAGTGTTTATGGTTAACCATGCATCTT AGAATAGCTTAAGGCATTAACATAATAACATCAATGTTCTCCAAA GATTCACCTTACTTGTTGTACATAATCACAATGTTAAGCCTATGAA GGTAGAATGCTCTCATGATTTGGTTTAACCAAAAAATAAACTCTA AAATAATACGGAGTAATAAAAATTGGCCATAAATTATTTACAAAG TTTGATTTTTGTATAGGGTATCTTGTACTTGTGATAAAAAAAATTA AAAAAAAAAAAATTACTTATTTCTTCTATTTTTACTTGTTACACTTT TCTACAACAGAAACATCAAAACGCCCGCAACACACTATAAATGAA AAACCATTTTGTATGCAATGATATTTACGTTCTCACTTTATTCTCTT TAATAACTCCTACTACGTAATTCTCACCAATCAAATAAAATTATAG AAATTTTCATTTATACCCTCTTAAAATGATGTTGATTTNNNNNNNN NNNNNNNNNNNNNNNNNNNNNNNNNNNNNNNNNNNAGGTGTGAA AAAGTTTGTGTTATAAGTTACAACAATTTAAAAACGGAGAACATA CTTATAATACTAGTGTAATCTTTGGCCGGATTATGGTCGTTGACAA AAAAACTCCGGCCTTGACCCTCCACGTGCCGGTCAAGTGACTTAA TAAACCTTTATTCCACCCTTTTTCATTCTTCTTTTATTCTTCTTT TT |
| SEQ ID No:2: | ATGGCCGAAATCGGATACTCGGTTTGTGCGAAACTCATCGAAGTGATT GGCAGTGAGCTGATCAAAGAGATTTGTGACACATGGGGTTACAAATC TCTTCTTGAGGACCTCAACAAAACTGTATTGACGGTCAGGAACGTTCT CATTCAAGCCGGGGTGATGCGGGAGCTTACTAGTGAACAACAAGGTT TCATTGCAGACCTTAAAGATGTTGTTTATGATGCTGATGACTTGTTCG ACAAGTTACTCACTCGTGCTGAGCGAAAACAGATTGATGGAAACGAA |
| cds alpha-CMV | |
| | ATCTCTGAAAAGGTACGTCGTTTCTTTTCCTCTAGTAACAAGATCGGT CAAGCTTACTACATGTCTCGTAAGGTTAAGGAAATTAAGAAGCAGTT GGATGAAATTGTTGATAGGCATACAAAATTTGGGTTTAGTGCTGAGTT TATACCTGTTTGTAGGGGAAGGGGAAACGAGAGGGAAACACGTTCAT ATATAGATGTCAAGAATATTCTTGGGAGGGATAAAGATAAGAATGAT ATCATAGATAGGTTGCTTAATCGTAATGGTAATGAAGCTTGTAGTTTC CTGACCATAGTGGGAGCGGGAGGATTGGGAAAAACTGCTCTTGCACA ACTTGTGTTCAATGATGAAAGGGTCAAAATTGAGTTCCATGATTTGAG GTATTGGGTTTGTGTCTCTGATCAAGATGGGGGCCAATTTGATGTGAA AGAAATCCTTTGTAAGATTTTAGAGGTGGTTACTAAGGAGAAAGTTG ATAATAGTTCCACATTGGAATTGGTACAAAGCCAATTTCAAGAGAAG TTAAGAGGAAAGAAGTACTTCCTTGTTCTTGATGATGTATGGAACGAA GATCGTGAGAAGTGGCTTCCTTTGGAAGAGTTGTTAATGTTGGGTCAA GGGGGAAGCAAGGTTGTAGTGACCACACGTTCAGAGAAGACAGCAA ATGTCATAGGGAAAAGACATTTTATACACTGGAATGTTTGTCACCAG ATTATTCATGGAGCTTATTTGAAATGTCGGCTTTTCAGAAAGGGCATG AGCAGGAAAACCATCACGAACTAGTTGATATTGGGAAAAAGATTGTT GAAAAATGTTATAACAATCCACTTGCTATAACGGTGGTAGGAAGTCTT CTTTATGGAGAGGAGATAAGTAAGTGGCGGTCATTTGAAATGAGTGA GTTGGCCAAAATTGGCAATGGGGATAATAAGATTTTGCCGATATTAA AGCTCAGTTACCATAATCTTATACCCTCGTTGAAGAGTTGTTTTAGTT ATTGTGCAGTGTTTCCCAAGGATCATGAAATAAAGAAGGAGATGTTG ATTGAACTTTGGATGGCACAAGGATATGTTGTGCCGTTGGATGGAGGT CAAAGTATAGAAGATGCTGCCGAGGAACATTTTGTAATTTTGTTACGA AGGTGTTTCTTTCAAGATGTAAAGAAGGATAAATATGGTGATGTTGAT TCTGTTAAAATCCACGACTTGATGCACGATGTCGCCCAAGAAGTGGG GAGGGAGGAATTATGTGTAGTGAATGATAATACAAAGAACTTGGGTG ATAAAATCCGTCATGTACATCGTGATGTCATTAGATATGCACAAAGA GTCTCTCTGTGTAGCCATAGCCATAAGATTCGTTCGTATATTGGTGGT AATTGTGAAAAACGTTGTGTGGATACACTAATAGACAAGTGGATGTG TCTTAGGATGTTGGACTTGTCATGGTCGGATGTTAAAAATTTGCCTAA TTCAATAGGTAAATTGTTGCACTTGAGGTGTCTTAACCTGTCTTATAA TAAAGATCTGTTGATACTCCCTGATGCAATTACAAGACTGCATAATTT GCAGACACTGCTTTTAAAAGAGTGCAGAAGTTTAAAGGAGTTGCCAA AAGATTTTGCAAATTGGTCAAACTGAGGCACTTGGATTTAAGGTGTT CTGATTTGAAGGAGTTGCCAAAAGATTTTGCAAATTGGTCAAACTGA GGCACTTGGATTTATGGGGTTGTGATGATTTGATTGGTGTGCCATTGG GAATGGATAGGCTAATTAGTCTTAGAGTACTGCCATTCTTTGTGGTGG GTAGGAAGGAACAAAGTGATGATGATGAGCTGAAAGCCCTAAAAGG CCTCACCGAGATAAAAGGCTCCATTCGTATTAGAATCTATTCAAAGTA TAGAATAGTTGAAGGCATGAATGACACAGGAGGAGCTGGGTATTTAA AGAGCATGAAACATCTCACGGGGGTTGATATTACATTTGATGGTGGA TGTGTTAACCCTGAAGCTGTGTTGGCAACCCTAGAGCCACCTTCAAAT ATCAAGAGGTTAGAGATGTGGCATTACAGTGGTACAACAATTCCAGT ATGGGGAAGAGCAGAGATTAATTGGGCAATCTCCCTCTCACATCTTGT CGACATCCAGCTTTGGCATTGTCGTAATTTGCAGGAGATGCCAGTGCT GAGTAAACTGCCTCATTTGAAATCACTGGAACTTTATAATTTGATTAG TTTAGAGTACATGGAGAGCACAAGCAGAAGCAGTAGCAGTGACACAG AAGCAGCAACACCAGAATTACCAACATTCTTCCCTTCCCTTGAAAAAC TTACACTTTGGCGTCTGGACAAGTTGAAGGGTTTTGGGAACAGGAGA TCGAGTAGTTTTCCCCGCCTCTCTAAATTGGAAATCTGGAAATGCCCA GATCTAACGTCATTTCCTTCTTGTCCAAGCCTTGAAGAGTTGGAATTG AAAGAAAACAATGAAGCATTGCAAATAATAGTAAAAATAACAACAA CAAGAGGTAAAGAAGAAAAAGAAGAAGACAAGAATGCTGGTGTTGG AAATTCACAAGATGATGACAATGTCAAATTATGGACGGTGGAAATAG ACAATCTGGGTTATCTCAAATCACTGCCCACAAATTGTCTTACTCTGT |
| | TGGACTCACTCGAACTTTCAAATATAGAAGACCAGGAAGATGAGGGC GAAGACAACATCATATTCTGGAAATCCTTTCCTCAAAACCTCCGCAGT TTGGAAATTGAAAACTCTTACAAAATGACAAGTTTGCCCATGGGGAT GCAGTACTTAACCTCCCTCCAAACCCTCTATCTACACCATTGTTATGA ATTGAATTCCCTTCCAGAATGGATAAGCAGCTTATCATCTCTTCAATC CCTGCACATAGGAAAATGTCCAGCCCTAAAATCACTACCAGAAGCAA TGCGGAACCTCACCTCCCTTCAGACACTTGGGATATCGCGGTGTCCAG ACCTAATTGAAAGATGCGAAGAACCCAACGGCGAGGACTATCCCAAA ATTCAACACATCCCCAAAATTGTACTAAATGAATATTGGTGA |
| SEQ ID No:3: | ATGGCCGAAATCGGATACTCGGTTTGTGCGAAACTCATCGAAGTGATT GGCAGTGAGCTGATCAAAGAGATTTGTGACACATGGGGTTACAAATC TCTTCTTGAGGACCTCAACAAAACTGTATTGACGGTCAGGAACGTTCT CATTCAAGCCGGGGTGATGCGGAGCTTACTAGTGAACAACAAGGTT TCATTGCAGACCTTAAAGATGTTGTTTATGATGCTGATGACTTGTTCG ACAAGTTACTCACTCGTGCTGAGCGAAAACAGATTGATGGAAACGAA ATCTCTGAAAAGGTACGTCGTTTCTTTTCCTCTAGTAACAAGATCGGT CAAGCTTACTACATGTCTCGTAAGGTTAAGGAAATTAAGAAGCAGTT GGATGAAATTGTTGATAGGCATACAAAATTTGGGTTTAGTGCTGAGTT TATACCTGTTTGTAGGGGAAGGGGAAACGAGAGGGAAACACGTTCAT ATATAGATGTCAAGAATATTCTTGGGAGGGATAAAGATAAGAATGAT ATCATAGATAGGTTGCTTAATCGTAATGGTAATGAAGCTTGTAGTTTC CTGACCATAGTGGGAGCGGGAGGATTGGGAAAAACTGCTCTTGCACA ACTTGTGTTCAATGATGAAAGGGTCAAAATTGAGTTCCATGATTTGAG GTATTGGGTTTGTGTCTCTGATCAAGATGGGGGCCAATTTGATGTGAA AGAAATCCTTTGTAAGATTTTAGAGGTGGTTACTAAGGAGAAAGTTG ATAATAGTTCCACATTGGAATTGGTACAAAGCCAATTTCAAGAGAAG TTAAGAGGAAAGAAGTACTTCCTTGTTCTTGATGATGTATGGAACGAA GATCGTGAGAAGTGGCTTCCTTTGGAAGAGTTGTTAATGTTGGGTCAA GGGGGAAGCAAGGTTGTAGTGACCACACGTTCAGAGAAGACAGCAA ATGTCATAGGGAAAAGACATTTTTATACACTGGAATGTTTGTCACCAG ATTATTCATGGAGCTTATTTGAAATGTCGGCTTTTCAGAAAGGGCATG AGCAGGAAAACCATCACGAACTAGTTGATATTGGGAAAAAGATTGTT GAAAAATGTTATAACAATCCACTTGCTATAACGGTGGTAGGAAGTCTT CTTTATGGAGAGGAGATAAGTAAGTGGCGGTCATTTGAAATGAGTGA GTTGGCCAAAATTGGCAATGGGGATAATAAGATTTTGCCGATATTAA AGCTCAGTTACCATAATCTTATACCCTCGTTGAAGAGTTGTTTTAGTT ATTGTGCAGTGTTTCCCAAGGATCATGAAATAAAGAAGGAGATGTTG ATTGAACTTTGGATGGCACAAGGATATGTTGTGCCGTTGGATGGAGGT CAAAGTATAGAAGATGCTGCCGAGGAACATTTTGTAATTTTGTTACGA AGGTGTTTCTTTCAAGATGTAAAGAAGGATAAATATGGTGATGTTGAT TCTGTTAAAATCCACGACTTGATGCACGATGTCGCCCAAGAAGTGGG GAGGGAGGAATTATGTGTAGTGAATGATAATACAAAGAACTTGGGTG ATAAAATCCGTCATGTACATCGTGATGTCATTAGATATGCACAAAGA GTCTCTCTGTGTAGCCATAGCCATAAGATTCGTTCGTATATTGGTGGT AATTGTGAAAAACGTTGTGTGGATACACTAATAGACAAGTGGATGTG TCTTAGGATGTTGGACTTGTCATGGTCGGATGTTAAAAATTTGCCTAA TTCAATAGGTAAATTGTTGCACTTGAGGTGTCTTAACCTGTCTTATAA TAAAGATCTGTTGATACTCCCTGATGCAATTACAAGACTGCATAATTT GCAGACACTGCTTTTAAAAGAGTGCAGAAGTTTAAAGGAGTTGCCAA AAGATTTTTGCAAATTGGTCAAACTGAGGCACTTGGATTTAAGGTGTT CTGATTTGAAGGAGTTGCCAAAAGATTTTTGCAAATTGGTCAAACTGA GGCACTTGGATTTATGGGGTTGTGATGATTTGATTGGTGTGCCATTGG GAATGGATAGGCTAATTAGTCTTAGAGTACTGCCATTCTTTGTGGTGG GTAGGAAGGAACAAAGTGATGATGATGAGCTGAAAGCCCTAAAAGG CCTCACCGAGATAAAAGGCTCCATTCGTATTAGAATCTATTCAAAGTA TAGAATAGTTGAAGGCATGAATGACACAGGAGGAGCTGGGTATTTAA |
| cds of alpha-CMV (isoform 1) | |
| | AGAGCATGAAACATCTCACGGGGGTTGATATTACATTTGATGGTGGA TGTGTTAACCCTGAAGCTGTGTTGGCAACCCTAGAGCCACCTTCAAAT ATCAAGAGGTTAGAGATGTGGCATTACAGTGGTACAACAATTCCAGT ATGGGAAGAGCAGAGATTAATTGGGCAATCTCCCTCTCACATCTTGT CGACATCCAGCTTTGGCATTGTCGTAATTTGCAGGAGATGCCAGTGCT GAGTAAACTGCCTCATTTGAAATCACTGGAACTTTATAATTTGATTAG TTTAGAGTACATGGAGAGCACAAGCAGAAGCAGTAGCAGTGACACAG AAGCAGCAACACCAGAATTACCAACATTCTTCCCTTCCCTTGAAAAAC TTACACTTTGGCGTCTGGACAAGTTGAAGGGTTTTGGGAACAGGAGA TCGAGTAGTTTTCCCCGCCTCTCTAAATTGGAAATCTGGAAATGCCCA GATCTAACGTCATTTCCTTCTTGTCCAAGCCTTGAAGAGTTGGAATTG AAAGAAAACAATGAAGCATTGCAAATAATAGTAAAAATAACAACAA CAAGAGGTAAAGAAGAAAAAGAAGAAGACAAGAATGCTGGTGTTGG AAATTCACAAGATGATGACAATGTCAAATTATGGACGGTGGAAATAG ACAATCTGGGTTATCTCAAATCACTGCCCACAAATTGTCTTACTCTGT TGGACTCACTCGAACTTTCAAATATAGAAGACCAGGAAGATGAGGGC GAAGACAACATCATATTCTGGAAATCCTTTCCTCAAAACCTCCGCAGT TTGGAAATTGAAAACTCTTACAAAATGACAAGTTTGCCCATGGGGAT GCAGTACTTAACCTCCCTCCAAACCCTCTATCTACACCATTGTTATGA ATTGAATTCCCTTCCAGAATGGATAAGCAGCTTATCATCTCTTCAATC CCTGCACATAGGAAAATGTCCAGCCCTAAAATCACTACCAGAAGCAA TGCGGAACCTCACCTCCCTTCAGACACTTGGGATATCGCGGTGTCCAG ACCTAATTGAAAGATGCGAAGAACCCAACGGCGAGGACTATCCCAAA ATTCAACACATCCCCAAAATTTTACTCAACACTAGCTTGATCCTGAAC GCACCCAACCTTCAGGACATGGATTGA |
| SEQ ID No:4: | MAEIGYSVCAKLIEVIGSELIKEICDTWGYKSLLEDLNKTVLTVRNVLIQA GVMRELTSEQQGFIADLKDVVYDADDLFDKLLTRAERKQIDGNEISEKV RRFFSSSNKIGQAYYMSRKVKEIKKQLDEIVDRHTKFGFSAEFIPVCRGRG NERETRSYIDVKNILGRDKDKNDIIDRLLNRNGNEACSFLTIVGAGGLGK TALAQLVFNDERVKIEFHDLRYWVCVSDQDGGQFDVKEILCKILEVVTK EKVDNSSTLELVQSQFQEKLRGKKYFLVLDDVWNEDREKWLPLEELLM LGQGGSKVVVTTRSEKTANVIGKRHFYTLECLSPDYSWSLFEMSAFQKG HEQENHHELVDIGKKIVEKCYNNPLAITVVGSLLYGEEISKWRSFEMSEL AKIGNGDNKILPILKLSYHNLIPSLKSCFSYCAVFPKDHEIKKEMLIELWM AQGYVVPLDGGQSIEDAAEEHFVILLRRCFFQDVKKDKYGDVDSVKIHD LMHDVAQEVGREELCVVNDNTKNLGDKIRHVHRDVIRYAQRVSLCSHS HKIRSYIGGNCEKRCVDTLIDKWMCLRMLDLSWSDVKNLPNSIGKLLHL RCLNLSYNKDLLILPDAITRLHNLQTLLLKECRSLKELPKDFCKLVKLRHL DLRCSDLKELPKDFCKLVKLRHLDLWGCDDLIGVPLGMDRLISLRVLPFF VVGRKEQSDDDELKALKGLTEIKGSIRIRIYSKYRIVEGMNDTGGAGYLK SMKHLTGVDITFDGGCVNPEAVLATLEPPSNIKRLEMWHYSGTTIPVWG RAEINWAISLSHLVDIQLWHCRNLQEMPVLSKLPHLKSLELYNLISLEYM ESTSRSSSSDTEAATPELPTFFPSLEKLTLWRLDKLKGFGNRRSSSFPRLSK LEIWKCPDLTSFPSCPSLEELELKENNEALQIIVKITTTRGKEEKEEDKNAG VGNSQDDDNVKLWTVEIDNLGYLKSLPTNCLTLLDSLELSNIEDQEDEGE DNIIFWKSFPQNLRSLEIENSYKMTSLPMGMQYLTSLQTLYLHHCYELNS LPEWISSLSSLQSLHIGKCPALKSLPEAMRNLTSLQTLGISRCPDLIERCEE PNGEDYPKIQHIPKIVLNEYW* |
| protein sequence of alpha-CMV | |
| SEQ ID No:5: | MAEIGYSVCAKLIEVIGSELIKEICDTWGYKSLLEDLNKTVLTVRNVLIQA GVMRELTSEQQGFIADLKDVVYDADDLFDKLLTRAERKQIDGNEISEKV RRFFSSSNKIGQAYYMSRKVKEIKKQLDEIVDRHTKFGFSAEFIPVCRGRG NERETRSYIDVKNILGRDKDKNDIIDRLLNRNGNEACSFLTIVGAGGLGK TALAQLVFNDERVKIEFHDLRYWVCVSDQDGGQFDVKEILCKILEVVTK EKVDNSSTLELVQSQFQEKLRGKKYFLVLDDVWNEDREKWLPLEELLM LGQGGSKVVVTTRSEKTANVIGKRHFYTLECLSPDYSWSLFEMSAFQKG HEQENHHELVDIGKKIVEKCYNNPLAITVVGSLLYGEEISKWRSFEMSEL |
| protein sequence of alpha-CMV (isoform 1) | |
| | AKIGNGDNKILPILKLSYHNLIPSLKSCFSYCAVFPKDHEIKKEMLIELWM AQGYVVPLDGGQSIEDAAEEHFVILLRRCFFQDVKKDKYGDVDSVKIHD LMHDVAQEVGREELCVVNDNTKNLGDKIRHVHRDVIRYAQRVSLCSHS HKIRSYIGGNCEKRCVDTLIDKWMCLRMLDLSWSDVKNLPNSIGKLLHL RCLNLSYNKDLLILPDAITRLHNLQTLLLKECRSLKELPKDFCKLVKLRHL DLRCSDLKELPKDFCKLVKLRHLDLWGCDDLIGVPLGMDRLISLRVLPFF VVGRKEQSDDDELKALKGLTEIKGSIRIRIYSKYRIVEGMNDTGGAGYLK SMKHLTGVDITFDGGCVNPEAVLATLEPPSNIKRLEMWHYSGTTIPVWG RAEINWAISLSHLVDIQLWHCRNLQEMPVLSKLPHLKSLELYNLISLEYM ESTSRSSSSDTEAATPELPTFFPSLEKLTLWRLDKLKGFGNRRSSSFPRLSK LEIWKCPDLTSFPSCPSLEELELKENNEALQIIVKITTTRGKEEKEEDKNAG VGNSQDDDNVKLWTVEIDNLGYLKSLPTNCLTLLDSLELSNIEDQEDEGE DNIIFWKSFPQNLRSLEIENSYKMTSLPMGMQYLTSLQTLYLHHCYELNS LPEWISSLSSLQSLHIGKCPALKSLPEAMRNLTSLQTLGISRCPDLIERCEE PNGEDYPKIQHIPKILLNTSLILNAPNLQDMD* |
| SEQ ID No:6: | ACAAGTGGATGTGTCTTAGG |
| Forward primer LRR domain (Alpha) | |
| SEQ ID No:7: | TTCGCCCTCATCTTCCTGG |
| Reverse primer LRR domain | |
| SEQ ID No:8: | TCACGTGGGTTGTGTTGT |
| Forward primer LRR domain (Beta) | |
| SEQ ID No:9: | ACAAGTGGATGTGTCTTAGGATGTTGGACTTGTCATGGTCGGATGT TAAAAATTTGCCTAATTCAATAGGTAAATTGTTGCACTTGAGGTGT CTTAACCTGTCTTATAATAAAGATCTGTTGATACTCCCTGATGCAA TTACAAGACTGCATAATTTGCAGACACTGCTTTTAAAAGAGTGCA GAAGTTTAAAGGAGTTGCCAAAAGATTTTTGCAAATTGGTCAAAC TGAGGCACTTGGATTTAAGGTGTTCTGATTTGAAGGAGTTGCCAA AAGATTTTTGCAAATTGGTCAAACTGAGGCACTTGGATTTATGGG GTTGTGATGATTTGATTGGTGTGCCATTGGGAATGGATAGGCTAAT TAGTCTTAGAGTACTGCCATTCTTTGTGGTGGGTAGGAAGGAACA AAGTGATGATGATGAGCTGAAAGCCCTAAAAGGCCTCACCGAGAT AAAAGGCTCCATTCGTATTAGAATCTATTCAAAGTATAGAATAGTT GAAGGCATGAATGACACAGGAGGAGCTGGGTATTTAAAGAGCAT GAAACATCTCACGGGGGTTGATATTACATTTGATGGTGGATGTGTT AACCCTGAAGCTGTGTTGGCAACCCTAGAGCCACCTTCAAATATC AAGAGGTTAGAGATGTGGCATTACAGTGGTACAACAATTCCAGTA TGGGGAAGAGCAGAGATTAATTGGGCAATCTCCCTCTCACATCTT GTCGACATCCAGCTTTGGCATTGTCGTAATTTGCAGGAGATGCCAG TGCTGAGTAAACTGCCTCATTTGAAATCACTGGAACTTTATAATTT GATTAGTTTAGAGTACATGGAGAGCACAAGCAGAAGCAGTAGCA GTGACACAGAAGCAGCAACACCAGAATTACCAACATTCTTCCCTT CCCTTGAAAAACTTACACTTTGGCGTCTGGACAAGTTGAAGGGTTT TGGGAACAGGAGATCGAGTAGTTTCCCCGCCTCTCTAAATTGGA AATCTGGAAATGCCCAGATCTAACGTCATTTCCTTCTTGTCCAAGC CTTGAAGAGTTGGAATTGAAAGAAAACAATGAAGCATTGCAAATA ATAGTAAAAATAACAACAACAAGAGGTAAAGAAGAAAAAGAAGA AGACAAGAATGCTGGTGTTGGAAATTCACAAGATGATGACAATGT CAAATTATGGACGGTGGAAATAGACAATCTGGGTTATCTCAAATC ACTGCCCACAAATTGTCTTACTCTGTTGGACTCACTCGAACTTTCA AATATAGAAGACCAGGAAGATGAGGGCGAA |
| Amplicon of LRR domain of the alpha-CMV allele | |
| SEQ ID No:10: | KWMCLRMLDLSWSDVKNLPNSIGKLLHLRCLNLSYNKDLLILPDAIT RLHNLQTLLLKECRSLKELPKDFCKLVKLRHLDLRCSDLKELPKDFC KLVKLRHLDLWGCDDLIGVPLGMDRLISLRVLPFFVVGRKEQSDDDE LKALKGLTEIKGSIRIRIYSKYRIVEGMNDTGGAGYLKSMKHLTGVDI TFDGGCVNPEAVLATLEPPSNIKRLEMWHYSGTTIPVWGRAEINWAIS LSHLVDIQLWHCRNLQEMPVLSKLPHLKSLELYNLISLEYMESTSRSS SSDTEAATPELPTFFPSLEKLTLWRLDKLKGFGNRRSSSFPRLSKLEIW KCPDLTSFPSCPSLEELELKENNEALQIIVKITTTRGKEEKEEDKNAGV GNSQDDDNVKLWTVEIDNLGYLKSLPTNCLTLLDSLELSNIEDQEDE GE |
| amino acid sequence encoded by amplicon of LRR domain of alpha-CMV | |
| SEQ ID No:11: | TCACGTGGGTTGTGTTGTCGATAGAGATCCAGAAATAGTCTTTTA TGTAGCAATAAGATTCGTTCGTATATTAGCGGTCGCTGCATAAAG AATCCGGTGGATTCACAAATAGACAACTGGATGTGCCTTAGGGTG TTGGACTTGTCAGATTCATGTGTTAAAGATTTGTCTGATTCAATAG GTAAGCTGCTGCACTTAAGGTATCTTAACCTCTCTTCTAATATAAA GTTGGAGATAATCCCTGATGCAATTACAAGACTGCATAACTTGCA GACACTACTTTTAGAAGATTGCAGAAGTTTAAAGGAGTTGCCAAA AGATTTTTGCAAATTGGTCAAACTGAGGCACTTGGAATTACAGGG TTGTCATGATTTGATTGGTATGTCATTTGGAATGGATAAGCTAACT AGTCTTAGAATACTACCAAACATTGTGGTGGGTAGGAAGGAACAA AGTGTTGATGATGAGCTGAAAGCCCTAAAAGGCCTCACCGAGATA AAAGGCTCCATTGATATCACAATCTATTCAAAATATAGAAGAGTT GAAGGCATGAATGGCACAGGAGGAGGAGCTGGGTATTTGAAGAG CATGAAACATCTCACGGGGGTTAATATTACATTTGATGAAGGTGG ATGTGTTAACCCTGAAGCTGTGTATTTGAAGAGCATGAAACATCTC ACGAGGGTTATTATTATATTTGATTATAAAGGTGGATGTGTTAACC CTGAAGCTGTGTTGGCAACCCTAGAGCCACCTTCAAATATCAAGA GGTTAGAGATGTGGCATTACAGTGGTACAACAATTCCAGTATGGG GAAGAGCAGAGATTAATTGGGCAATCTCCCTCTCACATCTTGTCG ACATCACGCTTGAAGATTGTTACAATTTGCAGGAGATGCCAGTGC TGAGTAAACTGCCTCATTTGAAATCACTGGAACTTACAGAGTTGG ATAACTTAGAGTACATGGAGAGTAGAAGCAGCAGCAGTAGCAGT GACACAGAAGCAGCAACACCAGAATTACCAACATTCTTCCCTTCC CTTGAAAAACTTACACTTTGGCGTCTGGACAAGTTGAAGGGTTTTG GGAACAGGAGATCGAGTAGTTTTCCCCGCCTCTCTAAATTGGAAA TCTGGAAATGTCCAGATCTAACGTCATTTCCTTCTTGTCCAAGCCT TGAAGAGTTGGAATTGAAAGAAAACAATGAAGCGTTGCAAATAAT AGTAAAAATAACAACAACAAGAGGTAAAGAAGAAAAAGAAGAA GACAAGAATGCTGGTGTTGGAAATTCACAAGATGATGACAATGTC AAATTATGGAAGGTGGAAATAGACAATCTGGGTTATCTCAAATCA CTGCCCACAAATTGTCTGACTCACCTCGACCTTACAATAAGTGATT CCAAGGAGGGGGAGGGTGAATGGGAAGTTGGGGATGCATTTCAG AAGTGTGTATCTTCTTTGAGAAGCCTCACCATAATCGGAAATCACG GAATAAATAAAGTGAAGAGACTGTCTGGAAGAACAGGGTTGGAG CATTTCACTCTGTTGGAATCACTCAAACTTTCAGATATAGAAGACC AGGAAGATGAGGGCGAA |
| Amplicon of LRR domain of the beta-WOLF 0 allele | |
| SEQ ID No:12: | HVGCVVDRDPEIVFLCSNKIRSYISGRCIKNPVDSQIDNWMCLRVLDL SDSCVKDLSDSIGKLLHLRYLNLSSNIKLEIIPDAITRLHNLQTLLLEDC RSLKELPKDFCKLVKLRHLELQGCHDLIGMSFGMDKLTSLRILPNIVV GRKEQSVDDELKALKGLTEIKGSIDITIYSKYRRVEGMNGTGGGAGY LKSMKHLTGVNITFDEGGCVNPEAVYLKSMKHLTRVIIIFDYKGGCV NPEAVLATLEPPSNIKRLEMWHYSGTTIPVWGRAEINWAISLSHLVDI TLEDCYNLQEMPVLSKLPHLKSLELTELDNLEYMESRSSSSSSDTEAA TPELPTFFPSLEKLTLWRLDKLKGFGNRRSSSFPRLSKLEIWKCPDLTS FPSCPSLEELELKENNEALQIIVKITTTRGKEEKEEDKNAGVGNSQDD DNVKLWKVEIDNLGYLKSLPTNCLTHLDLTISDSKEGEGEWEVGDAF QKCVSSLRSLTIIGNHGINKVKRLSGRTGLEHFTLLESLKLSDIEDQED EGE |
| amino acid sequence encoded by amplicon of LRR domain Beta Wolf 0 (Viroflay) | |
| SEQ ID No:13: | TGGTCCACAACTCCACACTAAGAGGAACTCCAATGCTTAGTTACAAG GTGGTGTAGTTAAAAAGTAATTCCAATAGTTAACTACACGGTATTATA GTTAAATTTGCCAACTCAAATTTCTAACTACAATATATTTAAGCTACA AAGTTTCTCATTGGCTGACTACAATACGTTGTAGCGCCTTATAATATT TTATTCAATATACAATTTTATTTATTTTACCTTTTTAACAATTTTTTTTT GATCTACCTGCTGTCCTGTTCATATGAGCTACACTAATTTGATAGCTG CTTACGCAATTCTTATATCAACGGTTGGCTACTTGTTCAAATATTTTTA TTTTTTTACGAGTAAGTCATTTTATGATCATTGAAGTGGCTCTATTATT ATTATCATGCACCGATTAACGCAAGAATAATTAACTCGGTACGAATTA GTTTCAAAATAAAATCCCTCAAAAAAAAAAGTTTCAAAATAAAATTA ACAGAAAACCAACCTTCTCCGGTTTACTGTTGTTAGAGCATGGAATTT TCCAGTAATCGCAGACCCCAAATTATCTTCCAGTTGAATCAATCCTTG ATTTTTGGATTTGCCAGAAAACTCCTTGAATTTTAGGGTTCATATTTGA TCCGTAATTGGGAAAATTTTCAGCAATTGATCTTCCAAATCAGCCCTA CTTGTTTCCAGACTGCAAATGAAAGGTGCGAACTTTATACTGCATTTT GGTTTTCCATTAGTGTAATTTATTAAGATGAACTGCATTTTGCAATTGT TTTATTCGACTACTCATTTTTAAATCAAATTGCTTAATTGCTAGTTAGT TTTCTTATCATATTGCCAAAAAAAATTATTTTTGTTTAATTGGTGAAAA AGGGTAAATTATACCTAGTGTACAAGATTTTCTTGCACACCACCCTTA ATTTGTTGACACATCATCAAACGTACTGAAAAATGAGAATGAAAGAC AATAAATATGTCATTTTAACCAATAGAAAAACATGATGTAGTAAGAT CCTTAATTGATAGATAAATAATTAAATATCAGTCCATTAGTTGAATAT TCAATGAAAATGTATGGTCCAAAAATGGCGTTTAATAGTCAATGTCAT GCTTTATGGGGTGGTGGAGTACTATGTGACTGTGTGTGGACTTGGAGA AGACTAGAGAGTATGATTATCAACCTATGGACCCTCAAAATGAAAAT GAAAATGATGTTTTTACACTTTAAAATCGTCAAGAAACAACAATCCTT TTTAGCAATAGTATTTACACGCGCTAGTTGCACGAACTTTAATGCAAA TAGTATAAATTTACGGTCAAAGTTTCATACTTTAGACACATACTCTC TCCGTCCCTTAATACTCGCACCGCTTTCCTTTTCGGGCCGTCCCTTAAT ACTTGCACCGCTTCTATAAATGGAAATCTATTTCTCACAATTACCTAC TAACCCACCTACACTCATCGTCCCTACAAAAAATCATTTAAAAATTCA CACCCCACACTCACCACTCCTCACCCATTACACATTTCCTACTAACTA TATTAAAAAAATATCCACTATAAACTAACACTCATTAAATTAATAAG TCAATTCAAATATCTTAAACTCCGCACCGGTCAAATCGGTGCGAGTAT TAAGGGACGGAGGGAGTATTATAGAGTGTAAATAACTTTCATGAATG GAGGGAGTAAAAAATTGTTTTACTTGGCTAAAATACTTTTGTTCTTAT TGGCAGATAAACATGAGTCCATTATTGGCCAACTTGAACATATACCTC CAAACAATAATCAATGATGTCGATTATGAAGTTTGTGAATGCAATTTA TTATCACTTTCATTGCAAGTTGTCATCTGTGCTGAGTGTTGATTTATAA AAAGGACTACTTGATTAACACATACAATATTACTCCACCTTTCTCCAG ACAACCTTTCTTTTCTGCTTTTATCTTGTTTTCTCATCTTAATTCATCTC TTCATCTTTCTGAAAAACCCAACCCAATGGCTGAAATCGGATACTCGG TTTGTTCAAAACTTATTGAAGTGATGGGCAGTAAGATCATTAAAGAG ATTTGTGACATGTGGGGTTACAAATCTCATCTTGAAGACCTCAACAAA |
| Genomic sequence comprising beta-WOLF 3 allele (with a 2kb region upstream of the start codon) | |
| | TCTGTCTTGACGATCAAGGATGTGCTCTTGGATGCTGAGGCGAAGCGG GATCTTTCCCGTGAACAACAGAGTTACATTGCAGAACTTAAGGATGTT GTTTACGATGCTGATGATTTGTTCGATGAGTTCCTCACTCTTGCTGAGC TCAAACAGATTGATGGCAACAACAAGGGTGGTGGTAAATTCTCCAAA AAGGTACGTCGTTTCTTTTCTTCTAATAAGGAGAAGATGGGTCAAGCT TACAAGATGTCTCATATGGTTAAAGAAATTAAGAAGCAGTTGGGTGA AATTGTTGATAGGTATACCAAATTTGGGTTTATTGTTGATTATAAACC TATTATTAGGAGAAGGGAGGAAACATGTTCTTATTTTGTAGGTGCCAA GGAGATTGTTGGGAGGGATAAGGATAAAGATGTTATCATAGGCATGT TGCTAGATCATGATAACGATTGTAGTTTCTTGGCTGTTGTGGGGGTTG GAGGGGTGGGAAAAACTACTCTTGCCCAACTTGTGTATAATGATGAA AGAGTCAAAAGTGAGTTCCAAGATTTGAGGTATTGGGTTTGTGTCTCT GATCAAGATGGGGGACAATTTGATGACAAAAGAATTCTTTGTAAGAT TATAGAGTTAGTTACGGGCCAGATTCCTCCGAGTAACGAGAGCATGG AATCGGTGCGTAAGAAATTTCAAGAGGAATTAGGAGGAAAGAAGTAC TTCCTTGTTCTTGATGATGTATGGAACGAGGATCGCCAGAAGTGGCTT CATCTAGAAAATTTCTTGAAATTGGGTCAAGGGGGAAGCAAGATTGT GGTAACCACACGTTCAGAGAAGACGGCAAATGTTATAGGGAAAAGAC AAGACTATAAACTAGAATGTTTGTCAGCAGAGGATTCATGGCGCTTAT TTGAAATGTCAGCTTTTGACGAAGGGCATGGCCAGGAAAACTATGAC GAATTAGTGACGATTGGCAAGAAGATTGTTGAAAAATGTTATAACAA TCCACTTGCTATAACAGTGGTAGGAAGCCTTCTTTTTGGACAAGAGAT AAATAAGTGGCGGTCGTTTGAAAGCAGTGGATTAGCCCAAATTGCCA ATGGTGATAATCAGATTTTCCCGATATTAAAGCTCAGTTACCACAATC TTCCACACTCCTTGAAGAGCTGCTTTAGCTATTGTGCAGTGTTTCCCA AAGATTATGAAATAAAGAAGGAGATGTTGATTGATCTTTGGATAGCA CAAGGATACATTATACCGTTGGATGGAGGTCAAAGTATAGAAGATGC TGCCGAGGAACATTTTGTAATTTTGTTAAGAAGATGTTTCTTTCAAGA TGTAAAGAAGGATTCTCTTGGTAATGTTGATTATGTTAAAATCCACGA CTTAATGCACGATGTCGCTCAAGAAGTGGGGAAGGAGGAAATCTGTG TAGTGACTTCAGGTACAAAGAAGTTGGCTGATAAAATCCGTCACGTG GGTTGTGTTGTCGATAGAGATCCAGAAATAGTCTTTTTATGTAGCAAT AAGATTCGTTCGTATATTAGCGGTCGTTGTATAAAGAATCCGGTGGAT TCACAAATAGACAACTGGATGCGCCTTAGGGTGTTGGACTTGTCAGAT TCATGTGTTAAAGATTTGTCTGATTCAATAGGTAAGCTGCTGCACTTA AGGTATCTTAACCTCTCTTCTAATATAAAGTTGGAGATAATCCCTGAT GCAATTACAAGACTGCATAACTTGCAGACACTACTTTTAGAATATTGC AGAAGTTTAAAGGAGTTGCCAAAAGATTTTTGCAAATTGGTCAAACT GAGACACTTGGATTTAAGGGGTTGTCAGTGTTTGATTGGTATGCCATT GGGAATGGATAGGCTAATTAGTCTTAGAGTACTACCAAAAGTTGTGG TGGGTAAGAAGGAACAAAGTGATGATCAGCTGAAAGCCCTAAAAGG CCTCACCGAGATAAAAGGCTCCATTGATATCACAATCTATTCAAAGTA TAGAATAGTTGAAGGCATGAATGACACAGGAGGAGCTGGGTATTTGA AGAGCATGAAACATCTCACGGGGGTTGATATTAGATTTGATGATAGA GAAGGTGGATGTGTTAACCCTGAAGCTGTGTTGGCAACCCTAGAGCC ACCTTCAAATATCAAGAGGTTAGAGATGTGGCATTACAGTGGTACAA CAATTCCAGTATGGGGAAGAGCAGAGATTAATTGGGCAATCTCCCTC TCACATCTTGTCGACATCCAGCTTAGTTTTTGTAGAAATTTGCAGGAG ATGCCAGTGCTGAGTAAACTGCCTCATTTGAAATCACTGGAACTTACA GAGTTGGATAACTTAGAGTACATGGAGAGTAGAAGCAGCAGCAGTAG CAGTGACACAGAAGCAGCAACACCAGAATTACCAACATTCTTCCCTT CCCTTGAAAAACTTTCACTTTGGGGTCTGGAAAAGTTGAAGGGTTTGG GGAACAGGAGATCGAGTAGTTTTCCCCGCCTCTCTAAATTGGAAATCT GGGAATGCCCAGATCTAACGTCATTTCCTTCTTGTCCAAGCCTTGAAA AGTTGGAATTGAAAGAAAACAATGAAGCGTTGCAAATAATAGTAAAA ATAACAACAACAAGAGGTAAAGAAGAAAAAGAAGAAGACAAGAATG |
| | CTGGTGTTGGAAATTCACAAGATGATGACAATGTCAAATTATGGAAG GTGGAAATAGACAATCTGGGTTATCTCAAATCACTGCCCACAAATTGT CTTACTCACCTCGACCTTACAATAAGAGATTCCAAGGAGGGGGAGGG TGAATGGAAGTTGGGGAGGCATTTCAGAAGTGTGTATCTTCTTTGAG AAAGCTCAGCATAATCGGAAATCACGGAATAAATAAAGTGAAGAGA CTGTCTGGAAGAACAGGGTTGGAGCATTTCACTCTGTTGGACTCACTC GAACTTTCAAATATAGAAGACCAGGAAGATGAGGGCGAAGACAACA TCATGTTCTGGAAATCCTTTCCTCAAAACCTCCGCAATTTGGAAATTA ATTACTCTGACAAAATGACAAGTTTTCCCATGGGGATGCAGTACTTAA CCTCCCTCCAAACCATCCATCTTTATGATTGTTATAAATTGAATTCCAT TCCAGAATGGATAAGCAGCTTATCATCTCTTCAATCCCTGCACATAGG AAAATGTCCAGCCCTAAAATCACTACCAGAAGCAATGCGGAACCTCA CCTCCCTTCAGAGACTTACGATATGGCAGTGTCCAGACCTAATTGAAA GATGCAAAGAACCTAACGGGGAGGACTATCCCAAAATTGTAAGTCAT TGCAGAAAGTAATTTATTCATTTATATTTATTTTATGCTTAGAATGATA TACGCAGTCGTCCTTTGGTTTCAAATCTTGAATTTGGTTTTTGTTTTCT TTCTTTGTTTCTTTATTCAACACCAGTCCATTTATGATTGATTCATTAA AAAAAGGATGGAGTTTTATGGATTTGAAGAAGACAACGAATTGAGAT TCCTGGGGTTTTTTTTCGTTGGGGTTGGTTTCATGTATATGTTGCTG ATTAAATACCAGACTGATGATGATGATGTGTTTATGGGTTTAAATCA GATTAAATATATGGGAAATGTAAGTTAATTGGGGATGCACATAAGGT GTTTGATGAAATGTCTATTAGAAATGTTGTTTCTTGGACTTAGAATGA TATACACTGTCGTCCTTTGGTTTCCAATCTGGAATTTGGTTTTTGTTTT CTTAGTTTGTTTCTTTATTCCACACTAGCCCATTTTTTTAAACTACCT GCAACTACTGAATTTCATTTACCCTGTATCTCAGATTATATGGTAGTA ATTCTCATTTACTCAACACTAGCTTGATCCTGAACGCAGCCAACCTTC AGGTTAGAATCCGCCTTACTCATCCTTTTGTCATGAATTGTTTTAAGTT GTTTTGCTTGCTTGTGTAATCATAATTCATAGTATACGATTCATCATTC ACTATGTCTATAGGCAAGATATTGGAATTGTTCACGATTTCCTGAAGT TTCTTTGTTTTTGTTGATACCACCATATTGCAGCTTATAGTGACTAAGT TAATGAATGTTTCCAAAAATTAGTCATATAAATTCTTCTTCTCTCTCTA TTACATAAACTCTTTTTCTCTTTCTAACTTATCATGTTCATGTCTAAAA CGTATACATGCTCACATCATTGTTCGTTTCAGCTGACTTACTTATGTAA GAGAGCTATCTAGTTAACAACTCTTGTAACTTTTTATTTGCTAGTCAG AACATGGATTGGTGCAAGCATGGGAATTTGCCAACACTCTACCAAAT CGATTGGAGTTTGGACTTAGTTTCACCAGAAGCCATACCCGGACACTT ACTGGGGACTGTCAACAAAGCCGCATTGTGATGTACTTGGATGTTTCA CGTGCCTGAGGTGTGAGTTACTTGGAAGGGAAGCGGTTTATTTAATTG TTTTCCTAAGTAGATTTTGCTTACAAGCTTTTACTTTTCACTTGGAAGG GTTTTCTTGTTTTAAGCTTTTCGAATTAGAGTTTCGGTTGCATTAAGAG TAGTCGTATTAGTCTTTTTTACCTAAGACTCTTTTTTGTAATTTTCAGA CTATGCAATTCAAGTTTTGAGTGTTTTCTTGCTTGTGTGATTGTGAGTT GGTGAATTCGTCTTTCATACATTTTGAGATTATCAGAAGCTTTATGCTC CACCGGTAGTCTAGTACCTTTTCTGTTACTGTACGTGCAGGGAAGTAA TCTGGTACCTTCTATATATATGGAAAAACATACATTATACATTACGCA AAATTCTTACAGGTTAGTTACTTCCTGGAACTTCATTTACACTTGGTTT TTTTTGTTCCATTCCCTCGGAAGACTATTCCCTCTGAGAAATATGTAAT GAATTTCTGTATTCAGCTGCATTTACAATGAAGTTTAAGCAGACACTC TCTTTATATAGTGCCTCTTTCTGGAGCACCGTAGAGCTGTCTGTGGTTG ATCACCATATGCTGCCGAGAGATTCAGCAATCGCGTGTTTGATCAGGT AAAAGTTTTTATGTCAATGTGTTTTTTTTTTCCGTTTGATCAATTTATG TCTGTATTCAGATTCTTATCTTCTTACAGTAGCATAACACATTGTTTCT TTCATTTATGTAAACTGTTTCAAGATTACAGAGATGTATGCTTCAGTC GACATTGATGATAACTTAAGATGGCATTCCTACAACAGTTGCAGGCG CATTCTAACTCCGGCAATTCTAGTTAGGCAAGAGGAGCATTGCCAATA CCTGCCACCTCTGGGATTTACTATACCAGGGTTGAAGTTTATGGAAGA |
| | CACCAGCTATGCACAAGCCTTCAAGGGGTCATCCTACATAACAAGTT GAACCAACCAATTGCTTGTTGGTTCAGTGGTAATTGGAGCTGAATTCG GTAGGGATGGCCCGTGTTCGATCCCCACAACAACAATTGGGAGGGGA CTGGAACCTATCCACACAGAACTCGCCCTGAATCCGGATTAGCCCTA AGGGTGAACGGGGTGCTAACACCAAAAAAAAACATAACAAGTTGA ATCAAACATACTTTGTTTGAATTGAAGATTTAGTGATTTCATTTGATC GATTGAGATGTCTTATTATAAGCGTATATGCTCTTGGATTTGGCCACT TAGGTGTTGTTTGACAATTGGTCATTAACTCGCTTTTATATTTTCGTTT CTCTTAGGAAAGGTGATCCTGAGAATTTATATTGAAACACTTTTTTTA TCTCTCACTAGCTTTAAAAAAGTGTTCTGTGTTACCTGCAATTCAACTT GATTATTTTTCACATAGTTTTACCTGAAAAAGTGTTATCTGAAAATCA ACTGACATAAATTTTTGTTTGGATCAAATTAAGGATACTAGATAAATC GGAAAAAATAATCAACCAATTAAGTACTTCATAATTAAATATGAAGT ATATTATTATCTTATGCTTGTG |
| SEQ ID No:14: | TTTGAAGTTAGGCTTAACTTGCTCACCAATTCTTAAATAGACTCGACT TACACTGAATTTATTGTGTACTATGTCTAGAAAGTAAGGTCAACAACT TTCTTCTAAATTATTTGGCATGTTTATTAGGGTTATTATGAAAAACAT ATCAAATTGGTGTTGTTAGTTAGGCTTGAATAATATGATTTTAAGTCA CGAGACTTTTATAAATTAGGTAATTTGATTTAAAAAATTGTTACATAT CTAAGAAAATGGATGTTAAATTTATCAGTCAATGTATAAACAATAAA AGTCAATATGTATGTAAAAGGGTTGCTAGATAAAATCTTTGGTTTTTT GGTCCACAACTCCACACTAAGAGGAACTCCAATGCTTAGTTACAAGG TGGTGTAGTTAAAAAGTAACTCCAATAGTTAACTACACGGTATTATAG TTAAATTTGCCAACTCAAATTTCTAACTACAATATATTTAAGCTACAA AGTTTCTCATTGGCTGACTACAATACGTTGTAGCGCCTTATAATATTTT ATTCAATATACAATTTTATTTATTTACCTTTTTAACAATTTTTTTTTGA TCTACCTGCTGTCCTGTTCATATGAGCTACACTAATTTGATAGCTGCTT ACGCAATTCTTATATCAACGGTTGGCTACTTGTTCAAATATTTTTATTT TTTTACGAGTAAGTCATTTTATGATCATTGAAGTGGCTCTATTATTATT ATCATGCACCGATTAACGCAAGAATAATTAACTCGGTACGAATTAGTT TCAAAATAAAATCCCTCAAAAAAAAAAGTTTCAAAATAAAATTAACA GAAAACCAACCTTCTCCGGTTTACTGTTGTTAGAGCATGGAATTTTCC AGTAATCGCAGACCCCAAATTATCTTCCAGTTGAATCAATCCTTGATT TTTGGATTTGCCAGAAAACTCCTTGAATTTAGGGTTCATATTTGATCC GTAATTGGGAAAATTTTCAGCAATTGATCTTCCAAATCAGCCCTACTT GTTTCCAGACTGCAAATGAAAGGTGCGAACTTTATACTGCATTTTGGT TTTCCATTAGTGTAATTTATTAAGATGAACTGCATTTTGCAATTGTTTT ATTCGACTACTCATTTTTAAATCAAATTGCTTAATTGCTAGTTAGTTTT CTTATCATATTGCCAAAAAAAATTATTTTTGTTTAATTGGTGAAAAAG GGTAAATTATACCTAGTGTACAAGATTTTCTTGCACACCACCGTTAAT TTGTTGACACATCATCAAACGTACTGAAAAATGAGAATGAAAGACAA TAAATATGTCATTTTAACCAATAGAAAAACATGATGTAGTAAGATCCT TAATTGATAGATAAATAATTAAATATCAGTCCATTAGTTGAATATTCA ATGAAAATGTATGGTCCAAAAATGGCGTTTAATAGTCAATGTCATGCT TTATGGGGTGGTGGAGTACTATGTGACTGTGTGTGGACTTGGAGAAG ACTAGAGAGTATGATTATCAACCTATGGACCCTCAAAATGAAAATGA AAATGATGTTTACACGTGCTATTTGCACGGACTTCAATGCAAATAGTA TAAATTTACGGTCAAAGTTTCATTCTAAAGCGTAAATAACTTTCATG AATGGAGGACGGTAGTATAAGTATAACGTTATAGCCTACCATTTTCTT ATCATATTCACATAAATTTGTTGCTGAAATTTGTTTTACTTGGCTAAAA TACTTTTGTTCTTATTGGCAGATAAACATCAGTACGTCCATTATTGGCC AACTTGCTGAGTCCATTATTGGCCAACTTGAACATATACCTCCAAACA ATAATCAATAATGTCGATTATGAAGTTTGTGAATGCAATTTATTATCA CTTTCATTTATAAAATGACTACTTGATTAACACATACAATATTACCTTT CTCCAAACACCCTTTCAATTCTGCTTAATCTTGTTTCTCATCATCTCT TCATCTTTCTGAAAACACAACCCAATGGCCGAAATCGGATACTCGGTT |
| Genomic sequence comprising alpha-WOLF 6 allele (with a 2kb region upstream of the start codon) | |
| | TGTGCGAAACTCATCGAAGTGATTGGCAGTGAGCTGATCAAAGAGAT TTGTGACACATGGGGTTACAAATCTCTTCTTGAGGACCTCAACAAAAC TGTATTGACGGTCAGGAACGTTCTCATTCAAGCCGGGGTGATGCGGG AGCTTACTAGTGAACAACAAGGTTTCATTGCAGACCTTAAAGATGTTG TTTATGATGCTGATGACTTGTTCGACAAGTTACTCACTCGTGCTGAGC GAAAACAGATTGATGGAAACGAAATCTCTGAAAAGGTACGTCGTTTC TTTTCCTCTAGTAACAAGATCGGTCAAGCTTACTACATGTCTCGTAAG GTTAAGGAAATTAAGAAGCAGTTGGATGAAATTGTTGATAGGCATAC AAAATTTGGGTTTAGTGCTGAGTTTATACCTGTTTGTAGGGGAAGGGG AAACGAGAGGGAAACACGTTCATATATAGATGTCAAGAATATTCTTG GGAGGGATAAAGATAAGAATGATATCATAGATAGGTTGCTTAATCGT AATGGTAATGAAGCTTGTAGTTTCCTGACCATAGTGGGAGCGGGAGG ATTGGGAAAAACTGCTCTTGCACAACTTGTGTTCAATGATGAAAGGGT CAAAATTGAGTTCCATGATTTGAGGTATTGGGTTTGTGTCTCTGATCA AGATGGGGGCCAATTTGATGTGAAAGAAATCCTTTGTAAGATTTTAG AGGTGGTTACTAAGGAGAAAGTTGATAATAGTTCCACATTGGAATTG GTACAAAGCCAATTTCAAGAGAAGTTAAGAGGAAAGAAGTACTTCCT TGTTCTTGATGATGTATGGAACGAAGATCGTGAGAAGTGGCTTCCTTT GGAAGAGTTGTTAATGTTGGGTCAAGGGGGAAGCAAGGTTGTAGTGA CCACACGTTCAGAGAAGACAGCAAATGTCATAGGGAAAAGACATTTT TATACACTGGAATGTTTGTCACCAGATTATTCATGGAGCTTATTTGAA ATGTCGGCTTTTCAGAAAGGGCATGAGCAGGAAAACCATCACGAACT AGTTGATATTGGGAAAAAGATTGTTGAAAAATGTTATAACAATCCAC TTGCTATAACGGTGGTAGGAAGTCTTCTTTATGGAGAGGAGATAAGT AAGTGGCGGTCATTTGAAATGAGTGAGTTGGCCAAAATTGGCAATGG GGATAATAAGATTTTGCCGATATTAAAGCTCAGTTACCATAATCTTAT ACCCTCGTTGAAGAGTTGTTTAGTTATTGTGCAGTGTTTCCCAAGGA TCATGAAATAAAGAAGGAGATGTTGATTGAACTTTGGATGGCACAAG GATATGTTGTGCCGTTGGATGGAGGTCAAAGTATAGAAGATGCTGCC GAGGAACATTTTGTAATTTTGTTACGAAGGTGTTTCTTTCAAGATGTA AAGAAGGATAAATATGGTGATGTTGATTCTGTTAAAATCCACGACTTG ATGCACGATGTCGCCCAAGAAGTGGGGAGGGAGGAATTATGTGTAGT GAATGATAATACAAAGAACTTGGGTGATAAAATCCGTCATGTACATC GTGATGTCATTAGATATGCACAAAGAGTCTCTCTGTGTAGCCATAGCC ATAAGATTCGTTCGTATATTGGTGGTAATTGTGAAAAACGTTGTGTGG ATACACTAATAGACAAGTGGATGTGTCTTAGGATGTTGGACTTGTCAT GGTCGGATGTTAAAAATTTGCCTAATTCAATAGGTAAATTGTTGCACT TGAGGTGTCTTAACCTGTCTTATAATAAAGATCTGTTGATACTCCCTG ATGCAATTACAAGACTGCATAATTTGCAGACACTGCTTTTAAAAGAGT GCAGAAGTTTAAAGGAGTTGCCAAAAGATTTTTGCAAATTGGTCAAA CTGAGGCACTTGGATTTAAGGTGTTCTGATTTGAAGGAGTTGCCAAAA GATTTTTGCAAATTGGTCAAACTGAGGCACTTGGATTTATGGGGTTGT GATGATTTGATTGGTGTGCCATTGGGAATGGATAGGCTAATTAGTCTT AGAGTACTGCCATTCTTTGTGGTGGGTAGGAAGGAACAAAGTGATGA TGATGAGCTGAAAGCCCTAAAAGGCCTCACCGAGATAAAAGGCTCCA TTCGTATTAGAATCTATTCAAAGTATAGAATAGTTGAAGGCATGAATG ACACAGGAGGAGCTGGGTATTTAAAGAGCATGAAACATCTCACGGGG GTTGATATTACATTTGATGGTGGATGTGTTAACCCTGAAGCTGTGTTG GCAACCCTAGAGCCACCTTCAAATATCAAGAGGTTAGAGATGTGGCA TTACAGTGGTACAACAATTCCAGTATGGGGAAGAGCAGAGATTAATT GGGCAATCTCCCTCTCACATCTTGTCGACATCCAGCTTTGGCATTGTC GTAATTTGCAGGAGATGCCAGTGCTGAGTAAACTGCCTCATTTGAAAT CACTGGAACTTTATAATTTGATTAGTTTAGAGTACATGGAGAGCACAA GCAGAAGCAGTAGCAGTGACACAGAAGCAGCAACACCAGAATTACC AACATTCTTCCCTTCCCTTGAAAAACTTACACTTTGGCGTCTGGACAA GTTGAAGGGTTTTGGGAACAGGAGATCGAGTAGTTTTCCCCGCCTCTC |
| | TAAATTGGAAATCTGGAAATGCCCAGATCTAACGTCATTTCCTTCTTG TCCAAGCCTTGAAGAGTTGGAATTGAAAGAAAACAATGAAGCATTGC AAATAATAGTAAAAATAACAACAACAAGAGGTAAAGAAGAAAAAGA AGAAGACAAGAATGCTGGTGTTGGAAATTCACAAGATGATGACAATG TCAAATTACGGAAGGTGGAAATAGACAATGTGAGTTATCTCAAATCA CTGCCCACAAATTGTCTTACTCACCTCGACCTTACAATAAGAGATTCC AAGGAGGGGGAGGGTGAATGGGAAGTTGGGGATGCATTTCAGAAGT GTGTATCTTCTTTGAGAAGCCTCACCATAATCGGAAATCACGGAATAA ATAAAGTGAAGAGACTGTCTGGAAGAACAGGGTTGGAGCATTTCACT CTGTTGGAATCACTCAAACTTTCAGATATAGAAGACCAGGAAGATGA GGGCGAAGACAACATCATATTCTGGAAATCCTTTCCTCAAAACCTCCG CAGTTTGAGAATTAAAGACTCTGACAAAATGACAAGTTTGCCCATGG GGATGCAGTACTTAACCTCCCTCCAAACCCTCTATCTACACCATTGTT ATGAATTGAATTCCCTTCCAGAATGGATAAGCAGCTTATCATCTCTTC AATCCCTGCACATAGGAAAATGTCCAGCCCTAAAATCACTACCAGAA GCAATGCGGAACCTCACCTCCCTTCAGAGACTTACGATATGGCAGTGT CCAGACCTAATTGAAAGATGCAAAGAACCTAACGGGGAGGACTATCC CAAAATTCAACACATCCCCAAAATTGTAAGTCATTGCAGAAAGTAAT TTATTCATTTATATTTATTTTATGCTTAGAATGATATACACCGTCGTCC TTTGGTTTCAAATCTTGAATTTGGTTTTTGTTTCTTTCTTTGTTTCTTT ATTCAACACCAGCCCATTTATGATTGATTCATTAAAAAAAGGATGGA GTTTTGTGGATTTGAAGAAGACAACGAATTGAGATTCCTGGGGTTTTC TTTTTGTTGGGGTTGGATTTCATGTATATGTTGCTGATTAAATACGAG ACTGATGATGATGATGATGTGTTTATGGGTTTTAAATCAGATTAAATA TATGGGAAATGTAAGTTAATTTGGGATGCACATAAGGTGTTTGCTGAA ATGTCTATGAGAAATGTTGTTTCTTGGACTTAGAATGATATACACTGT CGTCCATTGGTTTCCAATCTTACATTTGGTTTGTGTTTTCTTAGTTTGTT TCTTTAATCAACACCAACCCATTTTTTTAAAACTACCTGCAACTACTA ATTTACGTTGACCCTGTATCTCAGGTACTAAATAAATATTGGTGATTT TCAGTTACTCAACACTAGCTTGATCCTGAACGCACCCAACCTTCAGGT TAGAATCCGGCTTACTCATCCTTTTGTCCAGTTTTCAAGTAATTGTTTT GGCAGGATCAATTCTCTAATTGTTGTACACCGTATATTGCAATTTATA GTGACTACAGTTAATGAATGTTTACAAAAAATTAGTCATGTAAAAACT TCTTCTCTGTCCATTACATAAACTCTTTTTCTCTTTCTAACTTATCATGT TCATGTCTAAAAAATTAAACATGCTCACATCAATGTTCATTTAAGCTA ACTTACTTCTGTAAGAGAGCGAGCTAGTTAAAAACTCCTTTAACTTTC TGTTTTATACTCAGGACATGGATTGATGCAAGCATGAAGAACTTCGGG AATTTGCTAAAACTCTACCAAAGCGATGAGAGTTTGGACTTTGTTTCA CTTGAAGTCAGGGACTGTCAACAAAGCCACAGTGTGCATGTTGGCTG TTTCACTTGGACGATAAAAAGGTTTATTTAATTGTTTTCCTAAGTGTAT TTGGCTTACAAGCTTTTACTTTTCGCTTGAAAGGGTTTTTCTTGTTTTA AGCTTTTTGAATTAGAGTTTCGGTTGAAGTAAGAGTAGTCGTATTAGT CTTTTACTTTGCAGGAGTCTATGCCTATATAAGGTAAGACTCGTATTT GTAATTTTCAGATTATGCAATTCAAGTTTTCGAGTGTTTTCTTAAAAA AACATATCATACCTGTGTGTAGCATAAAGATAAATTCTGATGCTGTGC TTCTTGTTATGGCTCACATTGGTTTTCATTGTTTGGATTGTTTCACAGG GAAGCAGAGAATACCTGGAACCTGTAAAGGACGCTATATCTATAGCA GCTTTCACAGGCCAAGTAATATAATGTTCTTTATCAATCACTCAATAC CTGGAGATTGTTTGAACACATAATTCACCTCTTTTTTTCCATCTCAAAT TGCAGACTTTTACTGGGATTTGAATGACAGCAAACATTTGATTGTCAC GAGGATTGATCTAACTGGTGAATGTTGTGATCACCATATTGGTCTTTC TCATTCAAACAAACTTGCATACCGTTCTTTTGAAGTTTGAAGACAAGC AGGCGATAATCAACTCAGGTCGAAGCTCATGTGCAGGAGAGAATAGA ATATAGAGGAGATTATTTTTAAAAGAAACGCTCAAAGGTATTGTAGC ACCTTTCCTATTTGCTACTGCAGTGATGTTTTTATTTCTTGTTTGCAGC GCTTGTTTCTCATATTTCAACCTACTTTTAGAAAAAAAAACATCTCCG |
| | AACATAACCAAAAATAAAGTTCCCTTATCAGTGCTTTCCCTGCTTTCT TCTAAACAACATATACAATTATAAACCCTTTTCTCTCTTACCTTTGTT ATTCTTCCTTGCTTCATTGAGATAACACTCTCCTGTTTTTGTTTTGTTGT TAGTCATTACATGGATATATCAAGGACAACAGTTCTGTAGTCCGTCAA CTGTGGTTAGGAAGGCTAAACTGGAGCACAATAACCCCATGTCAATT GAATAGTAAAGGTGTGCTATATCAGTTTCGTTTGGCTTGGCTTACCTG AAAAATGGCTGGTTATTTATCCTTGTCTCTTTCTATGACGTGCAGTGG CTTGTTAATGTGTCTCGGACAACAATTCCTCACTTTCCAAGTTCCATAC ACGCTGATGTAACTATCTTCTGCAGTCTGTTCTTTCATTTTTGCCACGT GCTCTAATTATAACTTTTTGTACTCAATAATCAACTCCTTGTCCCGGTA TTTGCAGAGACTACTTAAACAGGTAAAGTGACAATCCTGGCGAAGTT GTCTGTTTCTTAGCTCTGAACCCATCATTCAGGTAAGATTAAGTATTT AAGAAGAAATTTTGTTTTTACCTAAAATGAATGATCTTGTGTAACTGC TTGCTTCTTGCATTAAATAAGAACTTTCTGCTGCATATGTGACAGTTA CATCCACAAAAAAGTTGGAGGTTTGTTCAGGGATTGGAAATGAAGGT ACTTCAGAATTCCTGGAATGTTTATGAATGCTCCAGACTTCAGAGTCT TTAATGGAAAATTCGAGTCACTAAAAAAACATTATTCCTATCATCAGA GCTTTGAAGTTCCTCTATACAAGGTCAACTGAGTTCCTCTTTGCCTCTT GTTTAATTGTATTTACTTGTACCTTAATTATAATCTGTATATTGTTTTA GTTAAGTTCTAAAACAGGTTATTATTCATCATTTGTGCAGCATATTGC TGGAATCAAGAATCTAGTGCTTCTTCTTCCTGACTTCACTGTCAAAAT AGCAGTTCCCATGCTGGAAATAAGCGCGCTAGCAATGTAATTGATGA CATGGATGTTGCTGCTTCTGAGTTTTGATCATAAAAAGCTGTTATGTG TTTCTTGAATGTAATGAAGGAGAGGAGAAAACTGAAAACTCTTGGCA AAAACGTGAAATTGCAGTGCCTCGGGGTGGTAGGGATCACCCGGATT CAGTTCAGACGATACTTTTTTCAACCCGGTTTGTTCCGGTTTTCAGCTT CGGGTTTTCCTTGTACTTTTGGGGCACACATACAAGGCTACTTCATGT TTGAGAAACCTAATTGAGGCTATCTTGTAGCAAATGCACACCACACTC TTTCTCTCTTCTCTCCTTTTTTCACCTTCCATTGTAAAAATCCTCTTTT AAAGGTTAATAAAAAAAAGCTTCAAGTCTCGTAGGGTGGATGTAGGT CACATTGACCGAACCACGGTAAACTCTTTGTGTTCTTTCTTCTCTCTTT GTTTCTCATTTTTACGGCAAGTGTTTATGGTTAACCATGCATCTTAGAA TAGCTTAAGGCATTAACATAATAACATCAATGTTCTCCAAAGATTCAC CTTACTTGTTGTACATAATCACAATGTTAAGCCTATGAAGGTAGAATG CTCTCATGATTTGGTTTAACCAAAAAATAAACTCTAAAATAATACGGA GTAATAAAAATTGGCCATAAACTAATTACAAAGTTTGATTTTTGTATA GGGTATCTTGTACTTGTGATAAAAAAATTTTAAAAAAATTAAAAG TAAATAAATTACTTATTCCTTCTATTTTTACTTGTTACACTTTTCTACA ACAGAAACATCAAAACGCCCGCAACACACTATAAATGAAAAACCATT TTGTATGCAATGATATTTACGTTCTCACTTTATTCTCTTTAATAACTCC TACTACGTAATTCTCACCAATCAAATAAAATTATAGAAATTTTCATTT ATACCCTCTTAAAATGATGTTGATTTTTTTTTTTTTTTTAAGGTGTGA AAAAGTTTGTGTTATACTTACAACAATTTAAAAACGGAGAACATACTT ATAATACTAGTGTAATCTTTGGCCGGATTATGGTCGTTGACAAAAAAA CTCCGGCCTTGACCCTCCACGTGCCGGTCAAGTTCTTCTTTTTTCATTC TTCTTTTTATTCTTCTTTTTCTCTCCATTAATACAAATCAAGTGATTATG TCGATCCGATCCTTCTGTTCTCTACTGTAATTGATTACACCAACAACA ACCAAGCGAAACAGTCAATGTTACCGAATTGAATTGCGGAAAATAGT TTATGATTGATTCATTAAAAAAGGATGGAGTTTTGTGGATTTGAATAA GACAACGAATTGAGATTCCTGGGGTTTCTTTCTGTTGGGGTTGGATT TCATGTACTTGTT |
| SEQ ID No:15: | TTTGAAGTTAGGCTTAACTTGCTCACCAATTCTTAAATAGACTCGACT TACACTGAATTTATTGTGTACTATGTCTAGAAAGTAAGGTCAACAACT TTCTTCTAAATTATTTTGGCATGTTTATTAGGGTTATTATGAAAAACAT ATCAAATTGGTGTTGTTAGTTAGGCTTGAATAATATGATTTTAAGTCA CGAGACTTTTATAAATTAGGTAATTTGATTTAAAAAATTGTTACATAT |
| Genomic sequence | |
| comprising alpha-WOLF 6b allele (with a 2kb region upstream of the start codon) | CTAAGAAAATGGATGTTAAATTTATCAGTCAATGTATAAACAATAAA AGTCAATATGTATGTAAAAGGGTTGCTAGATAAAATCTTTGGTTTTTT GGTCCACAACTCCACACTAAGAGGAACTCCAATGCTTAGTTACAAGG TGGTGTAGTTAAAAGTAACTCCAATAGTTAACTACACGGTATTATAG TTAAATTTGCCAACTCAAATTTCTAACTACAATATATTTAAGCTACAA AGTTTCTCATTGGCTGACTACAATACGTTGTAGCGCCTTATAATATTTT ATTCAATATACAATTTTATTTATTTTACCTTTTTAACAATTTTTTTTGA TCTACCTGCTGTCCTGTTCATATGAGCTACACTAATTTGATAGCTGCTT ACGCAATTCTTATATCAACGGTTGGCTACTTGTTCAAATATTTTTATTT TTTTACGAGTAAGTCATTTTATGATCATTGAAGTGGCTCTATTATTATT ATCATGCACCGATTAACGCAAGAATAATTAACTCGGTACGAATTAGTT TCAAAATAAAATCCCTCAAAAAAAAAGTTTCAAAATAAAATTAACA GAAAACCAACCTTCTCCGGTTTACTGTTGTTAGAGCATGGAATTTTCC AGTAATCGCAGACCCCAAATTATCTTCCAGTTGAATCAATCCTTGATT TTTGGATTTGCCAGAAAACTCCTTGAATTTTAGGGTTCATATTTGATCC GTAATTGGGAAAATTTTCAGCAATTGATCTTCCAAATCAGCCCTACTT GTTTCCAGACTGCAAATGAAAGGTGCGAACTTTATACTGCATTTTGGT TTTCCATTAGTGTAATTTATTAAGATGAACTGCATTTTGCAATTGTTTT ATTCGACTACTCATTTTTAAATCAAATTGCTTAATTGCTAGTTAGTTTT CTTATCATATTGCCAAAAAAAATTATTTTTGTTTAATTGGTGAAAAAG GGTAAATTATACCTAGTGTACAAGATTTTCTTGCACACCACCGTTAAT TTGTTGACACATCATCAAACGTACTGAAAAATGAGAATGAAAGACAA TAAATATGTCATTTTAACCAATAGAAAAACATGATGTAGTAAGATCCT TAATTGATAGATAAATAATTAAATATCAGTCCATTAGTTGAATATTCA ATGAAAATGTATGGTCCAAAAATGGCGTTTAATAGTCAATGTCATGCT TTATGGGGTGGTGGAGTACTATGTGACTGTGTGTGGACTTGGAGAAG ACTAGAGAGTATGATTATCAACCTATGGACCCTCAAAATGAAAATGA AAATGATGTTTACACGTGCTATTTGCACGGACTTCAATGCAAATAGTA TAAATTTACGGTCAAAGTTTTCATTCTAAAGCGTAAATAACTTTCATG AATGGAGGACGGTAGTATAAGTATAACGTTATAGCCTACCATTTTCTT ATCATATTCACATAAATTTGTTGCTGAAATTTGTTTTACTTGGCTAAAA TACTTTTGTTCTTATTGGCAGATAAACATCAGTACGTCCATTATTGGCC AACTTGCTGAGTCCATTATTGGCCAACTTGAACATATACCTCCAAACA ATAATCAATAATGTCGATTATGAAGTTTGTGAATGCAATTTATTATCA CTTTCATTTATAAAATGACTACTTGATTAACACATACAATATTACCTTT CTCCAAACACCCTTTCAATTCTGCTTAATCTTGTTTTCTCATCATCTCT TCATCTTTCTGAAAACACAACCCAATGGCCGAAATCGGATACTCGGTT TGTGCGAAACTCATCGAAGTGATTGGCAGTGAGCTGATCAAAGAGAT TTGTGACACATGGGGTTACAAATCTCTTCTTGAGGACCTCAACAAAAC TGTATTGACGGTCAGGAACGTTCTCATTCAAGCCGGGGTGATGCGGG AGCTTACTAGTGAACAACAAGGTTTCATTGCAGACCTTAAAGATGTTG TTTATGATGCTGATGACTTGTTCGACAAGTTACTCACTCGTGCTGAGC GAAAACAGATTGATGGAAACGAAATCTCTGAAAAGGTACGTCGTTTC TTTTCCTCTAGTAACAAGATCGGTCAAGCTTACTACATGTCTCGTAAG GTTAAGGAAATTAAGAAGCAGTTGGATGAAATTGTTGATAGGCATAC AAAATTTGGGTTTAGTGCTGAGTTTATACCTGTTTGTAGGGGAAGGGG AAACGAGAGGGAAACACGTTCATATATAGATGTCAAGAATATTCTTG GGAGGGATAAAGATAAGAATGATATCATAGATAGGTTGCTTAATCGT AATGGTAATGAAGCTTGTAGTTTCCTGACCATAGTGGGAGCGGGAGG ATTGGGAAAAACTGCTCTTGCACAACTTGTGTTCAATGATGAAAGGGT CAAAATTGAGTTCCATGATTTGAGGTATTGGGTTTGTGTCTCTGATCA AGATGGGGGCCAATTTGATGTGAAAGAAATCCTTTGTAAGATTTTAG AGGTGGTTACTAAGGAGAAAGTTGATAATAGTTCCACATTGGAATTG GTACAAAGCCAATTTCAAGAGAAGTTAAGAGGAAAGAAGTACTTCCT TGTTCTTGATGATGTATGGAACGAAGATCGTGAGAAGTGGCTTCCTTT GGAAGAGTTGTTAATGTTGGGTCAAGGGGGAAGCAAGGTTGTAGTGA |
| | CCACACGTTCAGAGAAGACAGCAAATGTCATAGGAAAAGACATTTT TATACACTGGAATGTTTGTCACCAGATTATTCATGGAGCTTATTTGAA ATGTCGGCTTTTCAGAAAGGGCATGAGCAGGAAAACCATCACGAACT AGTTGATATTGGGAAAAAGATTGTTGAAAAATGTTATAACAATCCAC TTGCTATAACGGTGGTAGGAAGTCTTCTTTATGGAGAGGAGATAAGT AAGTGGCGGTCATTTGAAATGAGTGAGTTGGCCAAAATTGGCAATGG GGATAATAAGATTTTGCCGATATTAAAGCTCAGTTACCATAATCTTAT ACCCTCGTTGAAGAGTTGTTTTAGTTATTGTGCAGTGTTTCCCAAGGA TCATGAAATAAAGAAGGAGATGTTGATTGAACTTTGGATGGCACAAG GATATGTTGTGCCGTTGGATGGAGGTCAAAGTATAGAAGATGCTGCC GAGGAACATTTTGTAATTTTGTTACGAAGGTGTTTCTTTCAAGATGTA AAGAAGGATAAATATGGTGATGTTGATTCTGTTAAAATCCACGACTTG ATGCACGATGTCGCCCAAGAAGTGGGGAGGGAGGAATTATGTGTAGT GAATGATAATACAAAGAACTTGGGTGATAAAATCCGTCATGTACATC GTGATGTCATTAGATATGCACAAAGAGTCTCTCTGTGTAGCCATAGCC ATAAGATTCGTTCGTATATTGGTGGTAATTGTGAAAAACGTTGTGTGG ATACACTAATAGACAAGTGGATGTGTCTTAGGATGTTGGACTTGTCAT GGTCGGATGTTAAAAATTTGCCTAATTCAATAGGTAAATTGTTGCACT TGAGGTGTCTTAACCTGTCTTATAATAAAGATCTGTTGATACTCCCTG ATGCAATTACAAGACTGCATAATTTGCAGACACTGCTTTTAAAAGAGT GCAGAAGTTTAAAGGAGTTGCCAAAAGATTTTTGCAAATTGGTCAAA CTGAGGCACTTGGATTTAAGGTGTTCTGATTTGAAGGAGTTGCCAAAA GATTTTTGCAAATTGGTCAAACTGAGGCACTTGGATTTATGGGGTTGT GATGATTTGATTGGTGTGCCATTGGGAATGGATAGGCTAATTAGTCTT AGAGTACTGCCATTCTTTGTGGTGGGTAGGAAGGAACAAAGTGATGA TGATGAGCTGAAAGCCCTAAAAGGCCTCACCGAGATAAAAGGCTCCA TTCGTATTAGAATCTATTCAAAGTATAGAATAGTTGAAGGCATGAATG ACACAGGAGGAGCTGGGTATTTAAAGAGCATGAAACATCTCACGGGG GTTGATATTACATTTGATGGTGGATGTGTTAACCCTGAAGCTGTGTTG GCAACCCTAGAGCCACCTTCAAATATCAAGAGGTTAGAGATGTGGCA TTACAGTGGTACAACAATTCCAGTATGGGGAAGAGCAGAGATTAATT GGGCAATCTCCCTCTCACATCTTGTCGACATCCAGCTTTGGCATTGTC GTAATTTGCAGGAGATGCCAGTGCTGAGTAAACTGCCTCATTTGAAAT CACTGGAACTTTATAATTTGATTAGTTTAGAGTACATGGAGAGCACAA GCAGAAGCAGTAGCAGTGACACAGAAGCAGCAACACCAGAATTACC AACATTCTTCCCTTCCCTTGAAAAACTTAGACTTTGGCGTCTGGACAA GTTGAAGGGTTTTGGGAACAGGAGATCGAGTAGTTTCCCCGCCTCTC TAAATTGGAAATCTGGAAATGCCCAGATCTAACGTCATTTCCTTCTTG TCCAAGCCTTGAAGAGTTGGAATTGAAAGAAAACAATGAAGCATTGC AAATAATAGTAAAAATAACAACAACAAGAGGTAAAGAAGAAAAAGA AGAAGACAAGAATGCTGGTGTTGGAAATTCACAAGATGATGACAATG TCAAATTACGGAAGGTGGAAATAGACAATGTGAGTTATCTCAAATCA CTGCCCACAAATTGTCTTACTCACCTCGACCTTACAATAAGAGATTCC AAGGAGGGGGAGGGTGAATGGGAAGTTGGGGATGCATTTCAGAAGT GTGTATCTTCTTTGAGAAGCCTCACCATAATCGGAAATCACGGAATAA ATAAAGTGAAGAGACTGTCTGGAAGAACAGGGTTGGAGCATTTCACT CTGTTGGAATCACTCAAACTTTCAGATATAGAAGACCAGGAAGATGA GGGCGAAGACAACATCATATTCTGGAAATCCTTTCCTCAAAACCTCCG CAGTTTGAGAATTAAAGACTCTGACAAAATGACAAGTTTGCCCATGG GGATGCAGTACTTAACCTCCCTCCAAACCCTCTATCTACACCATTGTT ATGAATTGAATTCCCTTCCAGAATGGATAAGCAGCTTATCATCTCTTC AATCCCTGCACATAGGAAAATGTCCAGCCCTAAAATCACTACCAGAA GCAATGCGGAACCTCACCTCCCTTCAGAGACTTACGATATGGCAGTGT CCAGACCTAATTGAAAGATGCAAAGAACCTAACGGGGAGGACTATCC CAAAATTCAACACATCCCCAAAATTGTAAGTCATTGCAGAAAGTAAT TTATTCATTTATATTTATTTTATGCTTAGAATGATATACACCGTCGTCC |
| | TTTGGTTTCAAATCTTGAATTTGGTTTTGTTTCTTTCTTTGTTTCTTT ATTCAACACCAGCCCATTTATGATTGATTCATTAAAAAAAGGATGGA GTTTGTGGATTTGAAGAAGACAACGAATTGAGATTCCTGGGGTTTTC TTTTTGTTGGGGTTGGATTTCATGTATATGTTGCTGATTAAATACGAG ACTGATGATGATGATGATGTGTTTATGGGTTTTAAATCAGATTAAATA TATGGGAAATGTAAGTTAATTTGGGATGCACATAAGGTGTTTGCTGAA ATGTCTATGAGAAATGTTGTTTCTTGGACTTAGAATGATATACACTGT CGTCCATTGGTTTCCAATCTTACATTTGGTTTGTGTTTCTTAGTTTGTT TCTTTAATCAACACCAACCCATTTTTTAAAACTACCTGCAACTACTA ATTTACGTTGACCCTGTATCTCAGGTACTAAATGAATATTGGTGATTT TCAGTTACTCAACACTAGCTTGATCCTGAACGCACCCAACCTTCAGGT TAGAATCCGGCTTACTCATCCTTTTGTCCAGTTTTCAAGTAATTGTTTT GGCAGGATCAATTCTCTAATTGTTGTACACCGTATATTGCAATTTATA GTGACTACAGTTAATGAATGTTTACAAAAAATTAGTCATGTAAAAACT TCTTCTCTGTCCATTACATAAACTCTTTTCTCTTTCTAACTTATCATGT TCATGTCTAAAAAATTAAACATGCTCACATCAATGTTCATTTAAGCTA ACTTACTTCTGTAAGAGAGCGAGCTAGTTAAAAACTCCTTTAACTTTC TGTTTTATACTCAGGACATGGATTGATGCAAGCATGAAGAACTTCGGG AATTTGCTAAAACTCTACCAAAGCGATGAGAGTTTGGACTTTGTTTCA CTTGAAGTCAGGGACTGTCAACAAAGCCACAGTGTGCATGTTGGCTG TTTCACTTGGACGATAAAAAGGTTTATTTAATTGTTTTCCTAAGTGTAT TTGGCTTACAAGCTTTTACTTTTCGCTTGAAAGGGTTTTTCTTGTTTTA AGCTTTTTGAATTAGAGTTTCGGTTGAAGTAAGAGTAGTCGTATTAGT CTTTTACTTTGCAGGAGTCTATGCCTATATAAGGTAAGACTCGTATTT GTAATTTTCAGATTATGCAATTCAAGTTTTCGAGTGTTTTCTTAAAAA AACATATCATACCTGTGTGTAGCATAAAGATAAATTCTGATGCTGTGC TTCTTGTTATGGCTCACATTGGTTTTCATTGTTTGGATTGTTTCACAGG GAAGCAGAGAATACCTGGAACCTGTAAAGGACGCTATATCTATAGCA GCTTTCACAGGCCAAGTAATATAATGTTCTTTATCAATCACTCAATAC CTGGAGATTGTTTGAACACATAATTCACCTCTTTTTTTCCATCTCAAAT TGCAGACTTTTACTGGGATTTGAATGACAGCAAACATTTGATTGTCAC GAGGATTGATCTAACTGGTGAATGTTGTGATCACCATATTGGTCTTTC TCATTCAAACAAACTTGCATACCGTTCTTTTGAAGTTTGAAGACAAGC AGGCGATAATCAACTCAGGTCGAAGCTCATGTGCAGGAGAGAATAGA ATATAGAGGAGATTATTTTAAAAGAAACGCTCAAAGGTATTGTAGC ACCTTTCCTATTTGCTACTGCAGTGATGTTTTTATTTCTTGTTTGCAGC GCTTGTTTCTCATATTTCAACCTACTTTTAGAAAAAAAAACATCTCCG AACATAACCAAAAATAAAGTTCCCTTATCAGTGCTTTCCCTGCTTTCT TCTAAACAACATATACAATTATAAACCCTTTTTCTCTCTTACCTTTGTT ATTCTTCCTTGCTTCATTGAGATAACACTCTCCTGTTTTTGTTTTGTTGT TAGTCATTACATGGATATATCAAGGACAACAGTTCTGTAGTCCGTCAA CTGTGGTTAGGAAGGCTAAACTGGAGCACAATAACCCCATGTCAATT GAATAGTAAAGGTGTGCTATATCAGTTTCGTTTGGCTTGGCTTACCTG AAAAATGGCTGGTTATTTATCCTTGTCTCTTTCTATGACGTGCAGTGG CTTGTTAATGTGTCTCGGACAACAATTCCTCACTTTCCAAGTTCCATAC ACGCTGATGTAACTATCTTCTGCAGTCTGTTCTTTCATTTTTGCCACGT GCTCTAATTATAACTTTTTGTACTCAATAATCAACTCCTTGTCCCGGTA TTTGCAGAGACTACTTAAACAGGTAAAGTGACAATCCTGGCGAAGTT GTCTGTTTCTTAGCTCTGAACCCATCATTCAGGTAAGATTAAGTATTT AAGAAGAAATTTTGTTTTTACCTAAAATGAATGATCTTGTGTAACTGC TTGCTTCTTGCATTAAATAAGAACTTTCTGCTGCATATGTGACAGTTA CATCCACAAAAAAGTTGGAGGTTTGTTCAGGGATTGGAAATGAAGGT ACTTCAGAATTCCTGGAATGTTTATGAATGCTCCAGACTTCAGAGTCT TTAATGGAAAATTCGAGTCACTAAAAAAACATTATTCCTATCATCAGA GCTTTGAAGTTCCTCTATACAAGGTCAACTGAGTTCCTCTTTGCCTCTT GTTTAATTGTATTTACTTGTACCTTAATTATAATCTGTATATTGTTTTA |
| | GTTAAGTTCTAAAACAGGTTATTATTCATCATTTGTGCAGCATATTGC TGGAATCAAGAATCTAGTGCTTCTTCTTCCTGACTTCACTGTCAAAAT AGCAGTTCCCATGCTGGAAATAAGCGCGCTAGCAATGTAATTGATGA CATGGATGTTGCTGCTTCTGAGTTTTGATCATAAAAAGCTGTTATGTG TTTCTTGAATGTAATGAAGGAGAGGAGAAAACTGAAAACTCTTGGCA AAAACGTGAAATTGCAGTGCCTCGGGGTGGTAGGGATCACCCGGATT CAGTTCAGACGATACTTTTTTCAACCCGGTTTGTTCCGGTTTTCAGCTT CGGGTTTTCCTTGTACTTTTGGGGCACACATACAAGGCTACTTCATGT TTGAGAAACCTAATTGAGGCTATCTTGTAGCAAATGCACACCACACTC TTTCTCTCTTCTCTCCTTTTTTCACCTTCCATTTGTAAAAATCCTCTTTT AAAGGTTAATAAAAAAAAGCTTCAAGTCTCGTAGGGTGGATGTAGGT CACATTGACCGAACCACGGTAAACTCTTTGTGTTCTTTCTTCTCTCTTT GTTTCTCATTTTTACGGCAAGTGTTTATGGTTAACCATGCATCTTAGAA TAGCTTAAGGCATTAACATAATAACATCAATGTTCTCCAAAGATTCAC CTTACTTGTTGTACATAATCACAATGTTAAGCCTATGAAGGTAGAATG CTCTCATGATTTGGTTTAACCAAAAAATAAACTCTAAAATAATACGGA GTAATAAAAATTGGCCATAAATTATTTACAAAGTTTGATTTTTGTATA GGGTATCTTG |
| SEQ ID No:16: | ATGGCCGAAATCGGATACTCGGTTTGCGAAACTCATCGAAGTG ATTGGCAGTGAGCTGATCAAAGAGATTTGTGACACATGGGGTTAC AAATCTCTTCTTGAGGACCTCAACAAAACTGTATTGACGGTCAGG AACGTTCTCATTCAAGCCGGGGTGATGCGGGAGCTTACTAGTGAA CAACAAGGTTTCATTGCAGACCTTAAAGATGTTGTTTATGATGCTG ATGACTTGTTCGACAAGTTACTCACTCGTGCTGAGCGAAAACAGA TTGATGGAAACGAAATCTCTGAAAAGGTACGTCGTTTCTTTTCCTC TAGTAACAAGATCGGTCAAGCTTACTACATGTCTCGTAAGGTTAA GGAAATTAAGAAGCAGTTGGATGAAATTGTTGATAGGCATACAAA ATTTGGGTTTAGTGCCGAGTTTATACCTGTTTGTAGGGAAAGGGGG AACGAGAGGGAAACACGTTCATATATAGATGTCAAGAATATTCTT GGGAGGGATAAAGATAAGAATGATATCATAGATAGGTTGCTTAAT CGTAATGGTAATGAAGCTTGTAGTTTCCTGACCATAGTGGGAGCG GGAGGATTGGGAAAAACTGCTCTTGCACAACTTGTGTTCAATGAT GAAAGGGTCAAAATTGAGTTCCATGATTTGAGGTATTGGGTTTGT GTCTCTGATCAAGATGGGGGCCAATTTGATGTGAAAGAAATCCTT TGTAAGATTTTAGAGGTGGTTACTAAGGAGAAAGTTGATAATAGT TCCACATTGGAATTGGTACAAAGCCAATTTCAAGAGAAGTTAAGA GGAAAGAAGTACTTCCTTGTTCTTGATGATGTATGGAACGAAGAT CGTGAGAAGTGGCTTCCTTTGGAAGAGTTGTTAATGTTGGGTCAA GGGGGAAGCAAGGTTGTAGTGACCGCACGTTCAGAGAAGACAGC AAATGTCATAGGGAAAAGACATTTTTATACACTGGAATGTTTGTC ACCAGATTATTCATGGAGCTTATTTGAAATGTCGGCTTTTCAGAAA GGGCATGAGCAGGAAAACCATCACGAACTAGTTGATATTGGGAAA AAGATTGTTGAAAAATGTTATAACAATCCACTTGCTATAACGGTG GTAGGAAGTCTTCTTTATGGAGAGGAGATAAGTAAGTGGCGGTCA TTTGAAATGAGTGAGTTGGCCAAAATTGGCAATGGGGATAATAAG ATTTTGCCGATATTAAAGCTCAGTTACCATAATCTTATACCCTCGT TGAAGAGTTGCTTCAGTTATTGTGCAGTGTTTCCCAAGGATCATGA AATAAAGAAGGAGATGTTGATTGATCTTTGGATAGCACAAGGATA CGTTGTGGCACTTGATGGAGGTCAAAGTATAGAAGATGCTGCCGA AGAACATTTTGTAATTTTGTTACGGAGATGTTTCTTTCAAGATGTA AAGAAGGATGAATATGGTGATGTTGATTCTGTTAAAATCCACGAC TTGATGCACGATGTCGCCCAAGAAGTGGGGAGGGAGGAAATATGT GTAGTGAATGATAATACAAAGAACTTGGGTGATAAAATCCGTCAT GTACATGGTGATGTCAATAGATATGCACAAAGAGTCTCTCTGTGT |
| Genomic sequence of alpha-WOLF 8 allele | |
| | AGCCATAGCCATAAGATTCGTTCGTATATTGGTGGTGATTGTGAAA AACGTTGTGTGGATACACTAATAGACAAGTGGATGTGTCTTAGGA TGTTGGACTTGTCATGGTCGGATGTTAAAAATTTGCCTAATTCAAT AGGTAAATTGTTGCACTTGAGGTATCTTAACCTGTCAGATAATAGA AATCTAAAGATACTTCCTGATGCAATTACAAGACTGCATAATTTGC AGACACTGCTTTTAAAAGATTGCAGAAGTTTAAAGGAGTTGCCAA AAGATTTTTGCAAATTGGTCAAACTGAGACACTTGGATTTATGGG GTTGTGATGATTTGATTGGTATGCCATTTGGAATGGATAAGCTAAC TAGTCTTAGAATACTACCAAACATTGTGGTGGGTAGGAAGGAACA AAGTGTTGATGATGAGCTGAAAGCCCTTAAAGGCCTCACCGAGAT AAAAGGCGACATTGATATCAAAATCTGTGAAAATTATAGAATAGT TGAAGGCATGAATGACACAGGAGGAGCTGGGTATTTGAAGAGCAT GAAACATCTCAGGGAGATTGGTATTACATTTGATGGTGGATGTGTT AACCCTGAAGCTGTGTTGGCAACCCTAGAGCCACCTTCAAATATC AAGAGCTTATCTATACATCGTTTTGATGGTAAAACACTTCCAGTAT GGGGAAGAGCAGAGATTAATTGGGCAATCTCCCTCTCACATCTTG TCGACATCCAGCTTTGGCATTGTCGTAATTTGCAGGAGATGCCAGT GCTGAGTAAACTGCCTCATTTGAAATCACTGGAACTTTATAATTTG ATTAGTTTAGAGTACATGGAGAGCACAAGCAGAAGCAGTAGCAGT GACACAGAAGCAGCAACACCAGAATTACCAACATTCTTCCCTTCC CTTGAAAAACTTACACTTTGGGGTCTGGAAAAGTTGAAGGGTTTG GGAACAGGAGATCGAGTAGTTTTCCCCGCCTCTCTGAATTGAAA ATCATGGAATGCCCAGATCTAACGTGGTTTCCTCCCTGTCCAAGCC TTGAAAAACTTACACTTTGGCGTCTGGACAAGTTGAAGGGTTTTGG GAACCGGAGATCGAGTACTTTTCCCCGCCTCTCTGAATTGGAAATC AAGAAATGCCCAGATCTAACGTCATTTCCTTCTTGTCCAAGCCTTG AGAAGTTGGAATTGAAAGAAAGCAATGAAGCATTGCAAATAATA GTAAAAATAACAACAAGAGGTAAAGAAAAAGAAGAGAACAATAA TGCTGGTGTTAGAAATTCACAAGATGATGACAAAGTCAAATTACG GAAGATGGTGATAGACAATCTGGGTTATCTCAAATCACTGCCCAC AAATTGTCTTACTCACCTCGACCTTACAATAAGTGATTCCAAGGAG GGGGAGGGTGAATGGGAAGTTGGGGATGCATTTCAGAAGTGTGTA TCTTCTTTGAGAAGCCTCACCATAATCGGAAATCACGGAATAAAT AAAGTGAAGAGACTGTCTGGAAGAACAGGGTTGGAGCATTTCACT CTGTTGGAATCACTCAAACTTTCAGATATAGAAGACCAGGAAGAT GAGGGCGAAGACAACATCATATTCTGGAAATCCTTTCCTCAAAAC CTCCGCAGTTTGAGAATTAAAGACTCTGACAAAATGACAAGTTTG CCCATGGGGATGCAGTACTTAACCTCCCTCCAAACCCTCTATCTAC ACCATTGTTATGAATTGAATTCCCTTCCAGAATGGATAAGCAGCTT ATCATCTCTTCAATACCTGCGCATATACTACTGTCCAGCCCTGAAA TCACTACCAGAAGCAATGCGGAACCTCACCTCCCTTCAGACACTT GGGATATCGGATTGTCCAGACCTAGTTAAAAGATGCAGAAAACCC AACGGCAAGGACTATCCCAAAATTCAACACATCCCCTATTGGAGT ATAGAACATCAGGTTATAACTAGCTTGTAACTAACTTGTAACTACC TAGTATAAATACAGTAGTTTGTACTATTTTACATTCAATTACACAA TTAATAAAATGTAGACTCTCACTCTCTCTCTCTAAGCCACGAGCTC CAAGCTCGTCAATGGCTTCCCTTCTCTGTTCTTGCTTTCTTCTTTCC TCTTCAATTCACAAATTCAACATGGTATCAGAGCGGGACGATCCTT GCTCTTCACTTCCGCACAAAATTTTCGTTCAATTCAACCCATCAAA TTTTTTTTTCCCCCAAATTTTCTCGAATTCGGTCAAAATTCGACGA ATTAGGGATTCAATTTACCCTGATTTCTTCTGATTCCATTCAATGAT TGTTCATTTCGAATCTTGAATCAAATAATTGTTGATTCTGGATTCC CCAAATTCTAGGGTTCTTGAAGGATTTACAAGAATCTGGCATTGCT GATAGATTCTTGAAGCAATTTGCGTCTCCGTGTTCCTCGGTGGTCT |
| | TGAGTTTGTTTCCGTATTCGCTGCTCTCATCTTTACTGGGGATTGTG GTCTGATTTCTTGGCTTCCTCTGTCGATGATGTGATTGGTAATACTT AAAACCCCTCTCTCTCTTTCCGAAATTATTGATGCTGGTTCGTCATT TTTTTTTTTGGAATCATCTCAGTTTATCGCCGCAATTTGAGTTGTTG TTGGGTAATTGTTGTTGCTGCCGATGATGTTTTGTGAATTTGAGAA TTGTTAGAATGATTCTTGTTCAATCAATTTGGTTCTCATACTCTAAT GGAAGCCTGTTTTGGAGCGACGAATTATGCAATTCTGAGATTTCTT TTGATCCTTATTTCTTTCTTCACTTGAATTTCTGGTGTTTGTGAGT AATTCTTGGTTAATGTTTGATCTGGGTAGTTCTTGGGTTTACTGAA GACGTTTCTTGAAGGTTTTGACAGAAAAGCTGAGGTTTAATTCCAA AATTCTTCTGTCCAATTACATTTTTATTGTTGATGGTTCTTATGTGA GAACTAGACTGAGTTTTTTTATGAAATTGTTTCGACCTTCAGATG GATTCGAGAGATTTGAGTTCATTTTCTTTGATGAATGTGTTAGAAA AGGTTTTGGTGCAGTGACCATTTTAAACCAAATAGAGTTACATAA ATATTGGGATTCTTTTCTGGGAATGTAGTTAGGAGTTGAAATCTTT TGGAGCTGCTTTACCATAAAACCCAGCCTCAGAGTCTGTTAACCA GTTAGGACCGTGTAAACATGATCCCAGGCTGCATTTGCGTTATCAG ATTTGATTCAGTTTTGGAATTGTGGATTTTGAGGGTTAAAAGCTT ACAGTTGCTCCTGGAGAATGGTGTGAGCAATATAGGAATTCAGCA CTAGTATTGCAGAAAATGAAGCTTGGTTGTTGATTGTTGGCATGTT TTGTTGCCATTGTTTTGGGTTGATGTTTTCCTTTTCTTTTGAATGTT GGCACGATTCAACATTTCTTTCCTGCAACAGATTTGGAGTTCAGTA CCTGTATAATCAGGTCAATTTTGTTCATTTTTCCCAGCAACAGATC TGGAGAATCAGAACCTGTAAAANNNNNNNNNNNNNNNNNNNNNNNN NNNNNNNNNNNNNNNNNNNNNNNNNNNNNNNNNNNNNNNNNACCCA AAGAGGTCAGTTTTCATTGATCCATTGTGATCATTCTTTTGATGAG ACCCATTGAGGCTCATTTCTTCAAGGCAATATTGGAAGTTGTAGAT TGATATGAGCAGTTGGTACAACAGCAACAAAAGTGGCCAGCATCT ATGCTTGTTCATGAGGAGTTCTTGGTGCAGAGTTAATGAAGAGTCT GTTTTGAAGCTTTCAAACTGAAGATGTTTATCACCATCTCCAGTTT GAGGGGGGGTATTGGAGTATAGAACATCAGGTTATAACTAGCTTG TAACTAACTTGTAACTACCTAGTATAAATACAGTAGTTTGTACNNN NNNNNNNNNNNNNNNNNNNNNNNNNNNNNNNNNNNNNNNNNNNNNN NNNNCCACGAGCTCCAAGCTCGTCAATGGCTTCCCTTCTCTGTTCT TGCTTTCTTCTTTCCTCTTCAATTCACAAATTCAACATCCCCAAAAT TGTAAGTCATTGCAGAAAGTAATTTATTCATTTATATTTATTTTATG CTTAGAATGATATACGCAGTCGTCCTTTGGTTTCAAATCTTGAATT TGGTTTTTGTTTCTTTCTTTGTTTCTTTATTCAACACCAGCCCATTT ATGATTGATTCATTAAAAAAAGGATGGAGTTTTATGGATTTGAAG AAGACAACGAATTGAGATTCCTGGGGTTTTCTTTTTGTTGGGGTTG GATTTCATGTATATGTTGCTGATTAAATACGAGACTGATGATGATG ATGTGTTTATGGGTTTTAAATCAGATTAAATATATGGGAAATGCAA GTTAATTTGGGATGCACATAAGGTGTTTGCTGAAATGTCTATGAGA AATGTTGTTTCTTGGACTTAGAATGATATACACTGTCGTCCTTTGG TTTCCAATCTTACATTTGGTTTGTGTTTTCTTAGTTTGTTTCTTTAAT CAACACCAACCCGTTTTTTTAAACTACCTGCAACTACTAATTTAC GTTTACCCTGTATCTCAGGTACTAAATGAATATTGGTGATTTTCAG TTACTCAACACTAGCTTGATCCTGAACGCACCCAACCTTCAGGTTA GAATCCGGCTTACTCATCCTTTTGTCCAGTTTTCAAGTAATTGTTTT GGCAGGATCAATTCTCTAATTGTTGTACACCGTATATTGCAATTTA TAGTGACTACAGTTAATGAATGTTTACAAAAAATTAGTCATGTAA AAACTTCTTCTCTGTCCATTACATAAACTCTTTTTCTCTTTCTAACT TATCATGTTCATGTCTAAACAATTAAACATGCTCACATCAATGTTC ATTTAAGCTAACTTACTTCTGTAAGAGAGCGAGCTAGTTAAAAAC |
| | TCCTTTAACTTTCTGTTTTATACTCAGGACATGGATTGATGCAAGC ATGAAGAACTTCGGGAATTTGCTAAAACTCTACCAAAGCGATGAG AGTTTGGACTTTATTTCACTTGAAGTCAGGGACTGTCAACAAAGCC ACAGTGTGCATGTTGGCTGTTTCACTTGGACGATAAAAAGGTTTAT TTAATTGTTTTCCTAAGTGTATTTGGCTTACAAGCTTTTACTTTTCA CTTGAAAGGGTTTTCTTGTTTTAAGCTTTTCGAATTAGAGTTTTCG GTTGAAGTAAGAGTAGTCGTATTAGTCTTTTACCTAAGGAAGACTC TTTTTTGTAATTTTCAGACTATGCAATTCAAGTTTCGAGTGTTTTC TTGCTTGTGTGATTGTGAGTTGGTGAATTCGTCTTTCATACATTTTG AGATTATCAGAAGCTTTATGCTCCACCGGTAGTCTAGTACCTTTTC TGTTACTGTGCAGGGAAGTAATCTGGTACCTTCTATATATATGGAA AAACATACATTATACATTATGCAAAATTCTTACAGGTTAGTTACTT CCTGGAACTTCATTTACACTTAGTTTTTTTGTTCCATTCCCTCGGA ATCAAGTCATTCCCTCTGAGAAATATGTAATGAACTTCTGTATGTT GCTGTTTGGTTCCTGTTTAATCTTCAATTTCTTGTATAGTTACAG CTGCATTTACAATGAAGTTTAAGCAGACACTCTCTTTATATAGTGC CTCTTTCTGGAGCACCGTAGAGCTGTCTGTGGTTGATCACCATCTG CTGCCGAGAGATTCAGCAATCGCGTGTTTGATCAGGTAAAAGTTTT TATGTCAATGTGTTTTTTTTCCGTTTGATCAATTTATGTCTGTATT CAGATTCTTATCTTCTTACAGTAGCATAACACATTGTTTCTTTCATT TATGTAAACTGTTTCAAGATTACAGAGATGTATGCTTCAGTCGACA TTGATGATAACTTAAGATGGCATTCCTACAACAGTTGCAGGCGCA TTCTAACTCCGGCAATTCTAGTTAGGCAAGAGGAGCATTGCCAAT ACCTGCCACCTCTGGGATTTACTATACCAGGGTTGAAGTTTATGGA AGACACCAGCTATGCACAAGCCTTCAAGGGGTCATCCTACATAAC AAGTTGAACCAACCAATTGCTTGTTGGTTCAGTGGTAATTGAAGCT GAATTTGGTAGGGATGGCCCGTGTTCGATCCCCACAACAACAATT GGGAGGGGACTGGAACCTATCCACACAGAACTCGCCCTGAATCCG GATTAGCCCTAAGGGTGAACGGGGTGCTAACACCAAAAAAAAAA ACATAACAAGTTGAACCAAACATACTTTGTTTGAATTGAAGATTTA GTGATTTCATTTGATCGATTGAGATGTCTTATTATAAGCGTATATG CTCTTGGATTTGGCCACTTAGGTGTTGTTTGACAATTGGACATTAA CTCGCTTTTATATTTTCTTTTCTCTTAGGAAAGGTGATCCTGAGAAT TTATATTGGAACACTTTTTTTTTCTCACTAGCTTTAAAAAAGTGTTC TGTGTTACCTGCAATTCAATTTGATTATTTTTCACATAGTTTTACCT GAAAAAGTGTTACCTGAAAAAGTGTTACCTGAAAATCAACTGACA TAAGTTTTTGTTTGGATCCAATTAAGGACACTAGATAAATCGGAAT AAATAATCAACCAATTAAGTACTTCATAATTAAATATGAAGTGTA TTATTATCTTATGCTTGTGACATTGAAGGATGTTATGATATTTTAA CTCAATACCTTGCAAAATATACTGG |
| SEQ ID No:17: | TTTGAAGTTAGGCTTAACTTGCTCACCAATTCTTAAATAGACTCGACT TACACTGAATTTATTGTGTACTATGTCTAGAAAGTAAGGTCAACAACT TTCTTCTAAATTATTTGGCATGTTTATTAGGGTTATTATGAAAAACAT ATCAAATTGGTGTTGTTAGTTAGGCTTGAATAATATGATTTTAAGTCA CGAGACTTTTATAAATTAGGTAATTTGATTTAAAAAATTGTTACATAT CTAAGAAAATGGATGTTAAATTTATCAGTCAATGTATAAACAATAAA AGTCAATATGTATGTAAAAGGGTTGCTAGATAAAATCTTTGGTTTTTT GGTCCACAACTCCACACTAAGAGGAACTCCAATGCTTAGTTACAAGG TGGTGTAGTTAAAAAGTAACTCCAATAGTTAACTACACGGTATTATAG TTAAATTTGCCAACTCAAATTTCTAACTACAATATATTTAAGCTACAA AGTTTCTCATTGGCTGACTACAATACGTTGTAGCGCCTTATAATATTTT ATTCAATATACAATTTTATTTATTTTACCTTTTTAACAATTTTTTTTTGA TCTACCTGCTGTCCTGTTCATATGAGCTACACTAATTTGATAGCTGCTT ACGCAATTCTTATATCAACGGTTGGCTACTTGTTCAAATATTTTTATTT |
| Genomic sequence comprising alpha-WOLF 9 allele (with a 2kb region upstream of the start codon) | |
| | TTTTACGAGTAAGTCATTTTATGATCATTGAAGTGGCTCTATTATTATT ATCATGCACCGATTAACGCAAGAATAATTAACTCGGTACGAATTAGTT TCAAAATAAAATCCCTCAAAAAAAAAAGTTTCAAAATAAAATTAACA GAAAACCAACCTTCTCCGGTTTACTGTTGTTAGAGCATGGAATTTTCC AGTAATCGCAGACCCCAAATTATCTTCCAGTTGAATCAATCCTTGATT TTTGGATTTGCCAGAAAACTCCTTGAATTTTAGGGTTCATATTTGATCC GTAATTGGGAAAATTTTCAGCAATTGATCTTCCAAATCAGCCCTACTT GTTTCCAGACTGCAAATGAAAGGTGCGAACTTTATACTGCATTTTGGT TTTCCATTAGTGTAATTTATTAAGATGAACTGCATTTTGCAATTGTTTT ATTCGACTACTCATTTTTAAATCAAATTGCTTAATTGCTAGTTAGTTTT CTTATCATATTGCCAAAAAAAATTATTTTTGTTTAATTGGTGAAAAAG GGTAAATTATACCTAGTGTACAAGATTTTCTTGCACACCACCGTTAAT TTGTTGACACATCATCAAACGTACTGAAAAATGAGAATGAAAGACAA TAAATATGTCATTTTAACCAATAGAAAAACATGATGTAGTAAGATCCT TAATTGATAGATAAATAATTAAATATCAGTCCATTAGTTGAATATTCA ATGAAAATGTATGGTCCAAAAATGGCGTTTAATAGTCAATGTCATGCT TTATGGGGTGGTGGAGTACTATGTGACTGTGTGTGGACTTGGAGAAG ACTAGAGAGTATGATTATCAACCTATGGACCCTCAAAATGAAAATGA AAATGATGTTTACACGTGCTATTTGCACGGACTTCAATGCAAATAGTA TAAATTTACGGTCAAAGTTTTCATTCTAAAGCGTAAATAACTTTCATG AATGGAGGACGGTAGTATAAGTATAACGTTATAGCCTACCATTTTCTT ATCATATTCACATAAATTTGTTGCTGAAATTTGTTTTACTTGGCTAAAA TACTTTTGTTCTTATTGGCAGATAAACATCAGTACGTCCATTATTGGCC AACTTGCTGAGTCCATTATTGGCCAACTTGAACATATACCTCCAAACA ATAATCAATAATGTCGATTATGAAGTTTGTGAATGCAATTTATTATCA CTTTCATTTATAAAATGACTACTTGATTAACACATACAATATTACCTTT CTCCAAACACCCTTTCAATTCTGCTTAATCTTGTTTTCTCATCATCTCT TCATCTTTCTGAAAACACAACCCAATGGCCGAAATCGGATACTCGGTT TGTGCGAAACTCATCGAAGTGATTGGCAGTGAGCTGATCAAAGAGAT TTGTGACACATGGGGTTACAAATCTCTTCTTGAGGACCTCAACAAAAC TGTATTGACGGTCAGGAACGTTCTCATTCAAGCCGGGGTGATGCGGG AGCTTACTAGTGAACAACAAGGTTTCATTGCAGACCTTAAAGATGTTG TTTATGATGCTGATGACTTGTTCGACAAGTTACTCACTCGTGCTGAGC GAAAACAGATTGATGGAAACGAAATCTCTGAAAAGGTACGTCGTTTC TTTTCCTCTAGTAACAAGATCGGTCAAGCTTACTACATGTCTCGTAAG GTTAAGGAAATTAAGAAGCAGTTGGATGAAATTGTTGATAGGCATAC AAAATTTGGGTTTAGTGCCGAGTTTATACCTGTTTGTAGGGAAAGGGG GAACGAGAGGGAAACACGTTCATATATAGATGTCAAGAATATTCTTG GGAGGGATAAAGATAAGAATGATATCATAGATAGGTTGCTTAATCGT AATGGTAATGAAGCTTGTAGTTTCCTGACCATAGTGGGAGCGGGAGG ATTGGGAAAAACTGCTCTTGCACAACTTGTGTTCAATGATGAAAGGGT CAAAATTGAGTTCCATGATTTGAGGTATTGGGTTTGTGTCTCTGATCA AGATGGGGGCCAATTTGATGTGAAAGAAATCCTTTGTAAGATTTTAG AGGTGGTTACTAAGGAGAAAGTTGATAATAGTTCCACATTGGAATTG GTACAAAGCCAATTTCAAGAGAAGTTAAGAGGAAAGAAGTACTTCCT TGTTCTTGATGATGTATGGAACGAAGATCGTGAGAAGTGGCTTCCTTT GGAAGAGTTGTTAATGTTGGGTCAAGGGGGAAGCAAGGTTGTAGTGA CCGCACGTTCAGAGAAGACAGCAAATGTCATAGGGAAAAGACATTTT TATACACTGGAATGTTTGTCACCAGATTATTCATGGAGCTTATTTGAA ATGTCGGCTTTTCAGAAAGGGCATGAGCAGGAAAACCATCACGAACT AGTTGATATTGGGAAAAAGATTGTTGAAAAATGTTATAACAATCCAC TTGCTATAACGGTGGTAGGAAGTCTTCTTTATGGAGAGGAGATAAGT AAGTGGCGGTCATTTGAAATGAGTGAGTTGGCCAAAATTGGCAATGG GGATAATAAGATTTTGCCGATATTAAAGCTCAGTTACCATAATCTTAT ACCCTCGTTGAAGAGTTGCTTCAGTTATTGTGCAGTGTTTCCCAAGGA TCATGAAATAAAGAAGGAGATGTTGATTGATCTTTGGATAGCACAAG |
| | GATACGTTGTGGCACTTGATGGAGGTCAAAGTATAGAAGATGCTGCC GAAGAACATTTTGTAATTTGTTACGGAGATGTTTCTTTCAAGATGTA AAGAAGGATGAATATGGTGATGTTGATTCTGTTAAAATCCACGACTTG ATGCACGATGTCGCCCAAGAAGTGGGAGGGAGGAAATATGTGTAGT GAATGATAATACAAAGAACTTGGGTGATAAAATCCGTCATGTACATC GTGATGTCATTAGATATGCACAAAGAGTCTCTCTGTGTAGCCATAGCC ATAAGATTCGTTCGTATATTGGTGGTAATTGTGAAAAACGTTGTGTGG ATACACTAATAGACAAGTGGATGTGTCTTAGGATGTTGGACTTGTCAT GGTCGGATGTTAAAAATTTGCCTAATTCAATAGGTAAATTGTTGCACT TGAGGTGTCTTAACCTGTCTTATAATAAAGATCTGTTGATACTCCCTG ATGCAATTACAAGACTGCATAATTTGCAGACACTGCTTTTAAAAGAGT GCAGAAGTTTAAAGGAGTTGCCAAAAGATTTTTGCAAATTGGTCAAA CTGAGGCACTTGGATTTAAGGTGTTCTGATTTGAAGGAGTTGCCAAAA GATTTTTGCAAATTGGTCAAACTGAGGCACTTGGATTTAAGGTGTTCT GATTTGAAGGAGTTGCCAAAAGATTTTTGCAAATTGGTCAAACTGAG GCACTTGGATTTATGGGGTTGTGATGATTTGATTGGTGTGCCATTGGG AATGGATAGGCTAATTAGTCTTAGAGTACTGCCATTCTTTGTGGTGGG TAGGAAGGAACAAAGTGATGATGATGAGCTGAAAGCCCTAAAAGGC CTCACCGAGATAAAAGGCTCCATTCGTATTAGAATCTATTCAAAGTAT AGAATAGTTGAAGGCATGAATGACACAGGAGGAGCTGGGTATTTAAA GAGCATGAAACATCTCACGGGGGTTGATATTACATTTGATGGTGGAT GTGTTAACCCTGAAGCTGTGTTGGCAACCCTAGAGCCACCTTCAAATA TCAAGAGGTTAGAGATGTGGCATTACAGTGGTACAACAATTCCAGTA TGGGGAAGAGCAGAGATTAATTGGGCAATCTCCCTCTCACATCTTGTC GACATCCAGCTTTGGTGTTGTAGTAATTTGCAGGAGATGCCAGTGCTG AGTAAACTGCCTCATTTGAAATCACTGTATCTTTTTAAGTTTTGTAAGT TAGAGTACATGGAGAGTAGAAGCAGCAGCAGTAGCAGTGACACAGA AGCAGCAACACCAGAATTACCAACATTCTTCCCTTCCCTTGAAAAACT TAGACTTTGGTATCTGGAAAAGTTGAAGGGTTTGGGGAACAGGAGAC CGAGTAGTTTTCCCCGCCTCTCTGAATTGGAAATCTGGGAATGCCCAG ATCTAACGTGGTTTCCTCCCTGTCCAAGCCTTGAAAAACTTACACTTT GGCGTCTGGACAAGTTGAAGGGTTTGGGGAACAGGAGATCGAGTAGT TTTCCCCGCCTCTCTAAATTGGTAATCTGGAAATGCCCAGATCTAACG TGGTTTCCTCCCTGTCCAAGCCTTGAAAAACTTACACTTTGGCGTCTG GACAAGTTGAAGGGTTTGGGGAACAGGAGATCGAGTAGTTTTCCCCG CCTCTCTGAATTGGAAATCAAGAAATGCCCAGATCTAACGTGGTTTCC TCCTTGTCCAAGCCTTGAAACGTTGAAATTGGAAAAAAACAATGAAG CGTTGCAAATAATAGTAAAAATAACAACAACAAGAGGTAAAGAAGA AAAAGAAGAAGACAAGAATGCTGGTGTTGGAAATTCACAAGATGATG ACAATGTCAAATTATGGACGGTGGAAATAGACAATCTGGGTTATCTC AAATCACTGCCCACAAATTGTCTTACTCACCTCAAAATAACTGGAATA GATTACAGGGAGGGGGAGATTGAATCAGATTCCGTGGAGGAGGAGAT TGAATTGGAAGTTGGGGAGGCATTTCAGAAGTGTGCATCTTCTTTGAG AAGCCTCATCATAATCGGAAATCACGGAATAAATAAAGTGATGAGAC TGTCTGGAAGAACAGGGTTGGAGCATTTCACTCTGTTGGACTCACTCA AACTTTCAAATATAGAAGACCAGGAAGATGAGGGCGAAGACAACATC ATATTCTGGAAATCCTTTCCTCAAAACCTTCGCAGTTTGAGAATTAAA GACTCTGACAAAATGACAAGTTTGCCCATGGGGATGCAGTACTTAAC CTCCCTCCAAACCCTCGAACTATCATATTGTGATGAATTGAATTCCCT TCCAGAATGGATAAGCAGCTTATCATCTCTTCAATACCTGCGCATATA CAACTGTCCAGCCCTGAAATCACTACCAGAAGCAATGCGGAACCTCA CCTCCCTTCAGACACTTGGGATATCGGATTGTCCAGACCTAGTTAAAA TATGCAGAAAACCCAACGGCGAGGACTATCCCAAAATTCAACACATC CCCGGCATTGTAAGTGATTGCAGAAAGTATTTTATTCATTTATATTTAT TTTATGCTTAGAATGATATACGCCGTCGTCCTTAGGTTTCCAATCTTGA ATTTGGTTTTTGTTTTCTTTCTTTGTTTCTTTATTCAACACCAGCCCATT |
| | TATGATTGATTCATTAAAAAAGGAAGAAGACAACGAATTGAGATTC CTGGGGTTTTTTTTCGTTGGGGTTGGTTTCATGTATATGTTGCTGAT TAAATACGAGACTGATGATGATTATGTGTTTATGGGTTTTAAATCAGA TTAAATATATGGAAAATGTAAGTTAGTTGGGGATGCACATAAGGTGT TTGATGAAATGTCTTTTAGAAATGTTGTTCTTGGACTTAGAATGATA TACACTGTCGTCCTTTGGTTTCCAATCTTACATTTGGTTTGTGTTTTCTT AGTTTGTTTCTTTAATCAACACCAACCCATTTTTTTAAACTACCTGCA ACTACTAATTTACGTTTACCCTTTATCTCAGGTACTAAATGAATATTG GTGATTTTCAGTTACTCAACACTAGCTTGATCCTGAACGCACCCAACC TTCAGGTTAGAATCCGGCTTACTCATCCTTTTGTCCAGTTTTCAAGTAA TTGTTTTGGCAGGATCAATTCTCTAATTGTTGTACACCGTATATTGCAA TTTATAGTGACTACAGTTAATGAATGTTTACAAAAAATTAGTCATGTA AAAACTTCTTCTCTGTCCATTACATAAACTCTTTTTCTCTTTCTAACTT ATCATGTTCATGTCTAAAAAATTAAACATGCTCACATCAATGTTCATT TAAGCTAACTTACTTCTGTAAGAGAGCGAGCTAGTTAAAAACTCCTTT AACTTTCTGTTTTATACTCAGGAGTCAGGAAGGATATGGATTGATGCA GCTTTCACAGGCCAAGTAATATAATGTTCTTTATCAATCACTCAATAC CTGGAGATTGTTTGAACACATAATTCACCTCTTTTTTTCCATCTCAAAT TGCAGCCTTTTACTGGGATTTGAATGACAGCAAACATTTGATTGTCAC GAGGATTGATCTAACTGGTGAATGTTGTGATCACCATATTGGTCTTTC TCATTCAAACAAACTTGCATACCGTTCTTTTGAAGTTTGAAGACAAGC AGGCGATAATCAACTCAGGTCGAAGCTCATGTGCAGGAGAGAATAGA ATATAGAGGAGATTATTTTTAAAAGAAACGCTCAAAGGTATTGTAGC ACCTTTCCTATTTGCTACTGCAGTGATGTTTTTATTTCTTGTTTGCAGC GCTTGTTTCTCATATTTCAACCTACTTTTAGAAAAAAAAACATCTCCG AACATAACCAAAAATAAAGTTCCCTTATCAGTGCTTTCCCTGCTTTCT TCTGAACAACATATACAATTATAAACCCTTTTTCTCTCTTACCTTTGTT ATTCTTCCTTGCTTCATTGAGATAACACTCTCCTGTTTTTGTTTTGTTGT TAGTCATTACATGGATATATCAAGGACAACAGTTCTGTAGTCCGTCAA CTGTGGTTAGGAAGGCTAAACTGGAGCACAATAACCCCATGTCAATT GAATAGTAAAGGTGTGCTATATCAGTTTCGTTTGGCTTGGCTTGTTTA CCTGAAAAATGGCTGGTTATTTATCCTTGTCTCTTTCTATGACGTGCAG TGGCTTGTTAATGTGTCTCGGACAACAATTCCTCACTTTCCAAGTTCC ATACACGCTGATGTAACTATCTTCTGCAGTCTGTTCTTTCATTTTTGCC ACGTGCTCTAATTATAACTTTTTGTACTCAATAATCAACTCCTTGTCCC GGTATTTGCAGAGACTACTTAAACAGGTAAAGTGACAATCCTGGCGA AGTTGTCTGTTTCTTAGCTCTGATCCCATCATTCAGGTAAGATTAAGT AGTTAAGAAGAAACATTTATTTTTACCTAATTTGAATGAACTTGTGTA ACTGCTTGCTTCCTGCATTAAATAAGAAATTTCTGCTGCATATGTGAC AGTTACATCCACAAAAAAGTTGGAGGTTTGTTCAGGGATTGGAAATG AAGGTACTTCAGAATTTGTTTATGAATGCTCCAGACTTCAGAGTCTTT AATGGAAAATTCGAGTCACTAAAAATACATTATTCCTATCATCAGAGC TTTCAAGTTCCTCTATACAAGGTCAACTGAGTTCCTCTTTGCCTCTTGT TTAATTGTATTTACTTGTACCTTAATTATAATCTGTATATTGTTTTAGT TAAGTTCTAAAACAGGTTACTATTCATCATTTGTGCAGCATATTGCTG GAATCAAGAATCTAGTGCTTCTTCTTCCTGACTTCACTGTCAAAATAG CAGTTCCCATGCTGGAAATAAGCGCGCTAGCAATGTAATTGATGACA TGGATGTTGCTGCTTCTGAGTTTTGATCATAAAAAGCTGTTATGTGTTT CTTGAATGTAATGTAGAAGAGGAGAAAACTGAAAACTCTTGGCAAAA ACGTGAAATTGCAGTGCCTCGGGGTGGGAGGGATCACCCGGATTCAG TTCAGACGATACTTTTTTCAACCCGGTTTGTTCCGGTTTTCAGCTTCAG GTTTTCCTTGTACTTTTGGGGGACACAGACAAGGCTACTTCATGTTTG AGAAACCTAATTGAGGCTATCTTGTAGCAAATGCACACCACACTCTTT CTCTCTTCTCTCCTTTTTTCACCTTCCATTTGTAAAAATCCTCCTTTAAA GGTTAATAAAAAAAAAGCTCCAAGTCTCGTAGGGTGGATGTAGGTCA CATTGACCGAACCACGGTAAACTCTTTGTGTTCTTTCTTCTCTCTTTGT |
| | TTCTCATTTTTACGGCAAGTGTTTATGGTTAACCATGCATCTTAGAATA GCTTAAGGCATTAACATAATAACATCAATGTTCTCCAAAGATTCACCT TACTTGTTGTACATAATCACAATGTTAAGCCTATGAAGGTAGAATGCT CTCATGATTTGGTTTAACCAAAAAATAAACTCTAAAATAATACGGAGT AATAAAAATTGGCCATAAACTAATTACAAAGTTTGATTTTTGT |
| SEQ ID No:18: | GGCTTAACTTGCTCACCAATTCTTAAATAGACTCGACTTACACTGAAT TTATTGTGTACTATGTCTAGAAAGTAAGGTCAACAACTTTCTTCTAAA TTATTTTGGCATGTTTATTAGGGTTATTATGAAAAACATATCAAATTG GTGTTGTTAGTTAGGCTTGAATAATATGATTTTAAGTCACGAGACTTT TATAAATTAGGTAATTTGATTTAAAAAATTGTTACATATCTAAGAAAA TGGATGTTAAATTTATCAGTCAATGTATAAACAATAAAAGTCAATATG TATGTAAAAGGGTTGCTAGATAAAATCTTTGGTTTTTGGTCCACAAC TCCACACTAAGAGGAACTCCAATGCTTAGTTACAAGGTGGTGTAGTTA AAAAGTAACTCCAATAGTTAACTACACGGTATTATAGTTAAATTTGCC AACTCAAATTTCTAACTACAATATATTTAAGCTACAAAGTTTCTCATT GGCTGACTACAATACGTTGTAGCGCCTTATAATATTTATTCAATATA CAATTTTATTTATTTACCTTTTTAACAATTTTTTTTGATCTACCTGCT GTCCTGTTCATATGAGCTACACTAATTTGATAGCTGCTTACGCAATTC TTATATCAACGGTTGGCTACTTGTTCAAATATTTTTATTTTTTTACGAG TAAGTCATTTTATGATCATTGAAGTGGCTCTATTATTATTATCATGCAC CGATTAACGCAAGAATAATTAACTCGGTACGAATTAGTTTCAAAATA AAATCCCTCAAAAAAAAAAGTTTCAAAATAAAATTAACAGAAAACCA ACCTTCTCCGGTTTACTGTTGTTAGAGCATGGAATTTTCCAGTAATCG CAGACCCCAAATTATCTTCCAGTTGAATCAATCCTTGATTTTTGGATTT GCCAGAAAACTCCTTGAATTTAGGGTTCATATTTGATCCGTAATTGG GAAAATTTTCAGCAATTGATCTTCCAAATCAGCCCTACTTGTTTCCAG ACTGCAAATGAAAGGTGCGAACTTTATACTGCATTTTGGTTTTCCATT AGTGTAATTTAGTGTAATTTATTAAGATAAACTGCATTTTGCAATTGT TTTATTCGACTACTCATTTTTAAATCAAATTGCTTAATTGCTAGTTAGT TTTCTTATCATATTGCCAAAAAAAATTATTTTTGTTTAATTGGTGAAAA AGGGTAAATTATACCTAGTGTACAAGATTTTCTTGCACACCACCGTTA ATTTGTTGACACATCATCAAACGTACTGAAAAATGAGAATGAAAGAC AATAAATATGTCATTTTAACCAATAGAAAAACATGATGTAGTAAGAT CCTTAATTGATAGATAAATAATTAAATATCAGTCCATTAGTTGAATAT TCAATGAAAATGTATGGTCCAAAAATGGCGTTTAATAGTCAATGTCAT GCTTTATGGGGTGGTGGAGTACTATGTGACTGTGTGTGGACTTGGAGA AGACTAGAGAGTATGATTATCAACCTATGGACCCTCAAAATGAAAAT GAAAATGATGTTTACACGTGCTATTTGCACGGACTTCAATGCAAATAG TATAAATTTACGGTCAAAGTTTTCATTCTAAAGCGTAAATAACTTTCA TGAATGGAGGACGGTAGTATAAGTATAACGTTATAGCCTACCATTTTC TTATCATATTCACATAAATTTGTTGCTGAAATTTGTTTTACTTGGCTAA AATACTTTTGTTCTTATTGGCAGATAAACATCAGTACGTCCATTATTG GCCAACTTGCTGAGTCCATTATTGGCCAACTTGAACATATACCTCCAA ACAATAATCAATAATGTCGATTATGAAGTTTGTGAATGCAATTTATTA TCACTTTCATTTATAAAATGACTACTTGATTAACACATACAATATTAC CTTTCTCCAAACACCCTTTCAATTCTGCTTAATCTTGTTTCTCATCAT CTCTTCATCTTTCTGAAAACACAACCCAATGGCCGAAATCGGATACTC GGTTTGTGCGAAACTCATCGAAGTGATTGGCAGTGAGCTGATCAAAG AGATTTGTGACACATGGGGTTACAAATCTCTTCTTGAGGACCTCAACA AAACTGTATTGACGGTCAGGAACGTTCTCATTCAAGCCGGGGTGATG CGGGAGCTTACTAGTGAACAACAAGGTTTCATTGCAGACCTTAAAGA TGTTGTTTATGATGCTGATGACTTGTTCGACAAGTTACTCACTCGTGCT GAGCGAAAACAGATTGATGGAAACGAAATCTCTGAAAAGGTACGTCG TTTCTTTTCCTCTAGTAACAAGATCGGTCAAGCTTACTACATGTCTCGT AAGGTTAAGGAAATTAAGAAGCAGTTGGATGAAATTGTTGATAGGCA TACAAAATTTGGGTTTAGTGCCGAGTTTATACCTGTTTGTAGGGAAAG |
| Genomic sequence comprising alpha-WOLF 11 allele (with a 2kb region upstream of the start codon) | |
| | GGGGAACGAGAGGGAAACACGTTCATATATAGATGTCAAGAATATTC TTGGGAGGGATAAAGATAAGAATGATATCATAGATAGGTTGCTTAAT CGTAATGGTAATGAAGCTTGTAGTTTCCTGACCATAGTGGGAGCGGG AGGATTGGGAAAAACTGCTCTTGCACAACTTGTGTTCAATGATGAAA GGGTCAAAATTGAGTTCCATGATTTGAGGTATTGGGTTTGTGTCTCTG ATCAAGATGGGGGCCAATTTGATGTGAAAGAAATCCTTTGTAAGATTT TAGAGGTGGTTACTAAGGAGAAAGTTGATAATAGTTCCACATTGGAA TTGGTACAAAGCCAATTTCAAGAGAAGTTAAGAGGAAAGAAGTACTT CCTTGTTCTTGATGATGTATGGAACGAAGATCGTGAGAAGTGGCTTCC TTTGGAAGAGTTGTTAATGTTGGGTCAAGGGGGAAGCAAGGTTGTAG TGACCGCACGTTCAGAGAAGACAGCAAATGTCATAGGGAAAAGACAT TTTTATACACTGGAATGTTTGTCACCAGATTATTCATGGAGCTTATTTG AAATGTCGGCTTTTCAGAAAGGGCATGAGCAGGAAAACCATCACGAA CTAGTTGATATTGGGAAAAAGATTGTTGAAAAATGTTATAACAATCC ACTTGCTATAACGGTGGTAGGAAGTCTTCTTTATGGAGAGGAGATAA GTAAGTGGCGGTCATTTGAAATGAGTGAGTTGGCCAAAATTGGCAAT GGGGATAATAAGATTTTGCCGATATTAAAGCTCAGTTACCATAATCTT ATACCCTCGTTGAAGAGTTGCTTCAGTTATTGTGCAGTGTTTCCCAAG GATCATGAAATAAAGAAGGAGATGTTGATTGATCTTTGGATAGCACA AGGATACGTTGTGGCACTTGATGGAGGTCAAAGTATAGAAGATGCTG CCGAAGAACATTTTGTAATTTTGTTACGGAGATGTTTCTTTCAAGATG TAAAGAAGGATGAATATGGTGATGTTGATTCTGTTAAAATCCACGACT TGATGCACGATGTCGCCCAAGAAGTGGGGAGGGAGGAAATATGTGTA GTGAATGATAATACAAAGAACTTGGGTGATAAAATCCGTCATGTACA TGGTGATGTCAATAGATATGCACAAAGAGTCTCTCTGTGTAGCCATAG CCATAAGATTCGTTCGTATATTGGTGGTGATTGTGAAAAACGTTGTGT GGATACACTAATAGACAAGTGGATGTGTCTTAGGATGTTGGACTTGTC ATGGTCGGATGTTAAAAATTTGCCTAATTCAATAGGTAAATTGTTGCA CTTGAGGTATCTTAACCTGTCAGATAATAGAAATCTAAAGATACTTCC TGATGCAATTACAAGACTGCATAATTTGCAGACACTGCTTTTAGAAGA TTGCAGAAGTTTAAAGGAGTTGCCAAAAGATTTTTGCAAATTGGTCAA ACTGAGACACTTGGATTTATGGGGTTGTGATGATTTGATTGGTATGCC ATTTGGAATGGATAAGCTAACTAGTCTTAGAATACTACCAAACATTGT GGTGGGTAGGAAGGAACAAAGTGTTGATGATGAGCTGAAAGCCCTTA AAGGCCTCACCGAGATAAAAGGCGACATTGATATCAAAATCTGTGAA AATTATAGAATAGTTGAAGGCATGAATGACACAGGAGGAGCTGGGTA TTTGAAGAGCATGAAACATCTCAGGGAGATTGGTATTACATTTGATGG TGGATGTGTTAACCCTGAAGCTGTGTTGGCAACCCTAGAGCCACCTTC AAATATCAAGAGCTTATCTATAGATAATTACGATGGTACAACAATTCC AGTATGGGGAAGAGCAGAGATTAATTGGGCAATCTCCCTCTCACATC TTGTCGACATCACGCTTGAAGATTGTTACAATTTGCAGGAGATGCCAG TGCTGAGTAAACTGCCTCATTTGAAATCACTGTATCTTTTTAAGTTTTG TAAGTTAGAGTACATGGAGAGTAGAAGCAGCAGCAGTAGCAGTGACA CAGAAGCAGCAACACCAGAATTACCAACATTCTTCCCTTCCCTTGAAA AACTTAGACTTTGGTATCTGGAAAAGTTGAAGGGTTTGGGGAACAGG AGACCGAGTAGTTTCCCCGCCTCTCTGAATTGGAAATCTGGGAATGC CCAGATCTAACGTGGTTTCCTCCTTGTCCAAGCCTTAAAACGTTGAAA TTGGAAAAAAACAATGAAGCGTTGCAAATAATAGTAAAAATAACAAC AACAAGAGGTAAAGAAGAAAAAGAAGAAGACAAGAATGCTGGTGTT GGAAATTCACAAGATGATGACAATGTCAAATTACGGAAGGTGGAAAT AGACAATGTGAGTTATCTCAAATCACTGCCCACAAATTGTCTTACTCA CCTCAAAATAACTGGAATAGATTACAGGGAGGGGGAGATTGAATCAG ATTCCGTGGAGGAGGAGATTGAATTGGAAGTTGGGGAGGCATTTCAG AAGTGTGCATCTTCTTTGAGAAGCCTCATCATAATCGGAAATCACGGA ATAAATAAAGTGATGAGACTGTCTGGAAGAACAGGGTTGGAGCATTT CACTCTGTTGGACTCACTCAAATTTTCAAAGATAGAAGACCAGGAAG |
| | ATGAGGGCGAAGACAACATCATATTCTGGAAATCCTTTCCTCAAAAC CTCCGCAGTTTGGAAATTAAAGGCTCTTGCAAAATGACAAGTTTGCCC ATGGGGATGCAGTACTTAACCTCCCTCCAAACCCTCGAACTATCATAT TGTGATGAATTGAATTCCCTTCCAGAATGGATAAGCAGCTTATCATCT CTTCAATACCTGCGCATATACAACTGTCCAGCCCTGAAATCACTACCA GAAGCAATGCGGAACCTCACCTCCCTTCAGACACTTGGGATATCGGA TTGTCCAGACCTAGTTAAAATATGCAGAAAACCCAACGGCGAGGACT ATCCCAAAATTCAACACATCCCCGGCATTGTAAGTGATTGCAGAAAG TATTTTATTCATTTATATTTATTTTATGCTTAGAATGATATACGCCGTC GTCCTTAGGTTTCCAATCTTGAATTTGGTTTTGTTTTCTTTCTTTGTTT CTTTATTCAACACCAGCCCATTTATGATTGATTCATTAAAAAAAGGAA GAAGACAACGAATTGAGATTCCTGGGTTTTTTTTCGTTGGGGTTGG TTTTCATGTATATGTTGCTGATTAAATACGAGACTGATGATGATTATG TGTTTATGGGTTTTAAATCAGATTAAATATATGGAAAATGTAAGTTAG TTGGGGATGCACATAAGGTGTTTGATGAAATGTCTATTAGAAATGTTG TTTCTTGGACTTAGAATGATATACACTGTCGTCCTTTGGTTTCCAATCT TACATTTGGTTTGTGTTTTCTTAGTTTGTTTCTTTAATCAACACCAACC CATTTTTTTAAACTACCTGCAACTACTAATTTACGTTTACCCTTTATC TCAGGTACTAAATGAATATTGGTGATTTTCAGTTACTCAACACTAGCT TGATCCTGAACGCACCCAACCTTCAGGTTAGAATCCGGCTTACTCATC CTTTTGTCCAGTTTTCAAGTAATTGTTTTGGCAGGATCAATTCTCTAAT TGTTGTACACCGTATATTGCAATTTATAGTGACTACAGTTAATGAATG TTTACAAAAAATTAGTCATGTAAAAACTTCTTCTCTGTCCATTACATA AACTCATTTTCTCTTTCTAACTTATCATGTTCATGTTTAAAAAATTAAA CATGCTCACATCATTGTTCATTTGAGCTAACTTACTTCTGTAAGAGAG CGAGCTAGTTAACAACTCCTTTAACTTTCTGTTTTATACTCAGGACAT GGATTGATGCAAGCATGAAGAACTTCTGGAATTTGCTAAAACTCTACC AAAGCGATGAGAGTTTGGACTTTGTTTCACTTGAAGTCAGGGATTGTC AACAAAGCCACAGTGTGCATGTTGGCTGTGTCACTTGGACGATAAAA AGGTTTATTTAATTGTTTTCCTAAGTGTATTTGGCTTACAAGCTTTTAC TTTTCACTTGAAAGGGTTTTTTTGTTTTAAGCTTTTTGAATTAGAGTT TCGGTTGAAGTAAGAGTAGTCGTATTAGTCTTTTACTTTGCAGGAGTC TATGCCTATATAAGGTAAGACTCGTATTTGTAATTTTCAGATTATGCA ATTCAAGTTTTCGAGTGTTTTCTTAAAAAAACATATCATACCTGTGTG TAGCATAAAGATAAATTCTGATGCTGTGCTTCTTGTTATGGCTCACAT TGGTTTTCATTGTTTGGATTGTTTCACAGGGAAGCAGAGAATACCTGG AACCTATGAAGGACGCTATATCTATAGCAGCTTTCACAGGCCAAGTA ATATAATGTTCTTTATCAATCACTCAATACCTGGAGATTGTTTGAACA CATAATTCACCTCTTTTTTTCCATCTCAAATTGCAGACTTTTACTGGGA TTTGAATGACAGCAAACATTTGATTGTCACGAGGATTGATCTAACTGG TGAATGTTGTGATCACCATATTGGTCTTTCTCATTCAAACAAACTTGC ATACCGTTCTTTTGAATTTTGAAGACAAGCAGGCGATAATCAACTCAG GTCGAAGCTCATGTGCAGGAGAGAATCGAATATAGAGGAGATTATTT TTAAAAGAAACGCTCAAAGGTATTGTAGCACCTTTCCTATTTGCTACT GCAGTGATGTTTTTATTTCTTGTTTGCAGCGCTTGTTTCTCATATTTCA ACCTACTTTTAGAAAAAAAACATCTCCGAACATAACCAAAAATAAA GTTCCCTTATCAGTGCTTTCCCTGCTTTCTTCTAAACAACATATACAAT TATAAACCCTTTTTCTCTCTTACCTTTGTTATTCTTCCTTGCTTCATTGA GATAACACTCTCCTGTTTTTGTTTTGTTGTTAGTCATTACATGGATATA TCAAGGACAACAGTTCTGTAGTCCGTCAACTGTGGTTAGGAAGGCTA AACTGGAGCACAATAACCCCATGTCAATTGAATAGTAAAGGTGTGCT ATATCAGTTTCGTTTGGCTTGGCTTGTTTACCTGAAAAATGGCTGGTT ATTTATCCTTGTCTCTTTCTATGACGTGCAGTGGCTTGTTAATGTGTCT CGGACAACAATTCCTCACTTTCCAAGTTCCATACACGCTGATGTAACT ATCTTCTGCAGTCTGTTCTTTCATTTTTGCCACGTGCTCTAATTATAAC TTTTTGTACTCAATAATCAACTCCTTGTCCCGGTATTTGCAGAGACTAC |
| | TTAAACAGGTAAAGTGACAATCCTGGCGAAGTTGTCTGTTTCTTAGCT CTGAACCCATCATTCAGGTAAGATTAAGTATTTAAGAAGAAATTTTGT TTTTACCTAAAATGAATGATCTTGTGTAACTGCTTGCTTCTTGCATTAA ATAAGAACTTTCTGCTGCATATGTGACAGTTACATCCACAAAAAAGTT GGAGGTTTGTTCAGGGATTGGAAATGAAGGTACTTCAGAATTCCTGG AATGTTTATGAATGCTCCAGACTTCAGAGTCTTTAATGGAAAATTCGA GTCACTAAAAATACATTATTCCTATCATCAGAGCTTTCAAGTTCCTCT ATACAAGGTCAACTGAGTTCCTCTTTGCCTCTTGTTTAATTGTATTTAC TTGTACCTTAACTATAATCTGTATATTGTTTTAGTTAAGTTCTAAAACA GGTTACTATTCATCATTTGTGCAGCATATTGCTGGAATCAAGAATCTA GTGCTTCTTCTTCCTGACTTCACTGTAAACCTTAATCTGTTGCTGAATT TTTTTAATCGAAAGACTTTCTGTTTAATTATATTCTAAATCTATTACGA TGTCTTAAACAGCTTGAAAATGACACAAAATATAATAGCTCCACACCT AGATAAGACCCTCAAATGGAAATGTCAGTAACTTGTTACATAGAGAC AATATGCTGATATATAGTTCCACATAGATCACTCTTCTTTACTAAAAA CACATTATTTTATAACCTGGACATTAGTCGAGAGGGGGATGTCACGT CATGAGAAATCTTCATCAGAGCCTTCATTACTCGGAATTTTATTTTCTC CCAAAGCTGGTGAATTTGCCATAGATGTTGCTGTACTTCATTCTTATG ATGTTCAGGTGACTTGTCAGGCCTCACTGCCTCAGACTAACACTCTCG TGTTTCTTTTCTGTTGTTAGTCATGACATGGATACATCACGAACAACA GTTCTGTAGTCCAGCAACTGTGGTGTAGGAAGACTAAACTGGAGCAC AATAACTCCATGTCAATTGAATGGTAAAGATGTGCTATCTCAGTTTCT TTTGGCTTGTTTACATGAAAAACGGCTCGTTTTTTATCTGTGTCTTTCT ATGACGTTCAGGTGCAGTGGCTTGTTAATGTGTCTCAGACAACAATTT TCTCACTTTCCAAGTTCCATACATGCTGATGTAACTATCTTCTGCACTC TGTTCTTTCATTTTGCCTTTTGCTCTAATTATAACTTTTTGTACTCAATA ATCAACTCCTTGTCGCGTATTTGCAGGGATTACTTAAATAGGTATAGT GACAATCCTGGTGAAGTTGTCTGTTTCTTAGCTCTGATCCCATCATTCA GGTAAGATTAAGTAGTTAAGAAGAAACATTTATTTTTACCTAATTTGA ATGAACTTGTGTTAACTGCTTGCTTCCTGCATTAAATAAGAACTTTCT GCTGCATATGTGACAGTTACATCCACAAAAAAGTTGGAGGTTTGTTCA GGGATTGGAAATGAAGGTACTTCAGAATTCCTGGAATGTTTATGAAT GCTCCAGACTTCAGAGTCTTTAATGGAAAATTCGAGTCACTAAAAATA CATTATTCCTATCATCAGAGCTTTCAAGTTCCTCTATACAAGGTCAAC TGAGTTCCTCTTTGCCTCTTGTTTAATTGTATTTACTCGTACCTTAATT ATAATCTGTATATTGTTTTAGTTAAGTTCTAAAACAGGTTACTATTCAT CATTTGTGCAGCATATTGCTGGAATCAAGAATCTAGTGCTTCATCTTC CTGACTTCACTGTCAAAATAGTAGTTCCCATGCTGGAAATAAGCGCGC TAGCAATGTAATTGATGACATGGATGTTGCTGCTTCTGAGTTTTGATC ATAAAAAGCTGTTATGTGTTTCTTGAATGTAATGAAGGAGAGGAGAA AACTGAAAACTCTTGGCAAAAACGTGAAATTGCAGTGCCTCGGGGTG GTAGGGATCACCCGGATTCAGTTCAGACGATACTTTTTTCAACCCGGT TTGTTCCGGTTTTCAGCTTCGGGTTTTCCTTGTACTTTTGGGGCACACA TACAAGGCTACTTCATGTTTGAGAAACCTAATTGAGGCTATCTTGTAG CAAATGCACACCACACTCTTTCTCTCTTCTCTCCTTTTTTCACCTTCCA TTTGTAAAAATCCTCTTTTAAAGGTTAATAAAAAAAAGCTTCAAGTCT CGTAGGGTGGATGTAGGTCACATTGACCGAACCACGGTAAACTCTTT GTGTTCTTTCTTCTCTCTTTGTTTCTCATTTTTACGGCAAGTGTTTATGG TTAACCATGCATCTTAGAATAGCTTAAGGCATTAACATAATAACATCA ATGTTCTCCAAAGATTCACCTTACTTGTTGTACATAATCACAATGTTA AGCCTATGAAGGTAGAATGCTCTCATGATTTGGTTTAACCAAAAAATA AACTCTAAAATAATACGGAGTAATAAAAATTGGCCATAAACTAATTA CAAAGTTTGATTTTTGTATAGGGTATCTTGTACTTGTGATAAAAAAAA TTAAAAAAAAAAAATTTACTTATTTCTTCTATTTTTACTTGTTACACTT TTCTACA |
| SEQ ID No:19: | TTTGAAGTTAGGCTTAACTTGCTCACCAATTCTTAAATAGACTCGACT TACACTGAATTTATTGTGTACTATGTCTAGAAAGTAAGGTCAACAACT TTCTTCTAAATTATTTGGCATGTTATTAGGGTTATTATGAAAAACAT ATCAAATTGGTGTTGTTAGTTAGGCTTGAATAATATGATTTTAAGTCA CGAGACTTTTATAAATTAGGTAATTTGATTTAAAAAATTGTTACATAT CTAAGAAAATGGATGTTAAATTTATCAGTCAATGTATAAACAATAAA AGTCAATATGTATGTAAAAGGGTTGCTAGATAAAATCTTTGGTTTTTT GGTCCACAACTCCACACTAAGAGGAACTCCAATGCTTAGTTACAAGG TGGTGTAGTTAAAAAGTAACTCCAATAGTTAACTACACGGTATTATAG TTAAATTTGCCAACTCAAATTTCTAACTACAATATATTTAAGCTACAA AGTTTCTCATTGGCTGACTACAATACGTTGTAGCGCCTTATAATATTTT ATTCAATATACAATTTATTTATTTTACCTTTTAACAATTTTTTTTGA TCTACCTGCTGTCCTGTTCATATGAGCTACACTAATTTGATAGCTGCTT ACGCAATTCTTATATCAACGGTTGGCTACTTGTTCAAATATTTTTATTT TTTTACGAGTAAGTCATTTTATGATCATTGAAGTGGCTCTATTATTATT ATCATGCACCGATTAACGCAAGAATAATTAACTCGGTACGAATTAGTT TCAAAATAAAATCCCTCAAAAAAAAAAGTTTCAAAATAAAATTAACA GAAAACCAACCTTCTCCGGTTTACTGTTGTTAGAGCATGGAATTTTCC AGTAATCGCAGACCCCAAATTATCTTCCAGTTGAATCAATCCTTGATT TTTGGATTTGCCAGAAAACTCCTTGAATTTAGGGTTCATATTTGATCC GTAATTGGGAAAATTTTCAGCAATTGATCTTCCAAATCAGCCCTACTT GTTTCCAGACTGCAAATGAAAGGTGCGAACTTTATACTGCATTTTGGT TTTCCATTAGTGTAATTTATTAAGATGAACTGCATTTTGCAATTGTTTT ATTCGACTACTCATTTTTAAATCAAATTGCTTAATTGCTAGTTAGTTTT CTTATCATATTGCCAAAAAAAATTATTTTTGTTTAATTGGTGAAAAAG GGTAAATTATACCTAGTGTACAAGATTTTCTTGCACACCACCGTTAAT TTGTTGACACATCATCAAACGTACTGAAAAATGAGAATGAAAGACAA TAAATATGTCATTTTAACCAATAGAAAAACATGATGTAGTAAGATCCT TAATTGATAGATAAATAATTAAATATCAGTCCATTAGTTGAATATTCA ATGAAAATGTATGGTCCAAAAATGGCGTTTAATAGTCAATGTCATGCT TTATGGGGTGGTGGAGTACTATGTGACTGTGTGTGGACTTGGAGAAG ACTAGAGAGTATGATTATCAACCTATGGACCCTCAAAATGAAAATGA AAATGATGTTTACACGTGCTATTTGCACGGACTTCAATGCAAATAGTA TAAATTTACGGTCAAAGTTTTCATTCTAAAGCGTAAATAACTTTCATG AATGGAGGACGGTAGTATAAGTATAACGTTATAGCCTACAATTTTCTT ATCATATTCATATAAATTTGTTTCTAAAAGTTGTTTTACTTGGCTAAAA TACTTTTGTTCTTATTGGCAGATAAACATCAGTACGTCCATTATTGGCC AACTTGCTGAGTCCATTATTGGCCAACTTGAACATATACCTCCAAACA ATAATCAATAATGTCGATTATGAAGTTTGTGAATGCAATTTATTATCA CTTTCATTTATAAAATGACTACTTGATTAACACATACAATATTACCTTT CTCCAAACACCCTTTCAATTCTGCTTAATCTTGTTTTCTCATCATCTCT TCATCTTTCTGAAAACACAACCCAATGGCCGAAATCGGATACTCGGTT TGTGCGAAACTCATCGAAGTGATTGGCAGTGAGCTGATCAAAGAGAT TTGTGACACATGGGGTTACAAATCTCTTCTTGAGGACCTCAACAAAAC TGTATTGACGGTCAGGAACGTTCTCATTCAAGCCGGGGTGATGCGGG AGCTTACTAGTGAACAACAAGGTTTCATTGCAGACCTTAAAGATGTTG TTTATGATGCTGATGACTTGTTCGACAAGTTACTCACTCGTGCTGAGC GAAAACAGATTGATGGAAACGAAATCTCTGAAAAGGTACGTCGTTTC TTTTCCTCTAGTAACAAGATCGGTCAAGCTTACTACATGTCTCGTAAG GTTAAGGAAATTAAGAAGCAGTTGGATGAAATTGTTGATAGGCATAC AAAATTTGGGTTTAGTGCTGAGTTTATACCTGTTTGTAGGGGAAGGGG AAACGAGAGGGAAACACGTTCATATATAGATGTCAAGAATATTCTTG GGAGGGATAAAGATAAGAATGATATCATAGATAGGTTGCTTAATCGT AATGGTAATGAAGCTTGTAGTTTCCTGACCATAGTGGGAGCGGGAGG ATTGGGAAAAACTGCTCTTGCACAACTTGTGTTCAATGATGAAAGGGT CAAAATTGAGTTCCATGATTTGAGGTATTGGGTTTGTGTCTCTGATCA |
| Genomic sequence comprising alpha-WOLF 12 allele (with a 2kb region upstream of the start codon) | |
| | AGATGGGGGCCAATTTGATGTGAAAGAAATCCTTTGTAAGATTTTAG AGGTGGTTACTAAGGAGAAAGTTGATAATAGTTCCACATTGGAATTG GTACAAAGCCAATTTCAAGAGAAGTTAAGAGGAAAGAAGTACTTCCT TGTTCTTGATGATGTATGGAACGAGGATCGTGAGAAGTGGCTTCCTTT GGAAGAGTTGTTAATGTTGGGTCAAGGGGGAAGCAAGGTTGTAGTGA CCACACGTTCAGAGAAGACAGCAAATGTCATAGGGAAAAGACATTTT TATACACTGGAATGTTTGTCACCAGATTATTCATGGAGCTTATTTGAA ATGTCGGCTTTTCAGAAAGGGCATGAGCAGGAAAACCATCACGAACT AGTTGATATTGGGAAAAAGATTGTTGAAAAATGTTATAACAATCCAC TTGCTATAACGGTGGTAGGAAGTCTTCTTTATGGAGAGGAGATAAGT AAGTGGCGGTCATTTGAAATGAGTGAGTTGGCCAAAATTGGCAATGG GGATAATAAGATTTTGCCGATATTAAAGCTCAGTTACCATAATCTTAT ACCCTCGTTGAAGAGTTGTTTTAGTTATTGTGCAGTGTTTCCCAAGGA TCATGAAATAAAGAAGGAGATGTTGATTGAACTTTGGATGGCACAAG GATATGTTGTGCCGTTGGATGGAGGTCAAAGTATAGAAGATGCTGCC GAGGAACATTTTGTAATTTTGTTACGAAGGTGTTTCTTTCAAGATGTA AAGAAGGATAAATATGGTGATGTTGATTCTGTTAAAATCCACGACTTG ATGCACGATGTCGCCCAAGAAGTGGGGAGGGAGGAATTATGTGTAGT GAATGATAATACAAAGAACTTGGGTGATAAAATCCGTCATGTACATC GTGATGTCATTAGATATGCACAAAGAGTCTCTCTGTGTAGCCATAGCC ATAAGATTCGTTCGTATATTGGTGGTAATTGTGAAAAACGTTGTGTGG ATACACTAATAGACAAGTGGATGTGTCTTAGGATGTTGGACTTGTCAA GGTCGGATGTTAAAAATTTGCCTAATTCAATAGGTAAATTGTTGCACT TGAGGTATCTTAACCTGTCAGATAATAGAAATCTAAAGATACTTCCTG ATGCAATTACAAGACTGCATAATTTGCAGACACTACTTTTAGAACGTT GCGAAAGTTTAAAGGAGTTGCCAAAAGATTTTTGCAAATTGGTCAAA CTGAGACACTTGGATTTAAGGGGATGTTTTTCTTTGATTGGTATGCCA TTGGGAATGGATAGGCTAATTAGTCTTAGAATACTACCAAACATTGTG GTGGGTAGGAAGGAACAAAGTGATGATGATGAGCTGAAAGCCCTAA AAGGCCTCACCGAGATAAAAGGCTCCATTCGTATTAGAATCTATTCAA AGTATAGAATAGTTGAAGGCATGAATGACACAGGAGGAGCTGGGTAT TTAAAGAGCATGAAACATCTCACGGGGGTTGATATTACATTTGATGGT GGATGTGTTAACCCTGAAGCTGTGTTGGCAACCCTAGAGCCACCTTCA AATATCAAGAGGTTAGAGATGTGGCATTACAGTGGTACAACAATTCC AGTATGGGGAAGAGCAGAGATTAATTGGGCAATCTCCCTCTCACATC TTGTCGACATCCAGCTTTGGCATTGTCGTAATTTGCAGGAGATGCCAG TGCTGAGTAAACTGCCTCATTTGAAATCACTGGAACTTTATAATTTGA TTAGTTTAGAGTACATGGAGAGCACAAGCAGAAGCAGTAGCAGTGAC ACAGAAGCAGCAACACCAGAATTACCAACATTCTTCCCTTCCCTTGAA AAACTTACACTTTGGCGTCTGGACAAGTTGAAGGGTTTTGGGAACAG GAGATCGAGTAGTTTTCCCCGCCTCTCTAAATTGGAAATCTGGGAATG CCCAGATCTAACGTGGTTTCCTCCTTGTCCAAGCCTTAAAACGTTGAA ATTGGAAAAAAACAATGAAGCGTTGCAAATAATAGTAAAAATAACAA CAACAAGAGGTAAAGAAGAAAAAGAAGAAGACAAGAATGCTGGTGT TGGAAATTCACAAGATGATGACAATGTCAAATTACGGAAGGTGGAAA TAGACAATGTGAGTTATCTCAAATCACTGCCCACAAATTGTCTTACTC ACCTCAAAATAACTGGAATAGATTACAGGGAGGGGGAGATTGAATCA GATTCCGTGGAGGAGGAGATTGAATTGGAAGTTGGGGAGGCATTTCA GAAGTGTGCATCTTCTTTGAGAAGCCTCATCATAATCGGAAATCACGG AATAAATAAAGTGATGAGACTGTCTGGAAGAACAGGGTTGGAGCATT TCACTCTGTTGGACTCACTCGAACTTTCAAAGATAGAAGACCAGGAA GATGAGGGCGAAGACAACATCATATTCTGGAAATCCTTTCCTCAAAA CCTCCGCAGTTTGGAAATTAAAGGCTCTTGCAAAATGACAAGTTTGCC CATGGGGATGCAGTACTTAACCTCCCTCCAAACCCTCAAACTAGAAA ATTGTGATGAATTGAATTCCCTTCCAGAATGGATAAGCAGCTTATCAT CTCTTCAATACCTGGGCATATTCAACTGTCCAGCCCTAGAATCACTAC |
| | CAGAAGCAATGCGGAACCTCACCTCCCTTCAGAGACTTGTGATACGG CTGTGTCCAGACCTAGTTAAAAGATGCGGAAAACCCAAAGGCAAGGA CTATCCCAAAATTCAACACATCCCCGAAATTGTAAGTGATTGCAGAA AGTATTTATTCATTTATATTTATTTATGCTTAGAATGATATACACCG TCGTCCTTTGGTTTCAAATCTTGAATTTGGTTTTGTTTCTTTCTTTGT TTCTTTATTCAACACCAGCCCATTTATGATTGATTCATTAAAAAAAGG ATGGAGTTTTGTGTATTTGAAGAAGACAACGAATTGAGATTCCTGGG GTTTTCTTTTTGTTGGGGTTGGATTTCATGTATATGTTGCTGATTAAAT ACGAGACTGATGATGATGATGATGTGTTTATGGGTTTTAAATCAGATT AAATATATGGGAAATGTAAGTTAATTTGGGATGCACATAAGGTGTTT GCTGAAATGTCTATGAGAAATGTTGTTTCTTGGACTTAGAATGATATA CACTGTCGTCCATTGGTTTCCAATCTTACATTTGGTTTGTGTTTTCTTA GTTTGTTTCTTTAATCAACACCAACCCATTTTTTTAAAACTACCTGCAA CTACTAATTTACGTTGACCCTGTATCTCAGGTACTAAATGAATATTGG TGATTTTCAGTTACTCAACACTAGCTTGATCCTGAACGCACCCAACCT TCAGGTTAGAATCCGGCTTACTCATCCTTTTGTCCAGTTTTCAAGTAAT TGTTTTGGCAGGATCAATTCTCTAATTGTTGTACACCGTATATTGCAAT TTATAGTGACTACAGTTAATGAATGTTTACAAAAAATTAGTCATGTAA AAACTTCTTCTCTGTCCATTACATAAACTCTTTTTCTCTTTCTAACTTAT CATGTTCATGTCTAAAAAATTAAACATGCTCACATCAATGTTCATTTA AGCTAACTTACTTCTGTAAGAGAGCGAGCTAGTTAAAAACTCCTTTAA CTTTCTGTTTTATACTCAGGACATGGATTGATGCAAGCATGAAGAACT TCGGGAATTTGCTAAAACTCTACCAAAGCGATGAGAGTTTGGACTTTG TTTCACTTGAAGTCAGGGACTGTCAACAAAGCCACAGTGTGCATGTTG GCTGTTTCACTTGGACGATAAAAAGGTTTATTTAATTGTTTTCCTAAGT GTATTTGGCTTACAAGCTTTACTTTTCGCTTGAAAGGGTTTTTCTTGT TTTAAGCTTTTTGAATTAGAGTTTCGGTTGAAGTAAGAGTAGTCGTAT TAGTCTTTTACTTTGCAGGAGTCTATGCCTATATAAGGTAAGACTCGT ATTTGTAATTTTCAGATTATGCAATTCAAGTTTTCGAGTGTTTTCTTAA AAAAACATATCATACCTGTGTGTAGCATAAAGATAAATTCTGATGCTG TGCTTCTTGTTATGGCTCACATTGGTTTTCATTGTTTGGATTGTTTCAC AGGGAAGCAGAGAATACCTGGAACCTGTAAAGGACGCTATATCTATA GCAGCTTTCACAGGCCAAGTAATATAATGTTCTTTATCAATCACTCAA TACCTGGAGATTGTTTGAACACATAATTCACCTCTTTTTTTCCATCTCA AATTGCAGACTTTTACTGGGATTTGAATGACAGCAAACATTTGATTGT CACGAGGATTGATCTAACTGGTGAATGTTGTGATCACCATATTGGTCT TTCTCATTCAAACAAACTTGCATACCGTTCTTTTGAAGTTTGAAGACA AGCAGGCGATAATCAACTCAGGTCGAAGCTCATGTGCAGGAGAGAAT AGAATATAGAGGAGATTATTTTTAAAAGAAACGCTCAAAGGTATTGT AGCACCTTTCCTATTTGCTACTGCAGTGATGTTTTTATTTCTTGTTTGC AGCGCTTGTTTCTCATATTTCAACCTACTTTTAGAAAAAAAAACATCT CCGAACATAACCAAAAATAAAGTTCCCTTATCAGTGCTTTCCCTGCTT TCTTCTAAACAACATATACAATTATAAACCCTTTTCTCTCTTACCTTT GTTATTCTTCCTTGCTTCATTGAGATAACACTCTCCTGTTTTGTTTTGT TGTTAGTCATTACATGGATATATCAAGGACAACAGTTCTGTAGTCCGT CAACTGTGGTTAGGAAGGCTAAACTGGAGCACAATAACCCCATGTCA ATTGAATAGTAAAGGTGTGCTATATCAGTTTCGTTTGGCTTGGCTTAC CTGAAAAATGGCTGGTTATTTATCCTTGTCTCTTTCTATGACGTGCAGT GGCTTGTTAATGTGTCTCGGACAACAATTCCTCACTTTCCAAGTTCCA TACACGCTGATGTAACTATCTTCTGCAGTCTGTTCTTTCATTTTTGCCA CGTGCTCTAATTATAACTTTTTGTACTCAATAATCAACTCCTTGTCCCG GTATTTGCAGAGACTACTTAAACAGGTAAAGTGACAATCCTGGCGAA GTTGTCTGTTTCTTAGCTCTGAACCCATCATTCAGGTAAGATTAAGTA TTTAAGAAGAAATTTTGTTTTTACCTAAAATGAATGATCTTGTGTAAC TGCTTGCTTCTTGCATTAAATAAGAACTTTCTGCTGCATATGTGACAG TTACATCCACAAAAAAAGTTGGAGGTTTGTTCAGGGATTGGAAATGAA |
| | GGTACTTCAGAATTCCTGGAATGTTTATGAATGCTCCAGACTTCAGAG TCTTTAATGGAAAATTCGAGTCACTAAAAAAACATTATTCCTATCATC AGAGCTTTGAAGTTCCTCTATACAAGGTCAACTGAGTTCCTCTTTGCC TCTTGTTTAATTGTATTTACTTGTACCTTAATTATAATCTGTATATTGTT TTAGTTAAGTTCTAAAACAGGTTATTATTCATCATTTGTGCAGCATATT GCTGGAATCAAGAATCTAGTGCTTCTTCTTCCTGACTTCACTGTCAAA ATAGCAGTTCCCATGCTGGAAATAAGCGCGCTAGCAATGTAATTGAT GACATGGATGTTGCTGCTTCTGAGTTTTGATCATAAAAAGCTGTTATG TGTTTCTTGAATGTAATGAAGGAGAGGAGAAAACTGAAAACTCTTGG CAAAAACGTGAAATTGCAGTGCCTCGGGGTGGTAGGGATCACCCGGA TTCAGTTCAGACGATACTTTTTCAACCCGGTTTGTTCCGGTTTTCAGC TTCGGGTTTTCCTTGTACTTTTGGGGCACACATACAAGGCTACTTCAT GTTTGAGAAACCTAATTGAGGCTATCTTGTAGCAAATGCACACCACAC TCTTTCTCTCTTCTCTCCTTTTTTCACCTTCCATTTGTAAAAATCCTCTT TTAAAGGTTAATAAAAAAAAAGCTTCAAGTCTCGTAGGGTGGATGTAG GTCACATTGACCGAACCACGGTAAACTCTTTGTGTTCTTTCTTCTCTCT TTGTTTCTCATTTTTACGGCAAGTGTTTATGGTTAACCATGCATCTTAG AATAGCTTAAGGCATTAACATAATAACATCAATGTTCTCCAAAGATTC ACCTTACTTGTTGTACATAATCACAATGTTAAGCCTATGAAGGTAGAA TGCTCTCATGATTTGGTTTAACCAAAAAATAAACTCTAAAATAATACG GAGTAATAAAAATTGGCCATAAATTATTTACAAGTTTGATTTTTGTA TAGGGTATCTTGTACTTGTGATAAAAAAAATTAAAAAAAAAAAAATT ACTTATTTCTTC |
| SEQ ID No:20: | ATGGCCGAAATCGGATACTCGGTTTGTGCGAAACTCATCGAAGTG ATTGGCAGTGAGCTGATCAAAGAGATTTGTGACACATGGGGTTAC AAATCTCTTCTTGAGGACCTCAACAAAACTGTATTGACGGTCAGG AACGTTCTCATTCAAGCCGGGGTGATGCGGGAGCTTACTAGTGAA CAACAAGGTTTCATTGCAGACCTTAAAGATGTTGTTTATGATGCTG ATGACTTGTTCGACAAGTTACTCACTCGTGCTGAGCGAAAACAGA TTGATGGAAACGAAATCTCTGAAAAGGTACGTCGTTTCTTTTCCTC TAGTAACAAGATCGGTCAAGCTTACTACATGTCTCGTAAGGTTAA GGAAATTAAGAAGCAGTTGGATGAAATTGTTGATAGGCATACAAA ATTTGGGTTTAGTGCCGAGTTTATACCTGTTTGTAGGGAAAGGGGG AACGAGAGGGAAACACGTTCATATATAGATGTCAAGAATATTCTT GGGAGGGATAAAGATAAGAATGATATCATAGATAGGTTGCTTAAT CGTAATGGTAATGAAGCTTGTAGTTTCCTGACCATAGTGGGAGCG GGAGGATTGGGAAAAACTGCTCTTGCACAACTTGTGTTCAATGAT GAAAGGGTCAAAATTGAGTTCCATGATTTGAGGTATTGGGTTTGT GTCTCTGATCAAGATGGGGGCCAATTTGATGTGAAAGAAATCCTT TGTAAGATTTTAGAGGTGGTTACTAAGGAGAAAGTTGATAATAGT TCCACATTGGAATTGGTACAAAGCCAATTTCAAGAGAAGTTAAGA GGAAAGAAGTACTTCCTTGTTCTTGATGATGTATGGAACGAAGAT CGTGAGAAGTGGCTTCCTTTGGAAGAGTTGTTAATGTTGGGTCAA GGGGGAAGCAAGGTTGTAGTGACCGCACGTTCAGAGAAGACAGC AAATGTCATAGGGAAAAGACATTTTTATACACTGGAATGTTTGTC ACCAGATTATTCATGGAGCTTATTTGAAATGTCGGCTTTTCAGAAA GGGCATGAGCAGGAAAACCATCACGAACTAGTTGATATTGGGAAA AAGATTGTTGAAAAATGTTATAACAATCCACTTGCTATAACGGTG GTAGGAAGTCTTCTTTATGGAGAGGAGATAAGTAAGTGGCGGTCA TTTGAAATGAGTGAGTTGGCCAAAATTGGCAATGGGGATAATAAG ATTTTGCCGATATTAAAGCTCAGTTACCATAATCTTATACCCTCGT TGAAGAGTTGCTTCAGTTATTGTGCAGTGTTTCCCAAGGATCATGA AATAAAGAAGGAGATGTTGATTGATCTTTGGATAGCACAAGGATA CGTTGTGGCACTTGATGGAGGTCAAAGTATAGAAGATGCTGCCGA AGAACATTTTGTAATTTTGTTACGGAGATGTTTCTTTCAAGATGTA |
| Genomic sequence of alpha-WOLF 15 allele | |
| | AAGAAGGATGAATATGGTGATGTTGATTCTGTTAAAATCCACGAC TTGATGCACGATGTCGCCCAAGAAGTGGGGAGGGAGGAAATATGT GTAGTGAATGATAATACAAAGAACTTGGGTGATAAAATCCGTCAT GTACATGGTGATGTCAATAGATATGCACAAAGAGTCTCTCTGTGT AGCCATAGCCATAAGATTCGTTCGTATATTGGTGGTGATTGTGAAA AACGTTGTGTGGATACACTAATAGACAAGTGGATGTGTCTTAGGA TGTTGGACTTGTCATGGTCGGATGTTAAAAATTTGCCTAATTCAAT AGGTAAATTGTTGCACTTGAGGTATCTTAACCTGTCAGATAATAGA AATCTAAAGATACTTCCTGATGCAATTACAAGACTGCATAATTTGC AGACACTGCTTTTAGAAGATTGCAGAAGTTTAAAGGAGTTGCCAA AAGATTTTTGCAAATTGGTCAAACTGAGGCACTTGGAATTACAGG GTTGTCATGATTTGATTGGTATGCCATTTGGAATGGATAAGCTAAC TAGTCTTAGAATACTACCAAACATTGTGGTGGGTAGGAAGGAACA AAGTGATGATGAGCTGAAAGCCCTAAAAGGCCTCACCGAGATAAA AGGCTCCATTTCTATCAGAATCTATTCAAAGTATAGAATAGTTGAA GGCATGAATGACACAGGAGGAGCTGCTTATTTGAAGAGCATGAAA CATCTCAGGGAGATTGATATTACATTTTTGGGTGAATGTGTTGGCC CTGAAGCTGTATTGGAAACCTTAGAGCCACCTTCAAATATCAAGA GCTTATATATATATAATTACAGTGGTACAACAATTCCAGTATGGGG AAGAGCAGAGATTAATTGGGCAATCTCCCTCTCACATCTCGTCGA CATCCAGCTTAGTTGTTGTAGTAATTTGCAGGAGATGCCAGTGCTG AGTAAACTGCCTCATTTGAAATCGCTGAAACTTGGATGGTTGGAT AACTTAGAGTACATGGAGAGTAGCAGTAGCAGTGACACAGAAGC AGCAACACCAGAATTACCAACATTCTTCCCTTCCCTTGAAAAACTT ACTTTACAGCATCTGGAAAAGTTGAAGGGTTTTGGAACAGGAGA TCGAGTAGTTTTCCCCGCCTCTCTGAATTGGAAATCAAGAAATGCC CAGATCTAACGTCATTTCCTTCTTGTCCAAGCCTTGAGAAGTTGGA ATTGAAAGAAAGCAATGAAGCATTGCAAATAATAGTAAAAATAA CAACAAGAGGTAAAGAAAAAGAAGAGAACAATAATGCTGGTGTT AGAAATTCACAAGATGATGACAAAGTCAAATTACGGAAGATGGTG ATAGACAATCTGGGTTATCTCACGGGGGTTGATATTAGATTTGATG ATAGAGAAGGTGGATTTGTTAACCCTGAAGCTGTGTTGGCAACCC TAGAGCCACCTTCAAATATCAAGAGCTTATCTATACATCGTTTTGA TGGTAAAACACTTCCAGTATGGGGAAGAGCAGAGATTAATTGGGC AATCTCCCTCTCACATCTTGTCGACATCCAGCTTTGGCATTGTCGT AATTTGCAGGAGATGCCAGTGCTGAGTAAACTGCCTCATTTGAAA TCACTGGAACTTTATAATTTGATTAGTTTAGAGTACATGGAGAGCA CAAGCAGAAGCAGTAGCAGTGACACAGAAGCAGCAACACCAGAA TTACCAACATTCTTCCCTTCCCTTGAAAAACTTAGACTTTGGTATCT GGAAAAGTTGAAGGGTTTGGGAACAGGAGACCGAGTAGTTTTCC CCGCCTCTCTGAATTGGAAATCTGGGAATGCCCAGATCTAACGTG GTTTCCTCCTTGTCCAAGCCTTAAAACGTTGAAATTGGAAAAAAAC AATGAAGCGTTGCAAATAATAGTAAAAATAACAACAACAAGAGG TAAAGAAGAAAAAGAAGAAGACAAGAATGCTGGTGTTGGAAATT CACAAGATGATGACAATGTCAAATTACGGAAGGTGGAAATAGAC AATGTGAGTTATCTCAAATCACTGCCCACAAATTGTCTTACTCACC TCAAAATAACTGGAATAGATTACAGGGAGGGGGAGATTGAATCA GATTCCGTGGAGGAGGAGATTGAATTGGAAGTTGGGGAGGCATTT CAGAAGTGTGCATCTTCTTTGAGAAGCCTCATCATAATCGGAAATC ACGGAATAAATAAAGTGATGAGACTGTCTGGAAGAACAGGGTTG GAGCATTTCACTCTGTTGGACTCACTCAAATTTTCAAAGATAGAAG ACCAGGAAGATGAGGGCGAAGACAACATCATATTCTGGAAATCCT TTCCTCAAAACCTTCGCAGTTTGAGAATTAAAGACTCTGACAAAAT GACAAGTTTGCCCATGGGGATGCAGTACTTAACCTCCCTCCAAAC |
| | CCTCGAACTATCATATTGTGATGAATTGAATTCCCTTCCAGAATGG ATAAGCAGCTTATCATCTCTTCAATACCTGCGCATATACTACTGTC CAGCCCTGAAATCACTACCAGAAGCAATGCGGAACCTCACCTCCC TTCAGACACTTGGGATATCGGATTGTCCAGACCTAGTTAAAAGAT GCAGAAAACCCAACGGCAAGGACTATCCCAAAATTCAACACATCC CCAAAATTGTAAGTCATTGCAGAAAGTAATTTATTCATTTATATTT ATTTTATGCTTAGAATGATATACGCAGTCGTCCTTTGGTTTCAAAT CTTGAATTTGGTTTTGTTTTCTTTCTTTGTTTCTTTATTCAACACCA GCCCATTTATGATTGATTCATTAAAAAAAGGATGGAGTTTTATGGA TTTGAAGAAGACAACGAATTGAGATTCCTGGGGTTTTCTTTTTGTT GGGGTTGGATTTCATGTATATGTTGCTGATTAAATACGAGACTGAT GATGATGATGTGTTTATGGGTTTTAAATCAGATTAAATATATGGGA AATGCAAGTTAATTTGGGATGCACATAAGGTGTTTGCTGAAATGT CTATGAGAAATGTTGTTTCTTGGACTTAGAATGATATACACTGTCG TCCTTTGGTTTCCAATCTTACATTTGGTTTGTGTTTTCTTAGTTTGTT TCTTTAATCAACACCAACCCGTTTTTTTAAACTACCTGCAACTAC TAATTTACGTTTACCCTGTATCTCAGGTACTAAATGAATATTGGTG ATTTTCAGTTACTCAACACTAGCTTGATCCTGAACGCACCCAACCT TCAGGTTAGAATCCGGCTTACTCATCCTTTTGTCCAGTTTTCAAGT AATTGTTTTGGCAGGATCAATTCTCTAATTGTTGTACACCGTATAT TGCAATTTATAGTGACTACAGTTAATGAATGTTTACAAAAAATTAG TCATGTAAAAACTTCTTCTCTGTCCATTACATAAACTCTTTTTCTCT TTCTAACTTATCATGTTCATGTCTAAACAATTAAACATGCTCACAT CAATGTTCATTTAAGCTAACTTACTTCTGTAAGAGAGCGAGCTAGT TAAAAACTCCTTTAACTTTCTGTTTTATACTCAGGACATGGATTGA TGCAAGCATGAAGAACTTCGGGAATTTGCTAAAACTCTACCAAAG CGATGAGAGTTTGGACTTTATTTCACTTGAAGTCAGGGACTGTCAA CAAAGCCACAGTGTGCATGTTGGCTGTTTCACTTGGACGATAAAA AGGTTTATTTAATTGTTTTCCTAAGTGTATTTGGCTTACAAGCTTTT ACTTTTCACTTGAAAGGGTTTTCTTGTTTTAAGCTTTTCGAATTAG AGTTTTCGGTTGAAGTAAGAGTAGTCGTATTAGTCTTTTACCTAAG GAAGACTCTTTTTTGTAATTTTCAGACTATGCAATTCAAGTTTTCG AGTGTTTTCTTGCTTGTGTGATTGTGAGTTGGTGAATTCGTCTTTCA TACATTTTGAGATTATCAGAAGCTTTATGCTCCACCGGTAGTCTAG TACCTTTTCTGTTACTGTGCAGGGAAGTAATCTGGTACCTTCTATA TATATGGAAAAACATACATTATACATTATGCAAAATTCTTACAGGT TAGTTACTTCCTGGAACTTCATTTACACTTAGTTTTTTTGTTCCAT TCCCTCGGAATCAAGTCATTCCCTCTGAGAAATATGTAATGAACTT CTGTATGTTGCTGTTTGGTTCCTGTTTTAATCTTCAATTTTCTTGTA TAGTTACAGCTGCATTTACAATGAAGTTTAAGCAGACACTCTCTTT ATATAGTGCCTCTTTCTGGAGCACCGTAGAGCTGTCTGTGGTTGAT CACCATCTGCTGCCGAGAGATTCAGCAATCGCGTGTTTGATCAGGT AAAAGTTTTTATGTCAATGTGTTTTTTTTCCGTTTGATCAATTTAT GTCTGTATTCAGATTCTTATCTTCTTACAGTAGCATAACACATTGTT TCTTTCATTTATGTAAACTGTTTCAAGATTACAGAGATGTATGCTT CAGTCGACATTGATGATAACTTAAGATGGCATTCCTACAACAGTT GCAGGCGCATTCTAACTCCGGCAATTCTAGTTAGGCAAGAGGAGC ATTGCCAATACCTGCCACCTCTGGGATTTACTATACCAGGGTTGAA GTTTATGGAAGACACCAGCTATGCACAAGCCTTCAAGGGGTCATC CTACATAACAAGTTGAACCAACCAATTGCTTGTTGGTTCAGTGGTA ATTGAAGCTGAATTTGGTAGGGATGGCCCGTGTTCGATCCCCACA ACAACAATTGGGAGGGGACTGGAACCTATCCACACAGAACTCGCC CTGAATCCGGATTAGCCCTAAGGGTGAACGGGGTGCTAACACCAA AAAAAAAAACATAACAAGTTGAACCAAACATACTTTGTTTGAATT |
| | GAAGATTTAGTGATTTCATTTGATCGATTGAGATGTCTTATTATAA GCGTATATGCTCTTGGATTTGGCCACTTAGGTGTTGTTTGACAATT GGACATTAACTCGCTTTTATATTTCTTTTCTCTTAGGAAAGGTGAT CCTGAGAATTTATATTGGAACACTTTTTTTTCTCACTAGCTTTAAA AAAGTGTTCTGTGTTACCTGCAATTCAATTTGATTATTTTTCACATA GTTTTACCTGAAAAAGTGTTACCTGAAAAAGTGTTACCTGAAAAT CAACTGACATAAGTTTTGTTTGGATCCAATTAAGGACACTAGATA AATCGGAATAAATAATCAACCAATTAAGTACTTCATAATTAAATA TGAAGTGTATTATTATCTTATGCTTGTGACATTGAAGGATGTTATG ATATTTTAACTCAATACCTTGCAAAATATACTGGTTAAATTTCTTA ACAAGGTAACTTGGCAACA |
| SEQ ID No: 21 | TTTGAAGTTAGGCTTAACTTGCTCACCAATTCTTAAATAGACTCGACT TACACTGAATTTATTGTGTACTATGTCTAGAAAGTAAGGTCAACAACT TTCTTCTAAATTATTTGGCATGTTTATTAGGGTTATTATGAAAAACAT ATCAAATTGGTGTTGTTAGTTAGGCTTGAATAATATGATTTTAAGTCA CGAGACTTTTATAAATTAGGTAATTTGATTTAAAAAATTGTTACATAT CTAAGAAAATGGATGTTAAATTTATCAGTCAATGTATAAACAATAAA AGTCAATATGTATGTAAAAGGGTTGCTAGATAAAATCTTTGGTTTTTT GGTCCACAACTCCACACTAAGAGGAACTCCAATGCTTAGTTACAAGG TGGTGTAGTTAAAAAGTAACTCCAATAGTTAACTACACGGTATTATAG TTAAATTTGCCAACTCAAATTTCTAACTACAATATATTTAAGCTACAA AGTTTCTCATTGGCTGACTACAATACGTTGTAGCGCCTTATAATATTTT ATTCAATATACAATTTTATTTATTTACCTTTTTAACAATTTTTTTTTGA TCTACCTGCTGTCCTGTTCATATGAGCTACACTAATTTGATAGCTGCTT ACGCAATTCTTATATCAACGGTTGGCTACTTGTTCAAATATTTTTATTT TTTTACGAGTAAGTCATTTTATGATCATTGAAGTGGCTCTATTATTATT ATCATGCACCGATTAACGCAAGAATAATTAACTCGGTACGAATTAGTT TCAAAATAAAATCCCTCAAAAAAAAAAGTTTCAAAATAAAATTAACA GAAAACCAACCTTCTCCGGTTTACTGTTGTTAGAGCATGGAATTTTCC AGTAATCGCAGACCCCAAATTATCTTCCAGTTGAATCAATCCTTGATT TTTGGATTTGCCAGAAAACTCCTTGAATTTAGGGTTCATATTTGATCC GTAATTGGGAAAATTTTCAGCAATTGATCTTCCAAATCAGCCCTACTT GTTTCCAGACTGCAAATGAAAGGTGCGAACTTTATACTGCATTTTGGT TTTCCATTAGTGTAATTTATTAAGATGAACTGCATTTTGCAATTGTTTT ATTCGACTACTCATTTTTAAATCAAATTGCTTAATTGCTAGTTAGTTTT CTTATCATATTGCCAAAAAAAATTATTTTGTTTAATTGGTGAAAAAG GGTAAATTATACCTAGTGTACAAGATTTTCTTGCACACCACCGTTAAT TTGTTGACACATCATCAAACGTACTGAAAAATGAGAATGAAAGACAA TAAATATGTCATTTTAACCAATAGAAAAACATGATGTAGTAAGATCCT TAATTGATAGATAAATAATTAAATATCAGTCCATTAGTTGAATATTCA ATGAAAATGTATGGTCCAAAAATGGCGTTTAATAGTCAATGTCATGCT TTATGGGGTGGTGGAGTACTATGTGACTGTGTGTGGACTTGGAGAAG ACTAGAGAGTATGATTATCAACCTATGGACCCTCAAAATGAAAATGA AAATGATGTTTACACGTGCTATTTGCACGGACTTCAATGCAAATAGTA TAAATTTACGGTCAAAGTTTTCATTCTAAAGCGTAAATAACTTTCATG AATGGAGGACGGTAGTATAAGTATAACGTTATAGCCTACCATTTTCTT ATCATATTCACATAAATTTGTTGCTGAAATTTGTTTTACTTGGCTAAAA TACTTTTGTTCTTATTGGCAGATAAACATCAGTACGTCCATTATTGGCC AACTTGCTGAGTCCATTATTGGCCAACTTGAACATATACCTCCAAACA ATAATCAATAATGTCGATTATGAAGTTTGTGAATGCAATTTATTATCA CTTTCATTTATAAAATGACTACTTGATTAACACATACAATATTACCTTT CTCCAAACACCCTTTCAATTCTGCTTAATCTTGTTTTCTCATCATCTCT TCATCTTTCTGAAAACACAACCCAATGGCCGAAATCGGATACTCGGTT TGTGCGAAACTCATCGAAGTGATTGGCAGTGAGCTGATCAAAGAGAT TTGTGACACATGGGGTTACAAATCTCTTCTTGAGGACCTCAACAAAAC TGTATTGACGGTCAGGAACGTTCTCATTCAAGCCGGGGTGATGCGGG |
| Genomic sequence of alpha-CMV (with 2kb upstream of the start codon) and adjacent beta-CMV | |
| | AGCTTACTAGTGAACAACAAGGTTTCATTGCAGACCTTAAAGATGTTG TTTATGATGCTGATGACTTGTTCGACAAGTTACTCACTCGTGCTGAGC GAAAACAGATTGATGGAAACGAAATCTCTGAAAAGGTACGTCGTTTC TTTTCCTCTAGTAACAAGATCGGTCAAGCTTACTACATGTCTCGTAAG GTTAAGGAAATTAAGAAGCAGTTGGATGAAATTGTTGATAGGCATAC AAAATTTGGGTTTAGTGCTGAGTTTATACCTGTTTGTAGGGGAAGGGG AAACGAGAGGGAAACACGTTCATATATAGATGTCAAGAATATTCTTG GGAGGGATAAAGATAAGAATGATATCATAGATAGGTTGCTTAATCGT AATGGTAATGAAGCTTGTAGTTTCCTGACCATAGTGGGAGCGGGAGG ATTGGGAAAAACTGCTCTTGCACAACTTGTGTTCAATGATGAAAGGGT CAAAATTGAGTTCCATGATTTGAGGTATTGGGTTTGTGTCTCTGATCA AGATGGGGGCCAATTTGATGTGAAAGAAATCCTTTGTAAGATTTTAG AGGTGGTTACTAAGGAGAAAGTTGATAATAGTTCCACATTGGAATTG GTACAAAGCCAATTTCAAGAGAAGTTAAGAGGAAAGAAGTACTTCCT TGTTCTTGATGATGTATGGAACGAAGATCGTGAGAAGTGGCTTCCTTT GGAAGAGTTGTTAATGTTGGGTCAAGGGGGAAGCAAGGTTGTAGTGA CCACACGTTCAGAGAAGACAGCAAATGTCATAGGGAAAAGACATTTT TATACACTGGAATGTTTGTCACCAGATTATTCATGGAGCTTATTTGAA ATGTCGGCTTTTCAGAAAGGGCATGAGCAGGAAAACCATCACGAACT AGTTGATATTGGGAAAAAGATTGTTGAAAAATGTTATAACAATCCAC TTGCTATAACGGTGGTAGGAAGTCTTCTTTATGGAGAGGAGATAAGT AAGTGGCGGTCATTTGAAATGAGTGAGTTGGCCAAAATTGGCAATGG GGATAATAAGATTTTGCCGATATTAAAGCTCAGTTACCATAATCTTAT ACCCTCGTTGAAGAGTTGTTTAGTTATTGTGCAGTGTTTCCCAAGGA TCATGAAATAAAGAAGGAGATGTTGATTGAACTTTGGATGGCACAAG GATATGTTGTGCCGTTGGATGGAGGTCAAAGTATAGAAGATGCTGCC GAGGAACATTTTGTAATTTTGTTACGAAGGTGTTTCTTTCAAGATGTA AAGAAGGATAAATATGGTGATGTTGATTCTGTTAAAATCCACGACTTG ATGCACGATGTCGCCCAAGAAGTGGGGAGGGAGGAATTATGTGTAGT GAATGATAATACAAAGAACTTGGGTGATAAAATCCGTCATGTACATC GTGATGTCATTAGATATGCACAAAGAGTCTCTCTGTGTAGCCATAGCC ATAAGATTCGTTCGTATATTGGTGGTAATTGTGAAAAACGTTGTGTGG ATACACTAATAGACAAGTGGATGTGTCTTAGGATGTTGGACTTGTCAT GGTCGGATGTTAAAAATTTGCCTAATTCAATAGGTAAATTGTTGCACT TGAGGTGTCTTAACCTGTCTTATAATAAAGATCTGTTGATACTCCCTG ATGCAATTACAAGACTGCATAATTTGCAGACACTGCTTTTAAAAGAGT GCAGAAGTTTAAAGGAGTTGCCAAAAGATTTTTGCAAATTGGTCAAA CTGAGGCACTTGGATTTAAGGTGTTCTGATTTGAAGGAGTTGCCAAAA GATTTTTGCAAATTGGTCAAACTGAGGCACTTGGATTTATGGGGTTGT GATGATTTGATTGGTGTGCCATTGGGAATGGATAGGCTAATTAGTCTT AGAGTACTGCCATTCTTTGTGGTGGGTAGGAAGGAACAAAGTGATGA TGATGAGCTGAAAGCCCTAAAAGGCCTCACCGAGATAAAAGGCTCCA TTCGTATTAGAATCTATTCAAAGTATAGAATAGTTGAAGGCATGAATG ACACAGGAGGAGCTGGGTATTTAAAGAGCATGAAACATCTCACGGGG GTTGATATTACATTTGATGGTGGATGTGTTAACCCTGAAGCTGTGTTG GCAACCCTAGAGCCACCTTCAAATATCAAGAGGTTAGAGATGTGGCA TTACAGTGGTACAACAATTCCAGTATGGGGAAGAGCAGAGATTAATT GGGCAATCTCCCTCTCACATCTTGTCGACATCCAGCTTTGGCATTGTC GTAATTTGCAGGAGATGCCAGTGCTGAGTAAACTGCCTCATTTGAAAT CACTGGAACTTTATAATTTGATTAGTTTAGAGTACATGGAGAGCACAA GCAGAAGCAGTAGCAGTGACACAGAAGCAGCAACACCAGAATTACC AACATTCTTCCCTTCCCTTGAAAAACTTACACTTTGGCGTCTGGACAA GTTGAAGGGTTTTGGGAACAGGAGATCGAGTAGTTTCCCCGCCTCTC TAAATTGGAAATCTGGAAATGCCCAGATCTAACGTCATTTCCTTCTTG TCCAAGCCTTGAAGAGTTGGAATTGAAAGAAAACAATGAAGCATTGC AAATAATAGTAAAAATAACAACAACAAGAGGTAAAGAAGAAAAAGA |
| | AGAAGACAAGAATGCTGGTGTTGGAAATTCACAAGATGATGACAATG TCAAATTATGGACGGTGGAAATAGACAATCTGGGTTATCTCAAATCA CTGCCCACAAATTGTCTTACTCTGTTGGACTCACTCGAACTTTCAAAT ATAGAAGACCAGGAAGATGAGGGCGAAGACAACATCATATTCTGGA AATCCTTTCCTCAAAACCTCCGCAGTTTGGAAATTGAAAACTCTTACA AAATGACAAGTTTGCCCATGGGGATGCAGTACTTAACCTCCCTCCAAA CCCTCTATCTACACCATTGTTATGAATTGAATTCCCTTCCAGAATGGA TAAGCAGCTTATCATCTCTTCAATCCCTGCACATAGGAAAATGTCCAG CCCTAAAATCACTACCAGAAGCAATGCGGAACCTCACCTCCCTTCAG ACACTTGGGATATCGCGGTGTCCAGACCTAATTGAAAGATGCGAAGA ACCCAACGGCGAGGACTATCCCAAAATTCAACACATCCCCAAAATTG TAAGTCATTGCAGAAAGTAATTTATTCATTTATATTTATTTATGCTTA GAATGATATACACCGTCGTCCTTTGGTTTCAAATCTTGAATTTGGTTTT TGTTTTCTTTCTTTGTTTCTTTATTCAACACCAGCCCATTTATGATTGAT TCATTAAAAAAAGGATGGAGTTTTGTGGATTTGAAGAAGACAACGAA TTGAGATTCCTGGGGTTTTCTTTTTGTTGGGGTTGGATTTCATGTATAT GTTGCTGATTAAATACGAGACTGATGATGATGATGATGTGTTTATGGG TTTTAAATCAGATTAAATATATGGGAAATGTAAGTTAATTTGGGATGC ACATAAGGTGTTTGCTGAAATGTCTATGAGAAATGTTGTTTCTTGGAC TTAGAATGATATACACTGTCGTCCATTGGTTTCCAATCTTACATTTGGT TTGTGTTTTCTTAGTTTGTTTCTTTAATCAACACCAACCCATTTTTTTAA AACTACCTGCAACTACTAATTTACGTTGACCCTGTATCTCAGGTACTA AATGAATATTGGTGATTTTCAGTTACTCAACACTAGCTTGATCCTGAA CGCACCCAACCTTCAGGTTAGAATCCGGCTTACTCATCCTTTTGTCCA GTTTTCAAGTAATTGTTTTGGCAGGATCAATTCTCTAATTGTTGTACAC CGTATATTGCAATTTATAGTGACTACAGTTAATGAATGTTTACAAAAA ATTAGTCATGTAAAAACTTCTTCTCTGTCCATTACATAAACTCTTTTTC TCTTTCTAACTTATCATGTTCATGTCTAAAAAATTAAACATGCTCACAT CAATGTTCATTTAAGCTAACTTACTTCTGTAAGAGAGCGAGCTAGTTA AAAACTCCTTTAACTTTCTGTTTTATACTCAGGACATGGATTGATGCA AGCATGAAGAACTTCGGGAATTTGCTAAAACTCTACCAAAGCGATGA GAGTTTGGACTTTGTTTCACTTGAAGTCAGGGACTGTCAACAAAGCCA CAGTGTGCATGTTGGCTGTTTCACTTGGACGATAAAAAGGTTTATTTA ATTGTTTTCCTAAGTGTATTTGGCTTACAAGCTTTTACTTTTCGCTTGA AAGGGTTTTTCTTGTTTTAAGCTTTTGAATTAGAGTTTCGGTTGAAGT AAGAGTAGTCGTATTAGTCTTTTACTTTGCAGGAGTCTATGCCTATAT AAGGTAAGACTCGTATTTGTAATTTTCAGATTATGCAATTCAAGTTTT CGAGTGTTTTCTTAAAAAAACATATCATACCTGTGTGTAGCATAAAGA TAAATTCTGATGCTGTGCTTCTTGTTATGGCTCACATTGGTTTTCATTG TTTGGATTGTTTCACAGGGAAGCAGAGAATACCTGGAACCTGTAAAG GACGCTATATCTATAGCAGCTTTCACAGGCCAAGTAATATAATGTTCT TTATCAATCACTCAATACCTGGAGATTGTTTGAACACATAATTCACCT CTTTTTTTCCATCTCAAATTGCAGACTTTTACTGGGATTTGAATGACAG CAAACATTTGATTGTCACGAGGATTGATCTAACTGGTGAATGTTGTGA TCACCATATTGGTCTTTCTCATTCAAACAAACTTGCATACCGTTCTTTT GAAGTTTGAAGACAAGCAGGCGATAATCAACTCAGGTCGAAGCTCAT GTGCAGGAGAGAATAGAATATAGAGGAGATTATTTTAAAAGAAACG CTCAAAGGTATTGTAGCACCTTTCCTATTTGCTACTGCAGTGATGTTTT TATTTCTTGTTTGCAGCGCTTGTTTCTCATATTTCAACCTACTTTTAGA AAAAAAAACATCTCCGAACATAACCAAAAATAAAGTTCCCTTATCAG TGCTTTCCCTGCTTTCTTCTAAACAACATATACAATTATAAACCCTTTT TCTCTCTTACCTTTGTTATTCTTCCTTGCTTCATTGAGATAACACTCTCC TGTTTTTGTTTTGTTGTTAGTCATTACATGGATATATCAAGGACAACA GTTCTGTAGTCCGTCAACTGTGGTTAGGAAGGCTAAACTGGAGCACA ATAACCCCATGTCAATTGAATAGTAAAGGTGTGCTATATCAGTTTCGT TTGGCTTGGCTTACCTGAAAAATGGCTGGTTATTTATCCTTGTCTCTTT |
| | CTATGACGTGCAGTGGCTTGTTAATGTGTCTCGGACAACAATTCCTCA CTTTCCAAGTTCCATACACGCTGATGTAACTATCTTCTGCAGTCTGTTC TTTCATTTTTGCCACGTGCTCTAATTATAACTTTTTGTACTCAATAATC AACTCCTTGTCCCGGTATTTGCAGAGACTACTTAAACAGGTAAAGTGA CAATCCTGGCGAAGTTGTCTGTTTCTTAGCTCTGAACCCATCATTCAG GTAAGATTAAGTATTTAAGAAGAAATTTTGTTTTTACCTAAAATGAAT GATCTTGTGTAACTGCTTGCTTCTTGCATTAAATAAGAACTTTCTGCTG CATATGTGACAGTTACATCCACAAAAAAGTTGGAGGTTTGTTCAGGG ATTGGAAATGAAGGTACTTCAGAATTCCTGGAATGTTTATGAATGCTC CAGACTTCAGAGTCTTTAATGGAAAATTCGAGTCACTAAAAAAACAT TATTCCTATCATCAGAGCTTTGAAGTTCCTCTATACAAGGTCAACTGA GTTCCTCTTTGCCTCTTGTTTAATTGTATTTACTTGTACCTTAATTATAA TCTGTATATTGTTTTAGTTAAGTTCTAAAACAGGTTATTATTCATCATT TGTGCAGCATATTGCTGGAATCAAGAATCTAGTGCTTCTTCTTCCTGA CTTCACTGTCAAAATAGCAGTTCCCATGCTGGAAATAAGCGCGCTAGC AATGTAATTGATGACATGGATGTTGCTGCTTCTGAGTTTTGATCATAA AAAGCTGTTATGTGTTTCTTGAATGTAATGAAGGAGAGGAGAAAACT GAAAACTCTTGGCAAAAACGTGAAATTGCAGTGCCTCGGGGTGGTAG GGATCACCCGGATTCAGTTCAGACGATACTTTTTTCAACCCGGTTTGT TCCGGTTTTCAGCTTCGGGTTTTCCTTGTACTTTTGGGGCACACATACA AGGCTACTTCATGTTTGAGAAACCTAATTGAGGCTATCTTGTAGCAAA TGCACACCACACTCTTTCTCTCTTCTCTCCTTTTTTCACCTTCCATTTGT AAAAATCCTCTTTTAAAGGTTAATAAAAAAAAGCTTCAAGTCTCGTAG GGTGGATGTAGGTCACATTGACCGAACCACGGTAAACTCTTTGTGTTC TTTCTTCTCTCTTTGTTTCTCATTTTTACGGCAAGTGTTTATGGTTAACC ATGCATCTTAGAATAGCTTAAGGCATTAACATAATAACATCAATGTTC TCCAAAGATTCACCTTACTTGTTGTACATAATCACAATGTTAAGCCTA TGAAGGTAGAATGCTCTCATGATTTGGTTTAACCAAAAAATAAACTCT AAAATAATACGGAGTAATAAAAATTGGCCATAAATTATTTACAAAGT TTGATTTTTGTATAGGGTATCTTGTACTTGTGATAAAAAAAATTAAAA AAAAAAAAATTACTTATTTCTTCTATTTTTACTTGTTACACTTTTCTAC AACAGAAACATCAAAACGCCCGCAACACACTATAAATGAAAAACCAT TTTGTATGCAATGATATTTACGTTCTCACTTTATTCTCTTTAATAACTC CTACTACGTAATTCTCACCAATCAAATAAAATTATAGAAATTTTCATT TATACCCTCTTAAAATGATGTTGATTTNNNNNNNNNNNNNNNNNNNN NNNNNNNNNNNNNNNNNNNNNNNNAGGTGTGAAAAAGTTTGTGTTATA AGTTACAACAATTTAAAAACGGAGAACATACTTATAATACTAGTGTA ATCTTTGGCCGGATTATGGTCGTTGACAAAAAAACTCCGGCCTTGACC CTCCACGTGCCGGTCAAGTGACTTAATAAACCTTTTATTCCACCCTTTT TTCATTCTTCTTTTTATTCTTCTTTTTCTCTCCATTAATACAAATCAAGT GATTATGTCGATCCGATCCTTCTGTTCTCTACTGTAATTGATTACACCA ACAACAACCAAGCGAAACAGTCAATGTTACCGAATTGAATTGCGGAA AATAGTTTATGATTGATTCATTAAAAAAGGATGGAGTTTTGTGGATTT GAATAAGACAACGAATTGAGATTCCTGGGGTTTTCTTTCTGTTGGGGT TGGATTTCATGTACTTGTTGCTGATCAAATATGTGATTGAAGATTGAA GATGATGATGTGTTTATGGGTTTGAAATCGGATTAAATTTATGGGAAA TGTAAGTGAATTGGGGATGCACATAAGGTGTTTGATGAAATGTCTATG AGAAATATTGTTTCTTGCACTTGTATGATAATTTGTGGGGATTTGATT AGTTCATGGTGCGAGTTTGATGCAACGCCGAAGAGAAATCAGGTCTT TAGAGAAACTCAAATGGTTGAATAGGCTTCCAACAATGTTGTTTCGTA GTACACTGCCATGATTGATAGAGAAGGAAGCAGATGAAGGAGAAGG CTCATGTGACCGGCACATGGGTAGTAAGTCAACGCCGGAAATCGTGG TCAACGTCCAAAACCGAGCTAAGGTAACATATTGGAGTACAAGTAAT TACAACAAAAAGTGTTACTTCCTTGTACATTATTATTTTACTTGAAAT GCTAGTTGTGTTTGTGCATCTGTGGAACTCTAAATTAATTAATTAACA ATCAACCAAACAACTTTAGTGTAAATTGGCCAACCTTTTGCCATCAGC |
| | CACAGAAAAGTGAAATATCACCCCATTATTGCCCATTCTGTATTTGCA CTTTTTTTTAAGGCATAGCACAGCCGGTTTTCCGGATCTTAGCTCCGTC TACATTCGGATCCGATCCATTTCGCACACCTTATTTGTGGTGGATGAG TCTCCCAACAAGAATTTCTCGCTCGAAACTGAGAACCCCCTTAAGCGG CATCAAGTTGCTTACCACTTGAGCCAACTCTATGTTGGTTTCTGCATTT GCAGTTAGTTAGGTCGTCTGAGTGCGAAATGGGAATGCTTTATCACAC ACTCCACAGTTTAGTCAGGCTGATGGAAACGTAATAATTGAGTTATTT GAGTGTTCAAACTTAAAGTCACTCACTCACTCAAACACTCAATACTTT CTCCATCTTGTTTCTCATTACATATGAAAACCCAAACACCTTTCATTT CTGCTTAATCTTCTTTCTCTCATCTTTCAGTTATTCACCTGTTCATCTTT CTGAAAACAACCCAAACACCCTTCATTTCTGTTTAATCTTCTTTCCTCA TCTTCATCCACCTGTTTATCTTTCTGTAAACACAACCCAAACACCTTTC ATTTCTGATTTATCTTGTTATCTCATCTTCATTCACCTGTTCATCTTTCT GAAAATCTAAACACCCTTCATTTCTGCTAATCTTCTTTTCTCATCTCCC CCTAAATCATCTTTCTGAAAACCCAAACACCTTTCTTTTCTGCTTTTAT CTTGTTTTCTCATCTTAATTCATCTCTTCATCTTTCTGAAAAACCCAAC CCAATGGCTGAAATCGGATACTCGGTTTGTTCAAAACTTATTGAAGTG ATGGGCAGTAATATCATTAAAGAGATTCGCGACATGTGGGGTTACAA TTCTCATCTTGAAGACCTCAACAAATCTGTCTTGACGATCAAGGATGT GCTCTTGGATGCTGAGGCGAAGCGGGATCTTTCCCGTGAACAACAGA GTTACATTGCAGAACTTAAGGATGTTGTTTACGATGCTGATGATTTGT TCGATGAGTTCCTCACTCTTGCTGAGCTCAAACAGATTGATGGAAACA AGGGTGGTGGTAAATTCTCCAAAAAGGTACGTCGTTTCTTTTCTTCTA ATAAGGAGAAGATGGGTCAAGCTTACAAGATGTCTCATATGGTTAAA GAAATTAAGAAGCAGTTGGGTGAAATTGTTGATAGGTATACCAAATT TGGGTTTATTGTTGATTATAAACCTATTATTAGGAGAAGGGAGGAAAC ATGTTCTTATTTGTAGGTGCCAAGGAGATTGTTGGGAGGGATAAGGA TAAAGATGTTATCATAGGCATGTTGCTAGATCATGATAACGATTGTAG TTTCTTGGCTGTTGTGGGGGTTGGAGGGGTGGGAAAAACTACTCTTGC CCAACTTGTGTATAATGATGAAAGAGTCAAAAGTGAGTTCCAAGATT TGAGGTATTGGGTTTGTGTCTCTGATCAAGATGGGGGACAATTTGATG ACAAAAGAATTCTTTGTAAGATTATAGAGTTAGTTACGGCCAGATTC CTCCGAGTAACGAGAGCATGGAATCGGTGCGTAAGAAATTTCAAGAG GAATTAGGAGGAAAGAAGTACTTCCTTGTTCTTGATGATGTATGGAAC GAGGATCGCCAGAAGTGGCTTCATCTAGAAAATTTCTTGAAATTGGGT CAAGGGGGAAGCAAGGTTGTGGTAACCACACGTTCAGAGAAGACGG CAAATGTTATAGGGAAAAGACAAGACTATAAACTAGAATGTTTGTCA GCAGAGGATTCATGGCGCTTATTTGAAATGTCAGCTTTTGACGAAGGG CATGGCCAGGAAAACTATGACGAATTAGTGACGATTGGCAAGAAGAT TGTTGAAAAATGTTATAACAATCCACTTGCTATAACAGTGGTAGGAA GCCTTCTTTTTGGACAAGAGATAAATAAGTGGCGGTCGTTTGAAAACA GTGGATTAGCCCAAATTGCCAATGGTGATAATCAGATTTTCCCGATAT TAAAGCTCAGTTACCACAATCTTCCACACTCCTTGAAGAGCTGCTTTA GCTATTGTGCAGTGTTTCCCAAAGATAATGAAATAAAGAAGGAGATG TTGATTGATCTTTGGATAGCACAAGGATACATTATACCGTTGGATGGA GGTCAAAGTATAGAAGATGCTGCCGAGGAACATTTTGTAATTTTGTTA AGAAGATGTTTCTTTCAAGATGTAAAGAAGGATTCTCTTGGTAATGTT GATTATGTTAAAATCCACGACTTAATGCACGATGTCGCTCAAGAAGTG GGGAAGGAGGAAATTTGTGTAGTGACTTCAGGTACAAAGAAGTTGGC TGATAAAATCCGTCACGTGGGTTGTGTTGTCGATAGAGATCCAGAAAT AGTCTTTTTATGTAGCAATAAGATTCGTTCGTATATTAGCGGTCGTTGT ATAAAGAATCCGGTGGATTCACAAATAGACAACTGGATGCGCCTTAG GGTGTTGGACTTGTCAGATTCATGTGTTAAAGATTTGTCTGATTCAAT AGGTAAGCTGCTGCACTTAAGGTATCTTAACCTCTCTTCTAATATAAA GTTGGAGATAATCCCTGATGCAATTACAAGACTGCATAACTTGCAGA CACTACTTTTAGAAGATTGCAGAAGTTTAAAGGAGTTGCCAAAAGAT |
| | TTTTGCAAATTGGTCAAACTGAGGCACTTGGAATTACAGGGTTGTCAT GATTTGATTGGTATGCCATTTGGAATGGATAAGCTAACTAGTCTTAGA ATACTACCAAACATTGTGGTGGGTAGGAAGGAACAAAGTGATGATGA GCTGAAAGCCCTAAAGGCCTCATCGAGATAAAGGCTCCATTTCTA TCAGAATCTATTCAAAGTATAGAATAGTTGAAGGCATGAATGACACA GGAGGAGCTGCTTATTTGAAGAGCATGAAACATCTCAGGGAGATTGA TATTACATTTTTGGGTGAATGTGTTAGCCCTGAAGCTGTATTGGAAAC CTTAGAGCCACCTTCAAATATCAAGAGCTTATATATATATAATTACAG TGGTACAACAATTCCAGTATGGGGAAGAGCAGAGATTAATTGGGCAA TCTCCCTCTCACATCTCGTCGACATCCAGCTTAGTTGTTGTAGTAATTT GCAGGAGATGCCAGTGCTGAGTAAACTGCCTCATTTGAAATCGCTGA AACTTGGATGGTTGGATAACTTAGAGTACATGGAGAGTAGCAGTAGC AGTGACACAGAAGCAGCAACACCAGAATTACCAACATTCTTCCCTTC CCTTGAAAAACTTACTTTACAGCATCTGGAAAAGTTGAAGGGTTTTGG GAACAGGAGATCGAGTAGTTTCCCCGCCTCTCTGAATTGGAAATCAA GAAATGCCCAGATCTAACGTCATTTCCTTCTTGTCCAAGCCTTGAGAA GTTGGAATTGAAAGAAAGCAATGAAGCGTTGCAAATAATAGTAAAAA TAACAACAAGAGGTAAAGAAAAAGAAGAGAACAATAATGCTGGTGT TGGAAATTCACAAGATGATGACAAAGTCAAATTACGGAAGATGGTGA TAGACAATCTGGGTTATCTCAAATCACTGCCCACAAATTGTCTTACTC ACCTTGAAATTGAGGAGTTTATTGAATCAGATTCCGAGGAAGAGATT GAATCAGAAGTCGGGGAGGAGGAGGTTGAATTGGAAGTTGTGGAGG CATTTCAGAAGTCTGCATCTTCTCTGCAAAGCCTCGAAATATACAGAA TAAATAAACTGAATAGACTAACTGGAACAACAGGATTAGTGCATTTC AGTGCCTTGGACGAACTCACATTGAATTTTGTCGACGATTTTGAAGTA TCCTTTCCTCAAAGCCTCCGCAGTTTGAAAATTGAATACTCTTATAAA ATGACAAGTCTGCCCATGGGGATGCAGTACTTAACCTCCCTCCAAACC CTCGAACTAAAATGTTGTGATGAATTGAATTCCCTTCCAGAATGGATA AGCAACTTATCATCTCTTCAATCCCTGTCCATATCCTACTGTGAAGCC CTGAAATCACTACCAGAAGCAATGCAGAACCTCACCTCCCTTCAGAG ACTTGTGATAAGAGAATGTCGAGACCTAGCTGAAAGATGCGAAGAAC CCAATGGGGAAGACTATCACAAAATTCAACACATCCCCAAAATTGTA AGTGATTGCGGAAAGTGTTTCTTTTATTTATTTTTAATTTTATGCTTAG AATGATATACATCAGCATTCAGCGTCCATTGGTTTCCAATCTTACATT TGGTTTTTGTTTCTTAGTTTGTTTCTTTAATCAACACCAGCCCATTTTTT TTAAACTACCTGCAACTACTAATTTTCATTTACCCTGTATCTCAGGAA ATATGGTAGTATTCTCATTTACTCAACACTAGCTTGATCCTGAACGCA GCCAACCTTCAGGTTAGAATCCGCCTTACTCATCCTTTTGTCATGCATT GTTTTAAGTTGTTTTGCTTGCTTGTGTAATCATAATTCATAGTATACGA TTCATCATTCACTATGTCTACAGGCAAGATATTGGAATTGTTCACGAT TCCCTGAAGTTTCTTTGTTTTTGTTGATACCACCATATTGCAGCTTATA GTGACTAAGTTAATGAATGTTTCCAAAAAATTAGTCATATAAATTCTT CTTCTCTCTCTATTACATAAACTCTTTTTCTCTTTCTAACTATCATGTTC ATGTCTAAAACTTATACATGCTCACATCATTGTTCGTTTCAGCTGACTT ACTTCTGTAAGAGAGCTATCTAGTTAACAACTCTTGTAACATTTTATTT GCTAGTCAGAACATGGATTGGTGCAAGCATGGGAATTTGCCAACACT CTACCAAATCGATTGGAGTTTGGACTTAGTTTCACCAGAAGCCATACC CGGACACTTACTGGGGACTGTCAACAAAGCCGCATTATGATGTACTTG GATGTTTCACGTGCCTGAGGTGTGAGTTACTTGGAAGGGAAGCGGTTT ATTTAATTGTTTTCCTAAGTAGATTTTGCTTAGAAGCTTTTACTTTTCA CTTGAAAGGGTTTTTCTTGTTTTAAGCTTTTCGAATTAGAGTTTCGGTT GCATTAAGAGTAGTCGTATTAGTCTTTTTTTACCTAAGGAAGACTTTTT TGTAATTTTCAGACGATGCAATTCAACTTTTCGAGTGTTTTGTTGCTTG TGTGATTGTGAGTTTGTGAATTTGTCTTTCATAAATATTGAGTTCATCA GAAGCTTTATGCTCCACCGGTAGTCTAGTACCTTTTGTTATTGTTCAGG GAAGTAATCTGGTACCTTCTATATATATGAGAAAACATACATTATGCA |
| | AAATTCTTACAGGTTAGTTACTTCCTAGAACTTCAGTTATACTTTTTTT TTGTTCCATGTCCTTGGAATCAAGTCATTCCCTCTGAAAAATGTGTAC TGAACTTTTGAAAGTTGCTGTTTGATTCCTGTTTGAATCTTCACTTTTC TTGCATCGTGACAGCTGTGTTTACAATGAAGTTTAAGCAGACACTCTC TTTATATAGTGCCTCCTTTTGGAGCATCGGAGAGTTGTGGCTGATCAC TATGTGCGACCAAGAGATTCATTAATCGCGTGTTTGATCAGGTAAAAG TTTTTATGTCAATGTGTTTTATTTTTCTTTCTGTTTGATCAGTTTATGTC TGTATTCAGATTCTTATCTTCTTCTAGTAGCATAACAAATTTGTTTGTT TCATTATATAAACCGTTTCAGGATTACAAATGATCGGACAGAGATGTA TGCTTCAGTCGATATTGATGATAACTTAAGGTAGTATTGCTAGAACAG TTACAGAGCTGTGGCTGATCACTATGTGCTGCAAACAGATTCATCAAT CACGTGTTTGATAAGGTAGAGTTTTCATGTCAACGCGTTTTTTCTGTTT GATCAATTTATGTCTGTATTCAGATTCTTATCTACTTCTAGTAGCATAA CATATCTGTTTCTATCATTATATAATTGTTTCAGGGTTACAAATGACCG GACAGAGATGTATGCTTCAGTCGATATTGATGCTAACTTAAGATAGCA TTGCTAGAACAGTTTCAGGTTGCCATTGAAATTTGAAAACAGAAAGA CACCATCAGGTAGAGTTTTCATGTCAATGCTTTTTTTTTTTGATCAAT TTATGTTTGTATAAAAATTTGTATCTTCTTCTATACTATAAATTCTATA TAACGTATCTGTTTATTTCATTATAATAAACCGTTTCAGGATTACAAA TGATCGAACAGTGATGTATGCTTCAGTCGATAACTTCAGGTAGCATTG CCAGAAGAATTGCAGACACATCCTAACTTAAGAGGGTTATGGTTGAT TGACTAACTCTCGAAATTCTAGTTAGGCAAGAGGAGCATTGCAGTAC CTGCCTTAAAAGGGGTCGTCTTATATAGATATCTCTATCAGTAGTCAT TTACGTCTTAAGTCCTGAAATAAGTTGAACTAAACATACTTTGTTTGA ATCGAAGATTTAGTGAATTTTACTTTGTATTTGATTGTGTTGAGATGA CCGTAGGGAAAAGTTAACTAATTATAAGCGTAAATTATGTTCTTGGAT TCGGCTTTTATATTTCTTTTCGTTTAATAAAAGTGGTCATAGAGATAT TCTACAATGTATTTTGGTAAGTTACCTAAAATTTAATTTGATTATTTTT CACATAATTAATCAACTGATAAAATTTTATTTGGATCAACCGATCAAG AAGTGAAAACGTAAAGAACAAAAAGAAACAGAGGGAGTATATTATT ATCTTTTACTTTTGATAGTGAAGGATGTTATGATATTTTAACTCAATCC CTTACAAAATATACTGGTTAAATTTCTTAACAATGTAGTACTTTGGCA ACAAGTTCAGGTTGAAAGCTTTGAGAAATAGAGTTAGGAAACATAAG AATCACAAAATTTATGCTTCTCTCATCTGTGAATCAAAACACAAATTC TTATTTACAAAGGTTGTACAATAATTATTGTACACCGAAGTAAAAGTT AACTCAAAATGCTTAAAAGTTAGGCTTATATATGTAAAAGTTATCTAT TGTTTAGTGATAAAAAAATTTCATTTTAATAAAACTTATTTTTTCAAAAT CACTAATAATGTATAAAATTAATCATTTAATTATTTAAAATATTTATCT ATCAAATTTTTTTACTAATATAAAAGTTACTCAAAACTAGGTTAAAAT TACAAAAAAATGGATTAAAGTTATTTGGTGTACAATAAATTTATTGT ATACTTTGTGCGCGC |

The present invention will be further clarified in the Examples that follow and that are given for illustration purposes only and are not intended to limit the invention in any way.

### EXAMPLES

### EXAMPLE 1

### Testing for resistance to CMV_in spinach plants

The resistance to CMV infection was assayed as described in the CPVO protocol for tests on distinctness, uniformity, and stability for spinach as available on: http://www.cpvo.europa.eu/documents/TP/vegetales/TP 055-5Rev Spinacia oleracea.pdf. Spinach seeds of the invention together with seeds of varieties Viroflay and Polka were sown in 5 x 5 cm soil blocks and under a cultivation regime with a day temperature of 20°C and a night temperature of 18°C and receiving at least 16 hours of light. When the plants had developed three true leaves they were inoculated with CMV. Inoculation was done by dusting the leaves of the plants with carborundrum powder and subsequently rubbing the leaves with a sponge soaked in inoculum. The inoculum was a dilution (1:10) of isolates NL 16 and SP 43 in water. After inoculation plants were slightly rinsed with water.

Symptoms may be observed 7 to 9 days after inoculation. A resistant plant, i.e. a plant comprising the allele of the invention, shows no symptoms, while a susceptible plant typically shows dwarf growth and mosaic symptoms in the heart of the plant.

Plants for this specific test were scored as resistant or susceptible based on the development of symptoms. Plants exhibiting no symptoms were in this specific test considered as resistant. Plants that showed symptoms of infection were in this test scored a susceptible.

All plants of varieties Viroflay and Polka were scored as susceptible since they showed symptoms such as dwarf growth and mosaic symptoms in the heart of the plant, while plants of the invention showed no symptoms and were scored resistant.

### EXAMPLE 2

### Transferring CMV resistance into a plant of variety Viroflay

In seeds of deposit NCIMB 42651 the alpha-CMV allele is heterozygously present. Seed of this deposit was grown into mature spinach plants which were individually selfed to produce an F2 population.

Fifty F2 seeds were used to develop plants for a disease test as described in Example 1. F2 plants that scored resistant in the disease test were further grown to maturity and again individually selfed to obtain for each plant a F3 population. Each F3 population was again subjected to a disease test as described in Example 1. Plants of the F3 populations that scored completely resistant in the CMV disease test can be considered to comprise the alpha CMV allele homozygously.

One of the plants that is homozygous for the alpha CMV allele was used to cross with a plant of variety Viroflay which is susceptible to CMV to obtain F1 seed and a subsequent F1 plant grown from the F1 seed. The F1 plant was selfed to obtain an F2 population of seeds and plants. This population was again subjected to the disease test as described in Example 1. The disease test showed that the segregation pattern for CMV resistance is 3:1 which is consistent with that of a dominant inheritance.

### EXAMPLE 3

### Amplification of the LRR domain-encoding region

Identification and selection of CMV resistant spinach plants carrying the Alpha CMV allele can also be efficiently done by screening the DNA for the presence of the alpha CMV allele. For example by amplifying and optionally determining the sequence of the LRR domain of alpha CMV allele as described below.

The isolated genomic DNA of spinach plants from the F2 population as obtained in Example 2 was amplified using forward primer ACAAGTGGATGTGTCTTAGG (SEQ ID No:6) and reverse primer TTCGCCCTCATCTTCCTGG (SEQ ID No:7). The primer pair amplifies the LRR domain-encoding region of an alpha-*WOLF* gene to which the alpha-CMV allele belongs, and has been designed for selectively amplifying part of an alpha*-WOLF* gene, and not of other CC-NBS-LRR protein-encoding genes.

PCR conditions for amplifying the LRR domain-encoding region of an alpha*-WOLF* gene using primers having SEQ ID No:6 and SEQ ID No:7 were as follows, using Platinum Taq enzyme (Thermo Fisher Scientific):
- 3 minutes at 95°C (initial denaturing step)
- 40 amplification cycles, each cycle consisting of: 30 seconds denaturation at 95°C, 30 seconds annealing at 60°C, and 30 seconds extension at 72°C
- 2 minutes at 72°C (final extension step)

The isolated genomic DNA of spinach plants from the F2 population as obtained in Example 2 was in parallel amplified using forward primer TCACGTGGGTTGTGTTGT (SEQ ID No:8) and reverse primer TTCGCCCTCATCTTCCTGG (SEQ ID No:7). This primer pair amplifies the LRR domain-encoding region of a beta-*WOLF* gene, to which the beta-WOLF 0 allele of Viroflay belongs, and has been designed for selectively amplifying part of a beta-*WOLF* gene, and not of other CC-NBS-LRR protein-encoding genes.

PCR conditions for amplifying the LRR domain-encoding region of a beta-*WOLF* gene using primers having SEQ ID No:7 and SEQ ID No:8 were as follows, using a Taq enzyme:
- 3 minutes at 95°C (initial denaturing step)
- 40 amplification cycles, each cycle consisting of: 30 seconds denaturation at 95°C, 50 seconds annealing at 58°C and 50 seconds extension at 72°C
- 2 minutes at 72°C (final extension step)

The PCR products were visualized on agarose gel (not shown), and DNA was purified from the PCR reaction. Subsequently the sequence of the PCR products was determined using methods well known in the art.

The sequence of the LRR domain of the alpha-CMV allele amplified by primers having SEQ ID No:6 and SEQ ID No:7 is provided in **Table 2** under SEQ ID No:9.

The sequence of the LRR domain of the beta-WOLF 0 allele amplified by primers having SEQ ID No:7 and SEQ ID No:8 is provided in **Table 2** under SEQ ID No: 1 1.

The PCR products were visualized on agarose gel (not shown), this demonstrated that approximately 25% of the plant only contained an alpha-WOLF fragment, approximately 50% contained both an alpha- and a beta-WOLF fragment, and that the remaining approximately 25% of the plants only contained a beta-WOLF fragment. The plants containing the alpha-WOLF fragment completely correlated with the plants that scored resistant for CMV. The plants only comprising the beta-WOLF fragment completely correlated with the plants that scored susceptible for CMV.

DNA from the PCR reaction was purified, and subsequently the sequence of the PCR products was determined. The alpha-WOLF PCR products gave a sequence that corresponded to the sequence of SEQ ID No:9, the genomic sequence of the LRR domain of the alpha-CMV allele. The beta-WOLF PCR products gave a sequence that corresponded to the sequence of SEQ ID No:11 the genomic sequence of the LRR domain of the beta-WOLF 0 allele.

Finally, the obtained sequences were translated into the corresponding amino acid sequence of the LRR domain having SEQ ID No: 10 and SEQ ID No: 12 for the alpha-CMV allele and the beta-WOLF 0, respectively (See also **Table 2**).

### EXAMPLE 4

### Creating hybrid and parent lines with CMV resistance

One of the WOLF alleles present in Racoon (51-317 RZ) is beta-WOLF 3 having SEQ ID No:13, which confers resistance to *Peronospora farinosa* f. sp. *spinaciae* races Pfs:1, Pfs:3, Pfs5, Pfs:9, Pfs: 11, Pfs:12, Pfs: 14, and Pfs:16. Next to the full resistance against these races, Beta-WOLF 3 provides intermediate resistance against Pfs:8. The goal of this experiment was to create a stable hybrid in which the beta-WOLF 3 allele is combined with the alpha-CMV allele of the invention.

A plant comprising the alpha-CMV allele homozygously, e.g. a plant as obtained in Example 2, was crossed with a plant of the male parent line of hybrid Racoon. F1 Plants were subjected to a CMV disease test as described in Example 1 and a resistant plant was selected. The selected plant was crossed again with a plant of the male parent line of hybrid Racoon to obtain a BC₁ population. The BC₁ population was subjected to a CMV disease test as described in Example 1 and again a CMV resistant plant was selected. This was repeated three more times until a BC₄ population was obtained.

Again, a resistant plant was selected from a CMV disease test and this plant was selfed. From the obtained S₁BC₄ population a plant homozygous for the alpha-CMV allele was selected by determining the sequence of the LRR domain of the WOLF gene as mentioned in Example 3.

The selected homozygous plant was further inbred to obtain a population that could serve as an alternative CMV resistant parent line for Racoon.

Plants from this CMV resistant parent line were crossed with the female parent line of hybrid Racoon to produce seed for a new hybrid variety resistant to CMV conferred by the alpha-CMV allele of the invention in combination with downy mildew resistance conferred by the beta-WOLF 3 allele.

In a similar fashion hybrids can be created in which the alpha-CMV allele is combined with an alpha/beta-WOLF allele conferring resistance against one or more downy mildew races.

## Claims

1. An allele designated alpha-CMV which confers resistance to CMV when present in a spinach plant, wherein the protein encoded by said allele is a CC-NBS-LRR protein that comprises in its amino acid sequence: a) the motif "MAEIGYSVC" at its N-terminus; b) the motif "KWMCLR"; and c) an LRR domain that has in order of increased preference at least 95%, 96%, 97%, 98%, 99%, 100% sequence similarity to SEQ ID No:10.

2. The allele of claim 1, wherein the allele has a genomic nucleotide sequence which in order of increased preference has at least 80%, 81%, 82%, 83%, 84%,85%, 86%, 87%, 88%, 89%, 90%, 91%, 92%, 93%, 94%, 95%, 96%, 97%, 98%, 99%, 100% sequence similarity to SEQ ID No: 1.

3. The allele of claim 1, wherein the allele has a coding sequence which in order of increased preference has at least 95%, 96%, 97%, 98%, 99%, 100% sequence similarity to SEQ ID No:2.

4. The allele of claim 1, wherein the allele has a coding sequence which in order of increased preference has at least 94%, 95%, 96%, 97%, 98%, 99%, 100% sequence similarity to SEQ ID No:3.

5. The allele of claim 1, wherein the allele encodes a protein having an amino acid sequence which in order of increased preference has at least 95%, 96%, 97%, 98%, 99%, 100% sequence similarity to SEQ ID No:4.

6. The allele of claim 1, wherein the allele encodes for a protein having an amino acid sequence which in order of increased preference has at least 95%, 96%, 97%, 98%, 99%, 100% sequence similarity to SEQ ID No:5.

7. Nucleic acid encoding a CC-NBS-LRR protein that comprises in its amino acid sequence: a) the motif "MAEIGYSVC" at its N-terminus; b) the motif "KWMCLR"; and c) an LRR domain that has in order of increased preference at least 95%, 96%, 97%, 98%, 99%, 100% sequence similarity to SEQ ID No:10, wherein the nucleic acid in particular has a genomic nucleotide sequence which in order of increased preference has at least 80%, 81%, 82%, 83%, 84%,85%, 86%, 87%, 88%, 89%, 90%, 91%, 92%, 93%, 94%, 95%, 96%, 97%, 98%, 99%, 100% sequence similarity to SEQ ID No: 1.

8. Method for identifying or selecting a spinach plant carrying the allele as claimed in any of the claims 1 to 6 or a nucleic acid as claimed in claim 7, comprising determining the presence of a genomic nucleotide sequence in the genome of a plant, wherein said sequence has in order of increased preference 80%, 81%, 82%, 83%, 84%,85%, 86%, 87%, 88%, 89%, 90%, 91%, 92%, 93%, 94%, 95%, 96%, 97%, 98%, 99%, 100% sequence similarity to SEQ ID No:1.

9. Method for identifying or selecting a spinach plant carrying the allele as claimed in any of the claims 1-3 and 5 or a nucleic acid as claimed in claim 7, comprising determining the presence of a coding sequence in the genome of a plant, wherein said sequence has in order of increased preference 95%, 96%, 97%, 98%, 99%, 100% sequence similarity to SEQ ID No:2.

10. Method for identifying or selecting a spinach plant carrying the allele as claimed in any of the claims 1-2, 4 and 6 a nucleic acid as claimed in claim 7, comprising determining the presence of a coding sequence in the genome of a plant, wherein said sequence has in order of increased preference 94%, 95%, 96%, 97%, 98%, 99%, 100%sequence similarity to SEQ ID No:3.

11. The method as claimed in any of the claims 8-10, comprising determining the presence of the LRR domain as defined in claim 1, wherein the LRR domain is in particular determined by using a primer pair to amplify the LRR domain, wherein the forward primer is a nucleic acid molecule comprising the sequence of SEQ ID No:6, or wherein the LRR domain is in particular determined by using a primer pair to amplify the LRR domain, wherein the reverse primer is a nucleic acid molecule comprising the sequence of SEQ ID No:7.

12. Primer pair comprising a forward primer which is a nucleic acid molecule comprising the sequence of SEQ ID No:6 and a reverse primer which is a nucleic acid molecule comprising the sequence of SEQ ID No:7.

## Patentansprüche

1. Ein als alpha-CMV bezeichnetes Allel, welches, wenn es in einer Spinatpflanze vorhanden ist, gegen CMV Resistenz verleiht, wobei das durch das Allel kodierte Protein ein CC-NBS-LRR-Protein ist, welches in seiner Aminosäuresequenz aufweist: a) das Motiv "MAEIGYSVC" an seinem N-Terminus; b) das Motiv "KWMCLR"; sowie c) eine LRR-Domäne, die in der Reihenfolge erhöhter Präferenz mindestens 95 %, 96 %, 97 %, 98 %, 99 %, 100 % Sequenzähnlichkeit zu SEQ ID Nr. 10 besitzt.

2. Das Allel nach Anspruch 1, wobei das Allel eine genomische Nukleotidsequenz aufweist, die in der Reihenfolge erhöhter Präferenz mindestens 80 %, 81 %, 82 %, 83 %, 84 %, 85 %, 86 %, 87 %, 88 %, 89 %, 90 %, 91 %, 92 %, 93 %, 94 %, 95 %, 96 %, 97 %, 98 %, 99 %, 100 % Sequenzähnlichkeit zu SEQ ID Nr. 1 besitzt.

3. Das Allel nach Anspruch 1, wobei das Allel eine kodierende Sequenz aufweist, die in der Reihenfolge erhöhter Präferenz mindestens 95 %, 96 %, 97 %, 98 %, 99 %, 100 % Sequenzähnlichkeit zu SEQ ID Nr. 2 besitzt.

4. Das Allel nach Anspruch 1, wobei das Allel eine kodierende Sequenz aufweist, die in der Reihenfolge erhöhter Präferenz mindestens 94 %, 95 %, 96 %, 97 %, 98 %, 99 %, 100 % Sequenzähnlichkeit zu SEQ ID Nr. 3 besitzt.

5. Das Allel nach Anspruch 1, wobei das Allel für ein Protein kodiert, mit einer Aminosäuresequenz, die in der Reihenfolge erhöhter Präferenz mindestens 95 %, 96 %, 97 %, 98 %, 99 %, 100 % Sequenzähnlichkeit zu SEQ ID Nr. 4 besitzt.

6. Das Allel nach Anspruch 1, wobei das Allel für ein Protein kodiert, mit einer Aminosäuresequenz, die in der Reihenfolge erhöhter Präferenz mindestens 95 %, 96 %, 97 %, 98 %, 99 %, 100 % Sequenzähnlichkeit zu SEQ ID Nr. 5 besitzt.

7. Nukleinsäure, die für ein CC-NBS-LRR-Protein kodiert, das in seiner Aminosäuresequenz aufweist: a) das Motiv "MAEIGYSVC" an seinem N-Terminus; b) das Motiv "KWMCLR"; und c) eine LRR-Domäne, die in der Reihenfolge erhöhter Präferenz mindestens 95 %, 96 %, 97 %, 98 %, 99 %, 100 % Sequenzähnlichkeit zu SEQ ID Nr. 10 besitzt, wobei die Nukleinsäure insbesondere eine genomische Nukleotidsequenz aufweist, die in der Reihenfolge erhöhter Präferenz mindestens 80 %, 81 %, 82 %, 83 %, 84 %, 85 %, 86 %, 87 %, 88 %, 89 %, 90 %, 91 %, 92 %, 93 %, 94 %, 95 %, 96 %, 97 %, 98 %, 99 %, 100 % Sequenzähnlichkeit zu SEQ ID Nr. 1 besitzt.

8. Verfahren zum Identifizieren oder Selektieren einer Spinatpflanze, die das in einem der Ansprüche 1 bis 6 beanspruchte Allel oder eine gemäß Anspruch 7 beanspruchte Nukleinsäure trägt, aufweisend das Bestimmen des Vorhandenseins einer genomischen Nukleotidsequenz im Genom einer Pflanze, wobei die Sequenz in der Reihenfolge erhöhter Präferenz 80 %, 81 %, 82 %, 83 %, 84 %, 85 %, 86 %, 87 %, 88 %, 89 %, 90 %, 91 %, 92 %, 93 %, 94 %, 95 %, 96 %, 97 %, 98 %, 99 %, 100 % Sequenzähnlichkeit zu SEQ ID Nr. 1 besitzt.

9. Verfahren zum Identifizieren oder Selektieren einer Spinatpflanze, die das in einem der Ansprüche 1 bis 3 sowie 5 beanspruchte Allel oder eine gemäß Anspruch 7 beanspruchte Nukleinsäure trägt, aufweisend das Bestimmen des Vorhandenseins einer kodierenden Sequenz im Genom einer Pflanze, wobei die Sequenz in der Reihenfolge erhöhter Präferenz 95 %, 96 %, 97 %, 98 %, 99 %, 100 % Sequenzähnlichkeit zu SEQ ID Nr. 2 besitzt.

10. Verfahren zum Identifizieren oder Selektieren einer Spinatpflanze, die das Allel nach einem der Ansprüche 1 bis 2, 4 und 6 trägt und eine Nukleinsäure nach Anspruch 7, aufweisend das Bestimmen des Vorhandenseins einer kodierenden Sequenz im Genom einer Pflanze, wobei die Sequenz in der Reihenfolge erhöhter Präferenz 94 %, 95 %, 96 %, 97 %, 98 %, 99 %, 100 % Sequenzähnlichkeit zu SEQ ID Nr. 3 besitzt.

11. Das Verfahren nach einem der Ansprüche 8 bis 10, aufweisend das Bestimmen des Vorhandenseins der in Anspruch 1 definierten LRR-Domäne, wobei die LRR-Domäne insbesondere durch Verwendung eines Primer-Paars zur Amplifizierung der LRR-Domäne bestimmt wird, wobei der Vorwärtsprimer ein Nukleinsäure-Molekül ist, das die Sequenz von SEQ ID Nr. 6 aufweist, oder wobei die LRR-Domäne insbesondere durch Verwendung eines Primer-Paars zur Amplifizierung der LRR-Domäne bestimmt wird, wobei der Rückwärtsprimer ein Nukleinsäure-Molekül ist, das die Sequenz von SEQ ID Nr. 7 aufweist.

12. Primer-Paar, bestehend aus einem Vorwärtsprimer, bei dem es sich um ein Nukleinsäure-Molekül handelt, das die Sequenz von SEQ ID Nr. 6 aufweist, und einem Rückwärtsprimer, bei dem es sich um ein Nukleinsäure-Molekül handelt, das die Sequenz von SEQ ID Nr. 7 aufweist.

## Revendications

1. Allèle désigné alpha-CMV qui confère une résistance au CMV quand il est présent dans une plante d'épinard, dans lequel la protéine codée par ledit allèle est une protéine CC-NBS-LRR qui comprend dans sa séquence d'acides aminés : a) le motif « MAEIGYSVC » à son extrémité N-terminale ; b) le motif « KWMCLR » ; et c) un domaine LRR qui présente, par ordre de préférence croissante, au moins 95 %, 96 %, 97 %, 98 %, 99 %, 100 % de similarité de séquence avec SEQ ID No : 10.

2. Allèle selon la revendication 1, dans lequel l'allèle présente une séquence nucléotidique génomique qui, par ordre de préférence croissante, présente au moins 80 %, 81 %, 82 %, 83 %, 84 %, 85 %, 86 %, 87 %, 88 %, 89 %, 90 %, 91 %, 92 %, 93 %, 94 %, 95 %, 96 %, 97 %, 98 %, 99 %, 100 % de similarité de séquence avec SEQ ID No : 1.

3. Allèle selon la revendication 1, dans lequel l'allèle présente une séquence codante qui, par ordre de préférence croissante, présente au moins 95 %, 96 %, 97 %, 98 %, 99 %, 100 % de similarité de séquence avec SEQ ID No : 2.

4. Allèle selon la revendication 1, dans lequel l'allèle présente une séquence codante qui, par ordre de préférence croissante, présente au moins 94 %, 95 %, 96 %, 97 %, 98 %, 99 %, 100 % de similarité de séquence avec SEQ ID No : 3.

5. Allèle selon la revendication 1, dans lequel l'allèle code pour une protéine présentant une séquence d'acides aminés qui, par ordre de préférence croissante, présente au moins 95 %, 96 %, 97 %, 98 %, 99 %, 100 % de similarité de séquence avec SEQ ID No : 4.

6. Allèle selon la revendication 1, dans lequel l'allèle code pour une protéine présentant une séquence d'acides aminés qui, par ordre de préférence croissante, présente au moins 95 %, 96 %, 97 %, 98 %, 99 %, 100 % de similarité de séquence avec SEQ ID No : 5.

7. Acide nucléique codant pour une protéine CC-NBS-LRR qui comprend dans sa séquence d'acides aminés : a) le motif « MAEIGYSVC » à son extrémité N-terminale ; b) le motif « KWMCLR » ; et c) un domaine LRR qui présente, par ordre de préférence croissante, au moins 95 %, 96 %, 97 %, 98 %, 99 %, 100 % de similarité de séquence avec SEQ ID No : 10, dans lequel l'acide nucléique présente en particulier une séquence nucléotidique génomique qui, par ordre de préférence croissante, présente au moins 80 %, 81 %, 82 %, 83 %, 84 %, 85 %, 86 %, 87 %, 88 %, 89 %, 90 %, 91 %, 92 %, 93 %, 94 %, 95 %, 96 %, 97 %, 98 %, 99 %, 100 % de similarité de séquence avec SEQ ID No : 1.

8. Procédé d'identification ou de sélection d'une plante d'épinard portant l'allèle selon l'une quelconque des revendications 1 à 6 ou un acide nucléique selon la revendication 7, comprenant la détermination de la présence d'une séquence nucléotidique génomique dans le génome d'une plante, dans lequel ladite séquence présente, par ordre de préférence croissante, 80 %, 81 %, 82 %, 83 %, 84 %, 85 %, 86 %, 87 %, 88 %, 89 %, 90 %, 91 %, 92 %, 93 %, 94 %, 95 %, 96 %, 97 %, 98 %, 99 %, 100 % de similarité de séquence avec SEQ ID No : 1.

9. Procédé d'identification ou de sélection d'une plante d'épinard portant l'allèle selon l'une quelconque des revendications 1 à 3 et 5 ou un acide nucléique selon la revendication 7, comprenant la détermination de la présence d'une séquence codante dans le génome d'une plante, dans lequel ladite séquence présente, par ordre de préférence croissante, 95 %, 96 %, 97 %, 98 %, 99 %, 100 % de similarité de séquence avec SEQ ID No : 2.

10. Procédé d'identification ou de sélection d'une plante d'épinard portant l'allèle selon l'une quelconque des revendications 1 à 2, 4 et 6 un acide nucléique selon la revendication 7, comprenant la détermination de la présence d'une séquence codante dans le génome d'une plante, dans lequel ladite séquence présente, par ordre de préférence croissante, 94 %, 95 %, 96 %, 97 %, 98 %, 99 %, 100 % de similarité de séquence avec SEQ ID No : 3.

11. Procédé selon l'une quelconque des revendications 8 à 10, comprenant la détermination de la présence du domaine LRR tel que défini dans la revendication 1, dans lequel le domaine LRR est en particulier déterminé en utilisant une paire d'amorces pour amplifier le domaine LRR, dans lequel l'amorce sens est une molécule d'acide nucléique comprenant la séquence de SEQ ID No : 6, ou dans lequel le domaine LRR est en particulier déterminé en utilisant une paire d'amorces pour amplifier le domaine LRR, dans lequel l'amorce antisens est une molécule d'acide nucléique comprenant la séquence de SEQ ID No : 7.

12. Paire d'amorces comprenant une amorce sens qui est une molécule d'acide nucléique comprenant la séquence de SEQ ID No : 6 et une amorce antisens qui est une molécule d'acide nucléique comprenant la séquence de SEQ ID No : 7.
